# EUROPEAN PATENT APPLICATION

(11) **EP 3 936 151 A1**
(43) Date of publication of application: **12.01.2022**
(21) Application number: 20765892.3
(22) Date of filing: 06.03.2020
(51) Int. Cl.: A61K 47/68, A61P 35/00, C07H 3/02, C07K 16/28, A61K 39/00

(54) **ANTIBODY-DRUG CONJUGATE AND USE THEREOF**

(30) Priority: 07.03.2019 US 201962814982 P
(71) Applicant: ABL Bio, Inc., Seongnam-si, Gyeonggi-do 13488 (KR)
(72) Inventor: JUNG, Jinwon, Seongnam-si Gyeonggi-do 13488 (KR); KIM, Juhee, Seongnam-si Gyeonggi-do 13488 (KR); PAK, Youngdon, Seongnam-si Gyeonggi-do 13488 (KR); SONG, Daehae, Seongnam-si Gyeonggi-do 13488 (KR); YEOM, Donghoon, Seongnam-si Gyeonggi-do 13488 (KR); EOM, Jaehyun, Seongnam-si Gyeonggi-do 13488 (KR); HONG, Youngeun, Seongnam-si Gyeonggi-do 13488 (KR); LEE, Bora, Seongnam-si Gyeonggi-do 13488 (KR)
(74) Representative: Mewburn Ellis LLP
(86) International application number: PCT/KR2020/003195
(87) International publication number: WO 2020/180158

(57) **Abstract**

The present invention relates to a novel compound comprising a galactose trigger moiety and cyclopropabenzindole (CBI), and an antibody-drug conjugate prepared by using same.

<110>    ABL Bio Inc.

<120>    Antibody-Drug Conjugate and Use Thereof

<130>    2353

<160>    20

<170>    PatentIn version 3.2

<210>    1
<211>    13
<212>    PRT
<213>    Artificial Sequence

<220>
<223>    Synthetic Sequence

<400>    1
Ser Gly Ser Ser Ser Asn Ile Gly Ser Asn Ser Val Ser
1               5                   10

<210>    2
<211>    7
<212>    PRT
<213>    Artificial Sequence

<220>
<223>    Synthetic Sequence

<400>    2
Ala Asp Ser Lys Arg Pro Ser
1               5

<210>    3
<211>    11
<212>    PRT
<213>    Artificial Sequence

<220>
<223>    Synthetic Sequence

<400>    3
Gly Ser Trp Asp Tyr Ser Leu Ser Gly Tyr Val
1               5                   10

<210>    4
<211>    216
<212>    PRT
<213>    Artificial Sequence

<220>
<223>    Synthetic Sequence

<400>    4
Gln Ser Val Leu Thr Gln Pro Pro Ser Ala Ser Gly Thr Pro Gly Gln

1               5                   10                  15

Arg Val Thr Ile Ser Cys Ser Gly Ser Ser Ser Asn Ile Gly Ser Asn
            20                  25                  30

Ser Val Ser Trp Tyr Gln Gln Leu Pro Gly Thr Ala Pro Lys Leu Leu
        35                  40                  45

Ile Tyr Ala Asp Ser Lys Arg Pro Ser Gly Val Pro Asp Arg Phe Ser
    50                  55                  60

Gly Ser Lys Ser Gly Thr Ser Ala Ser Leu Ala Ile Ser Gly Leu Arg
65                  70                  75                  80

Ser Glu Asp Glu Ala Asp Tyr Tyr Cys Gly Ser Trp Asp Tyr Ser Leu
                85                  90                  95

Ser Gly Tyr Val Phe Gly Gly Gly Thr Lys Leu Thr Val Leu Gly Gln
            100                 105                 110

Pro Lys Ala Ala Pro Ser Val Thr Leu Phe Pro Pro Ser Ser Glu Glu
        115                 120                 125

Leu Gln Ala Asn Lys Ala Thr Leu Val Cys Leu Ile Ser Asp Phe Tyr
    130                 135                 140

Pro Gly Ala Val Thr Val Ala Trp Cys Ala Asp Ser Ser Pro Val Lys
145                 150                 155                 160

Ala Gly Val Glu Thr Thr Thr Pro Ser Lys Gln Ser Asn Asn Lys Tyr
                165                 170                 175

Ala Ala Ser Ser Tyr Leu Ser Leu Thr Pro Glu Gln Trp Lys Ser His
            180                 185                 190

Arg Ser Tyr Ser Cys Gln Val Thr His Glu Gly Ser Thr Val Glu Lys
        195                 200                 205

Thr Val Ala Pro Thr Glu Cys Ser
    210                 215


<210>   5
<211>   5
<212>   PRT
<213>   Artificial Sequence

<220>
<223>   Synthetic Sequence


<400>   5
Asn Tyr Asp Met Ser
1               5


<210>   6
<211>   17
<212>   PRT
<213>   Artificial Sequence

<220>
<223>   Synthetic Sequence

<400>    6
Trp Ile Tyr Pro Ser Asp Ser Ser Ile Tyr Tyr Ala Asp Ser Val Lys
1               5                   10                  15

Gly

<210>    7
<211>    13
<212>    PRT
<213>    Artificial Sequence

<220>
<223>    Synthetic Sequence

<400>    7
Arg Gly Pro Phe Ala Asn Lys Tyr Arg Gln Phe Asp Tyr
1               5                   10

<210>    8
<211>    451
<212>    PRT
<213>    Artificial Sequence

<220>
<223>    Synthetic Sequence

<400>    8
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Asn Tyr
            20                  25                  30

Asp Met Ser Trp Val Arg Gln Ala Pro Gly Lys Gly Leu Glu Trp Val
        35                  40                  45

Ser Trp Ile Tyr Pro Ser Asp Ser Ser Ile Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Gly Pro Phe Ala Asn Lys Tyr Arg Gln Phe Asp Tyr Trp Gly
            100                 105                 110

Gln Gly Thr Leu Val Thr Val Ser Ser Ala Ser Thr Lys Gly Pro Ser
        115                 120                 125

Val Phe Pro Leu Ala Pro Ser Ser Lys Ser Thr Ser Gly Gly Thr Ala
    130                 135                 140

Ala Leu Gly Cys Leu Val Lys Asp Tyr Phe Pro Glu Pro Val Thr Val
145                 150                 155                 160

Ser Trp Asn Ser Gly Ala Leu Thr Ser Gly Val His Thr Phe Pro Ala
                165                 170                 175

Val Leu Gln Ser Ser Gly Leu Tyr Ser Leu Ser Ser Val Val Thr Val
            180                 185                 190

Pro Ser Ser Ser Leu Gly Thr Gln Thr Tyr Ile Cys Asn Val Asn His
        195                 200                 205

Lys Pro Ser Asn Thr Lys Val Asp Lys Lys Val Glu Pro Lys Ser Cys
    210                 215                 220

Asp Lys Thr His Thr Cys Pro Pro Cys Pro Ala Pro Glu Leu Leu Gly
225                 230                 235                 240

Gly Pro Ser Val Phe Leu Phe Pro Pro Lys Pro Lys Asp Thr Leu Met
                245                 250                 255

Ile Ser Arg Thr Pro Glu Val Thr Cys Val Val Val Asp Val Ser His
            260                 265                 270

Glu Asp Pro Glu Val Lys Phe Asn Trp Tyr Val Asp Gly Val Glu Val
        275                 280                 285

His Asn Ala Lys Thr Lys Pro Arg Glu Glu Gln Tyr Asn Ser Thr Tyr
    290                 295                 300

Arg Val Val Ser Val Leu Thr Val Leu His Gln Asp Trp Leu Asn Gly
305                 310                 315                 320

Lys Glu Tyr Lys Cys Lys Val Ser Asn Lys Ala Leu Pro Ala Pro Ile
                325                 330                 335

Glu Lys Thr Ile Ser Lys Ala Lys Gly Gln Pro Arg Glu Pro Gln Val
            340                 345                 350

Tyr Thr Leu Pro Pro Ser Arg Glu Glu Met Thr Lys Asn Gln Val Ser
        355                 360                 365

Leu Thr Cys Leu Val Lys Gly Phe Tyr Pro Ser Asp Ile Ala Val Glu
    370                 375                 380

Trp Glu Ser Asn Gly Gln Pro Glu Asn Asn Tyr Lys Thr Thr Pro Pro
385                 390                 395                 400

Val Leu Asp Ser Asp Gly Ser Phe Phe Leu Tyr Ser Lys Leu Thr Val
                405                 410                 415

Asp Lys Ser Arg Trp Gln Gln Gly Asn Val Phe Ser Cys Ser Val Met
            420                 425                 430

His Glu Ala Leu His Asn His Tyr Thr Gln Lys Ser Leu Ser Leu Ser
        435                 440                 445

Pro Gly Lys
    450


<210>   9
<211>   13
<212>   PRT
<213>   Artificial Sequence

<220>
<223>    Synthetic Sequence


<400>    9
Thr Gly Ser Ser Ser Asn Ile Gly Ser Asn Asp Val Thr
1               5                   10


<210>    10
<211>    7
<212>    PRT
<213>    Artificial Sequence

<220>
<223>    Synthetic Sequence


<400>    10
Ala Asp Ser Lys Arg Pro Ser
1               5


<210>    11
<211>    11
<212>    PRT
<213>    Artificial Sequence

<220>
<223>    Synthetic Sequence


<400>    11
Gly Thr Trp Asp Tyr Ser Leu Ser Gly Tyr Val
1               5                   10


<210>    12
<211>    216
<212>    PRT
<213>    Artificial Sequence

<220>
<223>    Synthetic Sequence


<400>    12
Gln Ser Val Leu Thr Gln Pro Pro Ser Ala Ser Gly Thr Pro Gly Gln
1               5                   10                  15

Arg Val Thr Ile Ser Cys Thr Gly Ser Ser Ser Asn Ile Gly Ser Asn
            20                  25                  30

Asp Val Thr Trp Tyr Gln Gln Leu Pro Gly Thr Ala Pro Lys Leu Leu
        35                  40                  45

Ile Tyr Ala Asp Ser Lys Arg Pro Ser Gly Val Pro Asp Arg Phe Ser
    50                  55                  60

Gly Ser Lys Ser Gly Thr Ser Ala Ser Leu Ala Ile Ser Gly Leu Arg
65                  70                  75                  80

Ser Glu Asp Glu Ala Asp Tyr Tyr Cys Gly Thr Trp Asp Tyr Ser Leu
                85                  90                  95

Ser Gly Tyr Val Phe Gly Gly Gly Thr Lys Leu Thr Val Leu Gly Gln
            100                 105                 110

Pro Lys Ala Ala Pro Ser Val Thr Leu Phe Pro Pro Ser Ser Glu Glu
        115                 120                 125

Leu Gln Ala Asn Lys Ala Thr Leu Val Cys Leu Ile Ser Asp Phe Tyr
    130                 135                 140

Pro Gly Ala Val Thr Val Ala Trp Cys Ala Asp Ser Ser Pro Val Lys
145                 150                 155                 160

Ala Gly Val Glu Thr Thr Thr Pro Ser Lys Gln Ser Asn Asn Lys Tyr
                165                 170                 175

Ala Ala Ser Ser Tyr Leu Ser Leu Thr Pro Glu Gln Trp Lys Ser His
            180                 185                 190

Arg Ser Tyr Ser Cys Gln Val Thr His Glu Gly Ser Thr Val Glu Lys
        195                 200                 205

Thr Val Ala Pro Ala Glu Cys Ser
    210                 215


<210>    13
<211>    5
<212>    PRT
<213>    Artificial Sequence

<220>
<223>    Synthetic Sequence


<400>    13
Asn Tyr Ala Met Ser
1               5


<210>    14
<211>    17
<212>    PRT
<213>    Artificial Sequence

<220>
<223>    Synthetic Sequence


<400>    14
Ser Ile Ser His Asn Ser Gly Ser Thr Tyr Tyr Ala Asp Ser Val Lys
1               5                   10                  15

Gly



<210>    15
<211>    18
<212>    PRT
<213>    Artificial Sequence

<220>
<223>    Synthetic Sequence

<400>    15
Phe Ile Ser Ala Arg Lys Ser Leu Gly Arg Ser Tyr Ser Asn Gly Met
1               5                   10                  15

Asp Val

<210>    16
<211>    457
<212>    PRT
<213>    Artificial Sequence

<220>
<223>    Synthetic Sequence

<400>    16
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Asn Tyr
            20                  25                  30

Ala Met Ser Trp Val Arg Gln Ala Pro Gly Lys Gly Leu Glu Trp Val
        35                  40                  45

Ser Ser Ile Ser His Asn Ser Gly Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Phe Ile Ser Ala Arg Lys Ser Leu Gly Arg Ser Tyr Ser Asn
            100                 105                 110

Gly Met Asp Val Trp Gly Gln Gly Thr Leu Val Thr Val Ser Ser Ala
        115                 120                 125

Ser Thr Lys Gly Pro Ser Val Phe Pro Leu Ala Pro Ser Ser Lys Ser
    130                 135                 140

Thr Ser Gly Gly Thr Ala Ala Leu Gly Cys Leu Val Lys Asp Tyr Phe
145                 150                 155                 160

Pro Glu Pro Val Thr Val Ser Trp Asn Ser Gly Ala Leu Thr Ser Gly
                165                 170                 175

Val His Thr Phe Pro Ala Val Leu Gln Ser Ser Gly Leu Tyr Ser Leu
            180                 185                 190

Ser Ser Val Val Thr Val Pro Ser Ser Ser Leu Gly Thr Gln Thr Tyr
        195                 200                 205

Ile Cys Asn Val Asn His Lys Pro Ser Asn Thr Lys Val Asp Lys Lys

210                 215                 220

Val Glu Pro Lys Ser Cys Asp Lys Thr His Thr Cys Pro Pro Cys Pro
225                 230                 235                 240

Ala Pro Glu Leu Leu Gly Gly Pro Ser Val Phe Leu Phe Pro Pro Lys
                245                 250                 255

Pro Lys Asp Thr Leu Met Ile Ser Arg Thr Pro Glu Val Thr Cys Val
            260                 265                 270

Val Val Asp Val Ser His Glu Asp Pro Glu Val Lys Phe Asn Trp Tyr
        275                 280                 285

Val Asp Gly Val Glu Val His Asn Ala Lys Thr Lys Pro Arg Glu Glu
    290                 295                 300

Gln Tyr Asn Ser Thr Tyr Arg Val Val Ser Val Leu Thr Val Leu His
305                 310                 315                 320

Gln Asp Trp Leu Asn Gly Lys Glu Tyr Lys Cys Lys Val Ser Asn Lys
                325                 330                 335

Ala Leu Pro Ala Pro Ile Glu Lys Thr Ile Ser Lys Ala Lys Gly Gln
            340                 345                 350

Pro Arg Glu Pro Gln Val Tyr Thr Leu Pro Pro Ser Arg Glu Glu Met
        355                 360                 365

Thr Lys Asn Gln Val Ser Leu Thr Cys Leu Val Lys Gly Phe Tyr Pro
    370                 375                 380

Ser Asp Ile Ala Val Glu Trp Glu Ser Asn Gly Gln Pro Glu Asn Asn
385                 390                 395                 400

Tyr Lys Thr Thr Pro Pro Val Leu Asp Ser Asp Gly Ser Phe Phe Leu
                405                 410                 415

Tyr Ser Lys Leu Thr Val Asp Lys Ser Arg Trp Gln Gln Gly Asn Val
            420                 425                 430

Phe Ser Cys Ser Val Met His Glu Ala Leu His Asn His Tyr Thr Gln
        435                 440                 445

Lys Ser Leu Ser Leu Ser Pro Gly Lys
    450                 455


<210>   17
<211>   219
<212>   PRT
<213>   Artificial Sequence

<220>
<223>   10H1 VL


<400>   17
Asp Ile Gln Met Thr Gln Ser Pro Ser Ser Leu Ser Ala Ser Val Gly
1               5                   10                  15

Asp Arg Val Thr Ile Thr Cys Arg Ser Ser Glu Thr Leu Val His Ser
            20                  25                  30

Ser Gly Asn Thr Tyr Leu Glu Trp Tyr Gln Gln Lys Pro Gly Lys Ala
        35                  40                  45

Pro Lys Leu Leu Ile Tyr Arg Val Ser Asn Arg Phe Ser Gly Val Pro
    50                  55                  60

Ser Arg Phe Ser Gly Ser Gly Ser Gly Thr Asp Phe Thr Leu Thr Ile
65                  70                  75                  80

Ser Ser Leu Gln Pro Glu Asp Phe Ala Thr Tyr Tyr Cys Phe Gln Gly
                85                  90                  95

Ser Phe Asn Pro Leu Thr Phe Gly Gln Gly Thr Lys Val Glu Ile Lys
            100                 105                 110

Arg Thr Val Ala Ala Pro Ser Val Phe Ile Phe Pro Pro Ser Asp Glu
        115                 120                 125

Gln Leu Lys Ser Gly Thr Ala Ser Val Val Cys Leu Leu Asn Asn Phe
    130                 135                 140

Tyr Pro Arg Glu Ala Lys Val Gln Trp Cys Val Asp Asn Ala Leu Gln
145                 150                 155                 160

Ser Gly Asn Ser Gln Glu Ser Val Thr Glu Gln Asp Ser Lys Asp Ser
                165                 170                 175

Thr Tyr Ser Leu Ser Ser Thr Leu Thr Leu Ser Lys Ala Asp Tyr Glu
            180                 185                 190

Lys His Lys Val Tyr Ala Cys Glu Val Thr His Gln Gly Leu Ser Ser
        195                 200                 205

Pro Val Thr Lys Ser Phe Asn Arg Gly Glu Cys
    210                 215

<210> 18
<211> 450
<212> PRT
<213> Artificial Sequence

<220>
<223> 10H1 VH

<400> 18
Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Ser Phe Ser Asp Phe
            20                  25                  30

Ala Met Ser Trp Val Arg Gln Ala Pro Gly Lys Gly Leu Glu Trp Val
        35                  40                  45

Ala Thr Ile Gly Arg Val Ala Phe His Thr Tyr Tyr Pro Asp Ser Met
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg His Arg Gly Phe Asp Val Gly His Phe Asp Phe Trp Gly Gln
            100                 105                 110

Gly Thr Leu Val Thr Val Ser Ser Cys Ser Thr Lys Gly Pro Ser Val
        115                 120                 125

Phe Pro Leu Ala Pro Ser Ser Lys Ser Thr Ser Gly Gly Thr Ala Ala
    130                 135                 140

Leu Gly Cys Leu Val Lys Asp Tyr Phe Pro Glu Pro Val Thr Val Ser
145                 150                 155                 160

Trp Asn Ser Gly Ala Leu Thr Ser Gly Val His Thr Phe Pro Ala Val
                165                 170                 175

Leu Gln Ser Ser Gly Leu Tyr Ser Leu Ser Ser Val Val Thr Val Pro
            180                 185                 190

Ser Ser Ser Leu Gly Thr Gln Thr Tyr Ile Cys Asn Val Asn His Lys
        195                 200                 205

Pro Ser Asn Thr Lys Val Asp Lys Lys Val Glu Pro Lys Ser Cys Asp
    210                 215                 220

Lys Thr His Thr Cys Pro Pro Cys Pro Ala Pro Glu Leu Leu Gly Gly
225                 230                 235                 240

Pro Ser Val Phe Leu Phe Pro Pro Lys Pro Lys Asp Thr Leu Met Ile
                245                 250                 255

Ser Arg Thr Pro Glu Val Thr Cys Val Val Val Asp Val Ser His Glu
            260                 265                 270

Asp Pro Glu Val Lys Phe Asn Trp Tyr Val Asp Gly Val Glu Val His
        275                 280                 285

Asn Ala Lys Thr Lys Pro Arg Glu Glu Gln Tyr Asn Ser Thr Tyr Arg
    290                 295                 300

Val Val Ser Val Leu Thr Val Leu His Gln Asp Trp Leu Asn Gly Lys
305                 310                 315                 320

Glu Tyr Lys Cys Lys Val Ser Asn Lys Ala Leu Pro Ala Pro Ile Glu
                325                 330                 335

Lys Thr Ile Ser Lys Ala Lys Gly Gln Pro Arg Glu Pro Gln Val Tyr
            340                 345                 350

Thr Leu Pro Pro Ser Arg Glu Glu Met Thr Lys Asn Gln Val Ser Leu
        355                 360                 365

Thr Cys Leu Val Lys Gly Phe Tyr Pro Ser Asp Ile Ala Val Glu Trp
    370                 375                 380

Glu Ser Asn Gly Gln Pro Glu Asn Asn Tyr Lys Thr Thr Pro Pro Val
385                 390                 395                 400

Leu Asp Ser Asp Gly Ser Phe Phe Leu Tyr Ser Lys Leu Thr Val Asp
                405                 410                 415

Lys Ser Arg Trp Gln Gln Gly Asn Val Phe Ser Cys Ser Val Met His
            420                 425                 430

Glu Ala Leu His Asn His Tyr Thr Gln Lys Ser Leu Ser Leu Ser Pro
        435                 440                 445

Gly Lys
    450

<210> 19
<211> 218
<212> PRT
<213> Artificial Sequence

<220>
<223> 6E7(N54A) VL

<400> 19
Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Ser Val Ser Thr Ser
            20                  25                  30

Ser Tyr Asn Tyr Met His Trp Tyr Gln Gln Lys Pro Gly Lys Pro Pro
        35                  40                  45

Lys Leu Leu Ile Lys Tyr Ala Ser Asn Leu Glu Ser Gly Val Pro Ser
    50                  55                  60

Arg Phe Ser Gly Ser Gly Ser Gly Thr Asp Phe Thr Leu Thr Ile Ser
65                  70                  75                  80

Ser Leu Gln Pro Glu Asp Phe Ala Thr Tyr Tyr Cys Gln His Ser Trp
                85                  90                  95

Glu Ile Pro Leu Thr Phe Gly Gln Gly Thr Lys Val Glu Ile Lys Arg
            100                 105                 110

Thr Val Ala Ala Pro Ser Val Phe Ile Phe Pro Pro Ser Asp Glu Gln
        115                 120                 125

Leu Lys Ser Gly Thr Ala Ser Val Val Cys Leu Leu Asn Asn Phe Tyr
    130                 135                 140

Pro Arg Glu Ala Lys Val Gln Trp Cys Val Asp Asn Ala Leu Gln Ser
145                 150                 155                 160

Gly Asn Ser Gln Glu Ser Val Thr Glu Gln Asp Ser Lys Asp Ser Thr
                165                 170                 175

Tyr Ser Leu Ser Ser Thr Leu Thr Leu Ser Lys Ala Asp Tyr Glu Lys
            180                 185                 190

His Lys Val Tyr Ala Cys Glu Val Thr His Gln Gly Leu Ser Ser Pro
        195                 200                 205

Val Thr Lys Ser Phe Asn Arg Gly Glu Cys
    210                 215

<210>    20
<211>    452
<212>    PRT
<213>    Artificial Sequence

<220>
<223>    6E7(N54A) VH


<400>    20
Glu Val Gln Leu Val Gln Ser Gly Ala Glu Val Lys Lys Pro Gly Ala
1               5                   10                  15

Ser Val Lys Val Ser Cys Lys Ala Ser Gly Tyr Ser Phe Thr Asp Tyr
            20                  25                  30

Tyr Met His Trp Val Arg Gln Ala Pro Gly Gln Gly Leu Glu Trp Ile
        35                  40                  45

Gly Arg Ile Asn Pro Tyr Ala Gly Ala Ala Phe Tyr Ser Gln Asn Phe
    50                  55                  60

Lys Asp Arg Val Thr Leu Thr Val Asp Thr Ser Thr Ser Thr Ala Tyr
65                  70                  75                  80

Leu Glu Leu Ser Ser Leu Arg Ser Glu Asp Thr Ala Val Tyr Tyr Cys
                85                  90                  95

Ala Ile Glu Arg Gly Ala Asp Leu Glu Gly Tyr Ala Met Asp Tyr Trp
            100                 105                 110

Gly Gln Gly Thr Leu Val Thr Val Ser Ser Ala Ser Thr Lys Gly Pro
        115                 120                 125

Ser Val Phe Pro Leu Ala Pro Ser Ser Lys Ser Thr Ser Gly Gly Thr
    130                 135                 140

Ala Ala Leu Gly Cys Leu Val Lys Asp Tyr Phe Pro Glu Pro Val Thr
145                 150                 155                 160

Val Ser Trp Asn Ser Gly Ala Leu Thr Ser Gly Val His Thr Phe Pro
                165                 170                 175

Ala Val Leu Gln Ser Ser Gly Leu Tyr Ser Leu Ser Ser Val Val Thr
            180                 185                 190

Val Pro Ser Ser Ser Leu Gly Thr Gln Thr Tyr Ile Cys Asn Val Asn
        195                 200                 205

His Lys Pro Ser Asn Thr Lys Val Asp Lys Lys Val Glu Pro Lys Ser
    210                 215                 220

Cys Asp Lys Thr His Thr Cys Pro Pro Cys Pro Ala Pro Glu Leu Leu
225                 230                 235                 240

Gly Gly Pro Ser Val Phe Leu Phe Pro Pro Lys Pro Lys Asp Thr Leu
                245                 250                 255

Met Ile Ser Arg Thr Pro Glu Val Thr Cys Val Val Val Asp Val Ser
            260                 265                 270

His Glu Asp Pro Glu Val Lys Phe Asn Trp Tyr Val Asp Gly Val Glu
        275                 280                 285

Val His Asn Ala Lys Thr Lys Pro Arg Glu Glu Gln Tyr Asn Ser Thr
    290                 295                 300

Tyr Arg Val Val Ser Val Leu Thr Val Leu His Gln Asp Trp Leu Asn
305                 310                 315                 320

Gly Lys Glu Tyr Lys Cys Lys Val Ser Asn Lys Ala Leu Pro Ala Pro
                325                 330                 335

Ile Glu Lys Thr Ile Ser Lys Ala Lys Gly Gln Pro Arg Glu Pro Gln
            340                 345                 350

Val Tyr Thr Leu Pro Pro Ser Arg Glu Glu Met Thr Lys Asn Gln Val
        355                 360                 365

Ser Leu Thr Cys Leu Val Lys Gly Phe Tyr Pro Ser Asp Ile Ala Val
    370                 375                 380

Glu Trp Glu Ser Asn Gly Gln Pro Glu Asn Asn Tyr Lys Thr Thr Pro
385                 390                 395                 400

Pro Val Leu Asp Ser Asp Gly Ser Phe Phe Leu Tyr Ser Lys Leu Thr
                405                 410                 415

Val Asp Lys Ser Arg Trp Gln Gln Gly Asn Val Phe Ser Cys Ser Val
            420                 425                 430

Met His Glu Ala Leu His Asn His Tyr Thr Gln Lys Ser Leu Ser Leu
        435                 440                 445

Ser Pro Gly Lys
    450

## Description

### [Technical Field]

The present invention relates to a novel compound comprising a galactose trigger moiety and cyclopropabenzindole (CBI) and an antibody-drug conjugate prepared using the same.

### [Background Art]

In recent years, methods for diagnosing or treating a variety of diseases using antibodies have been studied. In particular, various treatment methods using antibodies have been developed due to the target specificity of the antibodies, and various types of drugs including antibodies, for example, antibody-drug conjugates (ADCs), are being developed. Accordingly, methods to increase the *in-vivo* stability of antibodies or antibody-drug conjugates and maximize the therapeutic effect thereof are continuously being studied.

Among them, ADCs generally have lower *in-vivo* stability compared to natural antibodies, but were developed in order to improve the conventionally low therapeutic effect, which is the drawback of natural antibodies, through binding with drugs. Drugs having specific pharmaceutical efficacy, such as cytotoxins, which are combined with target-specific antibodies, have been developed in various ways and antibody-drug conjugates that can induce cancer cell death by binding drugs to cancer cell-specific antibodies have been commercialized.

Clinical trials are underway on ADCs containing a DNA minor groove alkylating agent as a payload among drugs. Examples of such drugs include pyrrolobenzodiazepine (PBD) dimers and cyclopropabenzindole (CBI)-based duocarmycin derivatives. In particular, CBI is known to be cytotoxic to various types of cancer, and a CBI dimer has been also reported to be highly cytotoxic (Tietze et al., Angew. Chem. Int. Ed. Engl. 2010, 49, 7336-7339).

Before an ADC is delivered to target cancer cells, the linker may be accidentally cleaved from the ADC and the drug may be released therefrom early, which poses a risk of systemic toxicity. The risk may be greater if the drug is highly cytotoxic. In order to prevent this phenomenon, when a derivative cleaved in the lysosome is used as the drug, the derivative acts as a trigger (prodrug functional group), so the trigger must be cleaved in addition to the linker before the active cytotoxic drug is released, so that the risk can be reduced.

It has been considered that a carbamate group or a phosphate group may be attached as a trigger to CBI. When the carbamate group is used as the trigger, it may be cleaved in the lysosome and/or cytoplasm by a carboxylesterase. When the phosphate group is used as the trigger, the phosphate group may be cleaved in the lysosome and/or cytoplasm by a phosphatase. However, these conventional triggers still have a safety problem.

Against this technical background, as a result of extensive efforts to develop a trigger moiety that can effectively exhibit drug activity as well as improved safety due to higher target-specific toxicity than that of conventional triggers, the present inventors have found that prodrugs containing galactose as a trigger can be used for ADCs, and completed the present invention based thereon.

### [Disclosure]

It is one object of the present invention to provide a compound represented by the following Formula (I) or (II), which is a CBI dimer compound bound with a trigger moiety, or a pharmaceutically acceptable salt or solvate thereof.

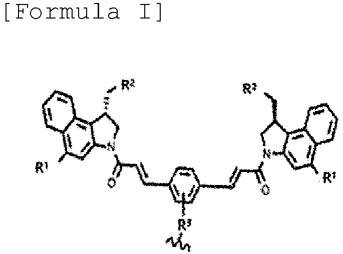

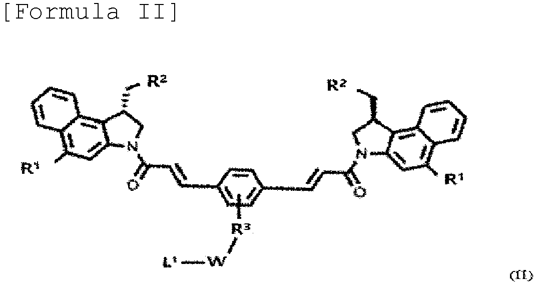

It is another object of the present invention to provide an antibody-drug conjugate represented by Formula (III), wherein an antibody is bound with a compound represented by Formula (II), which is a CBI dimer compound bound with a trigger moiety.

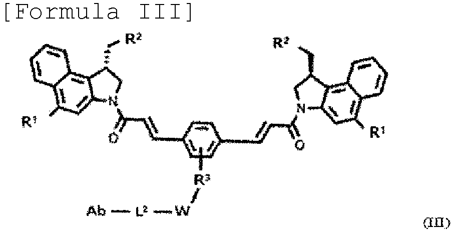

It is another object of the present invention to provide a composition for preventing or treating a proliferative disease comprising the antibody-drug conjugate.

In accordance with an aspect of the present invention, the above and other objects can be accomplished by the provision of a compound represented by the following Formula (I) or a pharmaceutically acceptable salt or solvate thereof: 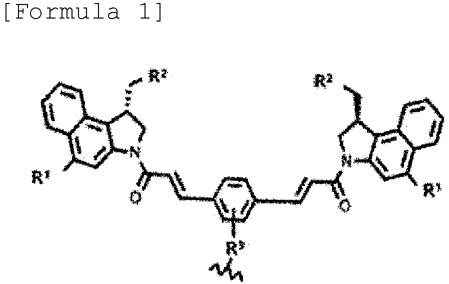 wherein R¹ is D-galactose β-pyranose or D-galactose α-pyranose;
R² is Cl or Br; and
R³ is a single bond, -O- or -NH-.

In accordance with another aspect of the present invention, there is provided a compound represented by the following Formula (II) or a pharmaceutically acceptable salt or solvate thereof: 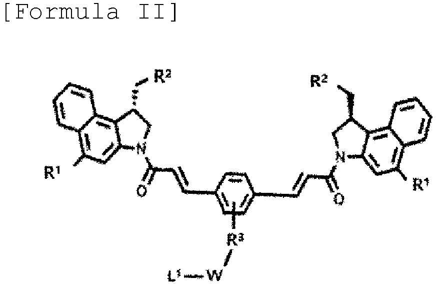 wherein R¹ is D-galactose β-pyranose or D-galactose α-pyranose;
R² is Cl or Br;
R³ is a single bond, -O- or -NH-;
W is a spacer; and
L¹ is a linker.

In accordance with another aspect of the present invention, there is provided an antibody-drug conjugate containing a compound represented by the following Formula (III) or a pharmaceutically acceptable salt or solvate thereof: 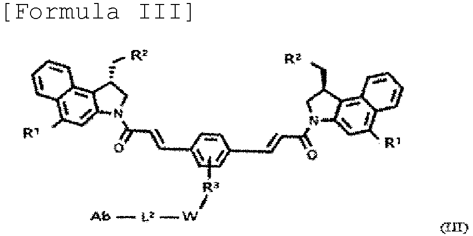 wherein
R¹ is D-galactose β-pyranose or D-galactose α-pyranose;
R² is Cl or Br;
R³ is a single bond, -O- or -NH-;
W is a spacer;
L² is a linker; and
Ab is an antibody or an antigen-binding fragment thereof.

In accordance with another aspect of the present invention, there is provided a pharmaceutical composition for preventing or treating a proliferative disease comprising the conjugate.

### [Description of Drawings]

FIG. 1 is a schematic diagram illustrating a process in which a prodrug CBI dimer is converted to an activated form.
FIG. 2 shows the result of detection of the expression of beta-galactosidase in various tumor cell lines.
FIG. 3 shows the result of detection of the efficacy of the prodrug CBI dimer according to the present invention on free drugs.
FIG. 4 shows the result of confirming the purity of the antibody-drug conjugate according to the present invention using SE-HPLC.
FIG. 5 shows the result of confirming the purity of the antibody-drug conjugate according to the present invention using SE-HPLC.
FIG. 6 shows the result of analyzing the DAR of the antibody-drug conjugate according to the present invention using LC/MS.
FIG. 7 shows the result of analyzing the DAR of the antibody-drug conjugate according to the present invention using LC/MS.
FIG. 8 shows the result of confirming the *in-vitro* cytotoxicity of the antibody-drug conjugate according to the present invention using a H929 cell line.
FIG. 9 shows the result of confirming the *in-vitro* cytotoxicity of the antibody-drug conjugate according to the present invention using each of Mino and Jeko-1 cell lines.
FIG. 10 shows the result of confirming the *in-vitro* cytotoxicity of the antibody-drug conjugate according to the present invention using each of NCI-N87 and HCC1954 cell lines.
FIG. 11 shows the result of confirming the *in-vitro* cytotoxicity of the antibody-drug conjugate according to the present invention using an OVCAR-3 cell line.
FIG. 12 shows the result of confirming the *in-vivo* cytotoxicity of the antibody-drug conjugate according to the present invention using a Jeko-1 model.
FIG. 13 shows the result of confirming the *in-vivo* cytotoxicity of the antibody-drug conjugate according to the present invention using a multiple myeloma model xenograft.
FIG. 14 shows the result of confirming the *in-vivo* cytotoxicity of the antibody-drug conjugate according to the present invention using an AML model xenograft.
FIG. 15 shows changes in body weight of animals administered with ADCs.
FIG. 16 shows changes in blood leukocyte levels in animals administered with ADCs.
FIG. 17 shows the result of confirming the toxicity of ADC detected through analysis of blood biochemical parameters such as ALT (alanine aminotransferase), AST (aspartate aminotransferase) and blood urea nitrogen (BUN).
FIG. 18 shows the change in body weight in a single-dose rat toxicity test.
FIG. 19 shows the change in blood leukocyte level detected through hematological examination.
FIG. 20 shows the presence of toxicity expression and recovery determined based on changes in body weight and hematologic and blood biochemical changes observed after administration of a single dose to SD rats.

### [Best Mode]

Unless defined otherwise, all technical and scientific terms used herein have the same meanings as those appreciated by those skilled in the field to which the present invention pertains. In general, the nomenclature used herein is well-known in the art and is ordinarily used.

In one aspect, the present invention is directed to a compound represented by the following Formula (I) or a pharmaceutically acceptable salt or solvate thereof: 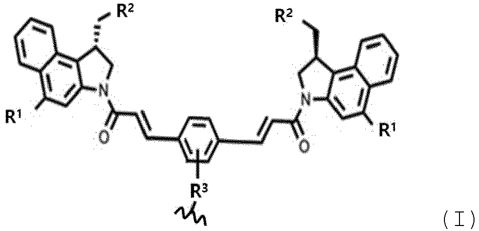 wherein
R¹ is D-galactose β-pyranose or D-galactose α-pyranose;
R² is Cl or Br; and
R³ is a single bond, -O- or -NH-.

In another aspect, the present invention is directed to a compound represented by the following Formula (II), or a pharmaceutically acceptable salt or solvate thereof: 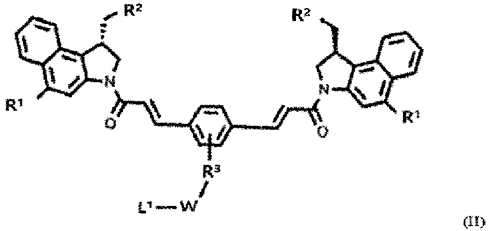 wherein R¹ is D-galactose β-pyranose or D-galactose α-pyranose;
- R²: is Cl or Br;
- R³: is a single bond, -O- or -NH-;
- W: is a spacer; and
- L¹: is a linker.

In accordance with another aspect of the present invention, there is provided an antibody-drug conjugate containing a compound represented by the following Formula (III), or a pharmaceutically acceptable salt or solvate thereof: 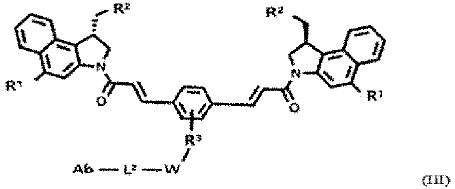 wherein
R¹ is D-galactose β-pyranose or D-galactose α-pyranose;
R² is Cl or Br;
R³ is a single bond, -O- or -NH-;
W is a spacer;
L² is a linker; and
Ab is an antibody or an antigen-binding fragment thereof.

The compounds of Formulas (I), (II) and (III) are novel compounds not previously known, the compound of Formula (I) may be used as a drug-tether combination for the preparation of antibody-drug complexes and linker-drug complexes, the compound of Formula (II) may be used as a linker-drug complex for the preparation of antibody-drug conjugates, and the compound of Formula (III) may be used as an antibody-drug conjugate or a prodrug thereof, but the present invention may not be limited thereto.

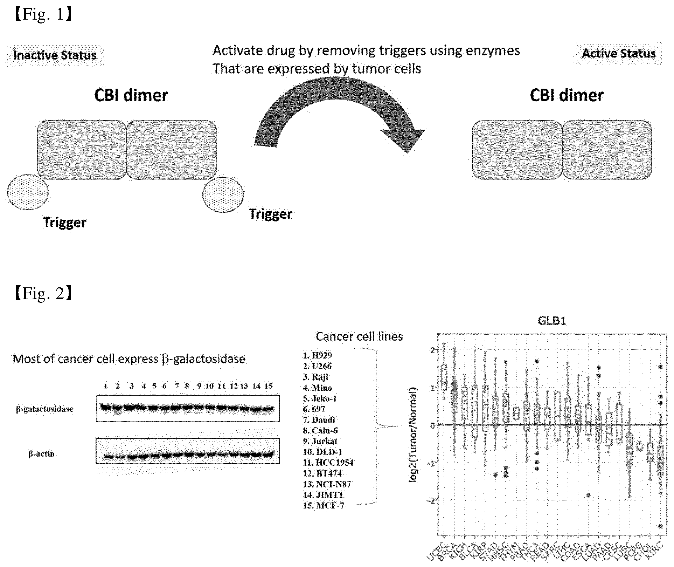

According to Formula (I), (II) or (III), R¹ represents a trigger of a prodrug, and as shown in FIG. 1, the trigger is removed through an enzyme in the cells to activate the CBI dimer, which is a cytotoxic drug. The trigger R¹ is D-galactose β-pyranose or D-galactose α-pyranose.

As used herein, the term "prodrug" refers to a compound that an inactive CBI dimer may be converted into an active CBI dimer, which exhibits activity, for example, cytotoxicity, by the trigger R¹ through enzymatic oxidation, reduction and/or hydrolysis under specific *in vivo* physiological conditions.

The present inventors found that when a phosphate group is contained as a trigger, the cytotoxic efficacy is excellent, but severe renal toxicity may be induced, which may be accompanied by irreversible renal impairment. Further, it is considered that ADCs containing phosphate groups as triggers are not stable in serum, but it is not limited to such a theory.

However, it was found by the present invention that R¹ includes D-galactose β-pyranose or D-galactose α-pyranose, which is an isomer of galactose, as a trigger, so the desired target-specific cytotoxicity can be maintained and the problem of undesired *in vivo* toxicity can be solved.

When galactose is used as a trigger, an enzyme capable of activating the galactose may be highly expressed in target cells, for example, cells with proliferative diseases, specifically cancer cells.

In one embodiment, R¹ is D-galactose β-pyranose or D-galactose α-pyranose, and in this case, the enzyme may be galactosidase, specifically beta-galactosidase. Beta-galactosidase is a lysosomal enzyme and is expressed at high levels in most cancer cells, which indicates that beta-galactosidase is considered to be a tumor-selective activating enzyme (FIG. 2).

In one embodiment, the following moiety in Formula (II) or (III) can link the CBI dimers to each other to enhance pharmacological effects, and represents a tether for stably binding the spacer W and the linker L¹ in Formula (II) or the spacer W and the linker L² in Formula (III) to a drug.

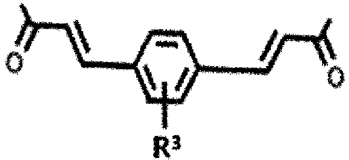

In the tether, R³ is a single bond, O- or -NH-.

Specifically, the conjugate according to the present invention may include a compound represented by the following formula:

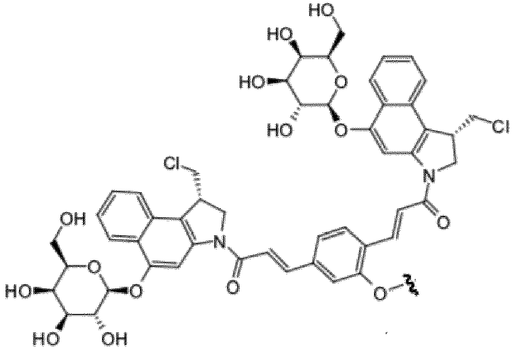

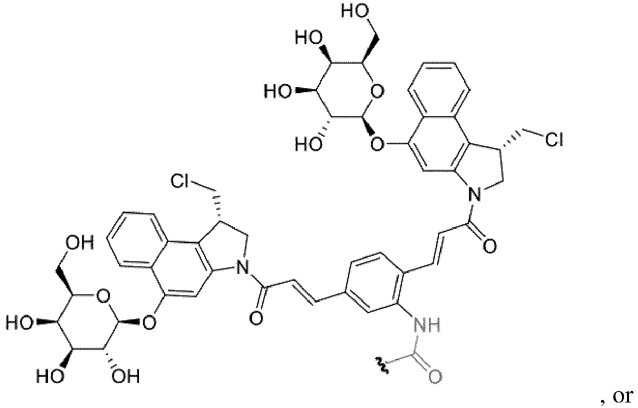

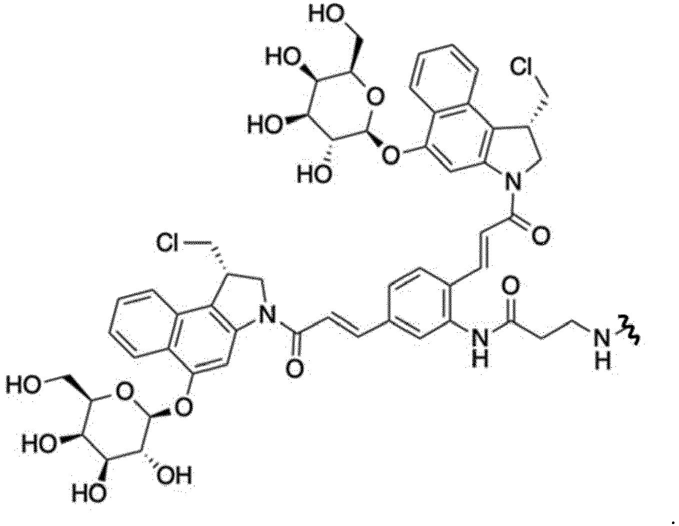

Among Formulas (II) and (III), the linker L is bound to the spacer W, and the spacer serves to maintain a sufficient distance between the antibody and the drug when the antibody is bound to deliver the drug to the target. Optionally, a hydrophilic moiety may be provided to improve solubility. In some embodiments, the term "linker" may also be referred to as including the spacer.

In one embodiment, the spacer W is -R⁴-A-R⁵-, -R⁴-A-, -(CH₂CH₂R⁶)ₓ-, -(CH₂)ᵣ(R⁷(CH₂)ₚ)_{q}-, -((CH₂)ₚR⁷)_{q}-, - (CH₂)ᵣ(R⁷(CH₂)ₚ)_{q}R⁸-, -((CH₂)ₚR⁷)_{q}(CH₂)ᵣ-, -R⁸((CH₂)ₚR⁷)_{q}- or - (CH₂)ᵣ(R⁷(CH₂)ₚ)_{q}R⁸CH₂- ,
wherein R⁴ and R⁵ are each independently - (CH₂)ᵣ(V(CH₂)ₓ)ₚ(CH₂)_{q}, A is a direct bond or a peptide bond and V is a single bond, O or S,
R⁶ is -O-, C₁-C₈ alkylene, -NR⁹- or -C(O)NR₂-,
R⁷ and R⁸ are each independently a single bond, -O-, -NR¹⁰-, -C(O)NR¹¹-, -NR¹²C(O) - or C₃-C₂₀ heteroaryl,
R⁹ to R¹² are each independently hydrogen, C₁-C₆ alkyl, (C₁-C₆ alkyl) C₆-C₂₀ aryl or (C₁-C₆ alkyl) C₃-C₂₀ heteroaryl,
X is an integer of 1 to 5, r is an integer of 0 to 10, p is an integer of 0 to 10, and q is an integer of 0 to 20, and
1 to 10 hydrogen atoms in W may be optionally substituted with hydroxy, C₁-C₈ alkyl, C₁-C₈ alkoxy, amino, ONH₂ or oxo.

In one embodiment, W in Formula (II) or Formula (III) may include the following:
(1) -R⁴-A-R⁵- or -R⁴-A-, wherein R⁴ and R⁵ are each independently -(CH₂)ᵣ(V(CH₂)ₓ)ₚ(CH₂)_{q}, A is a direct bond or peptide and V is a single bond, O or S,
   x is an integer of 1 to 5, specifically 1, 2, 3, 4 or 5,
   r is an integer of 0 to 10, specifically 0, 1, 2, 3, 4, 5, 6, 7, 8, 9 or 10,
   p is an integer of 0 to 10, specifically 1, 2, 3, 4, 5, 6, 7, 8, 9 or 10, or specifically an integer of 0 to 7, or an integer of 0 to 5,
   q is an integer of 0 to 20, specifically 1, 2, 3, 4, 5, 6, 7, 8, 9, 10, 11, 12, 13, 14 or 15, or specifically an integer of 0 to 10, an integer of 0 to 7 or an integer of 0 to 5; and
(2) a compound represented by -(CH₂CH₂R⁶)ₓ-, a compound represented by -((CH₂)ₚR⁷)_{q}(CH₂)ᵣ-, or a compound represented by -(CH₂)ᵣ(R⁷(CH₂)ₚ)_{q}R⁸CH₂-.

In this case, R⁶ is -O-, C₁-C₈ alkylene, -NR⁹- or - C (O)NR¹³-;
R⁷ and R⁸ are each independently a single bond, -O-, -NR¹⁰-, -C(O)NR¹¹-, -NR¹²C(O) - or C₃-C₂₀, specifically C₃-C₁₅, more specifically C₃-C₁₂ heteroaryl,
R⁹ to R¹³ are each independently hydrogen, C₁-C₆ alkyl, (C₁-C₆ alkyl) C₆-C₂₀ aryl, specifically (C₁-C₆ alkyl) C₃-C₁₅ aryl, more specifically, (C₁-C₆ alkyl) C₃-C₁₂ aryl, or (C₁-C₆ alkyl) C₃-C₂₀ heteroaryl, specifically (C₁-C₆ alkyl) C₃-C₁₅ heteroaryl, more specifically (C₁-C₆ alkyl) C₃-C₁₂ heteroaryl.
x is an integer of 1 to 5, specifically 1, 2, 3, 4 or 5,
r is an integer of 0 to 10, specifically 0, 1, 2, 3, 4, 5, 6, 7, 8, 9 or 10, or more specifically an integer of 0 to 7 or an integer of 0 to 5,
p is an integer of 0 to 10, specifically 1, 2, 3, 4, 5, 6, 7, 8, 9 or 10, or more specifically an integer of 0 to 7 or an integer of 0 to 5,
q is an integer of 0 to 20, specifically 1, 2, 3, 4, 5, 6, 7, 8, 9, 10, 11, 12, 13, 14 or 15, or more specifically an integer of 0 to 10, is an integer of 0 to 7 or an integer of 0 to 5.

The linker L¹ in Formula (II) or L² in Formula (III) stably binds the antibody to the drug, and when the antibody-drug conjugate reaching the target cell after circulating in the body, it enables the antibody-drug conjugate to enter the cell, the drug to be easily released therefrom through dissociation between the antibody and the drug, and provides the pharmaceutical effect on target cancer cells.

The linker L¹ in Formula (II) is hydroxy, aldehyde, ONH₂, NH₂, or 4- to 7-membered or 5- to 7-membered heteroaryl containing 1 to 3 heteroatoms selected from N, O and S, wherein the heteroaryl may be substituted with 1 to 5 substituents independently selected from hydroxy, aldehyde, C₁-C₈ alkyl, C₁-C₈ alkoxy, amino, ONH₂ and oxo.

L² in Formula (III) is -CH₂NH-, -ON=C(CH₃)-, -ON=, or 4- to 7-membered or 5- to 7-membered heterocycle containing 1 to 3 heteroatoms selected from N, O and S, wherein the heterocycle may be substituted with 1 to 5 substituents independently selected from hydroxy, aldehyde, C₁-C₈ alkyl, C₁-C₈ alkoxy, amino, ONH₂ and oxo.

Specifically, the linker L¹ or L² may include NH₂, - OH, -ONH₂ (hydroxylamine), -NH₂ aldehyde (formyl), -CO₂H, - SH, 2-formylpyridine, sulfonamide, (hetero)cyclooctyne, azide (-N₃), or maleimide.

In some cases, the linker L¹ in Formula (II) or L² in Formula (III) may include a compound represented by the following Formula (IV): 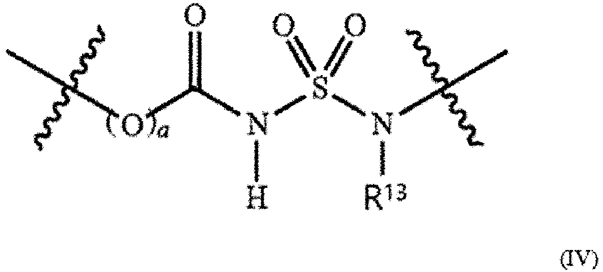
wherein a is 0 or 1,
R¹³ is selected from C₁-C₂₄ alkyl, C₃-C₂₄ cycloalkyl, C₃-C₂₄ aryl, C₃-C₂₄ heteroaryl, C₃-C₂₄ alkylaryl, C₃-C₂₄ alkylheteroaryl, C₃-C₂₄ arylalkyl and C₃-C₂₄ heteroarylalkyl, wherein the heteroaryl contains a heteroatom selected from O, S and NR¹⁴, wherein R¹⁴ is hydrogen or a C₁-C₄ alkyl group.

Specifically, the linker L¹ in Formula (II) may have a structure represented by the following Formula (I-a) or (I-b) : 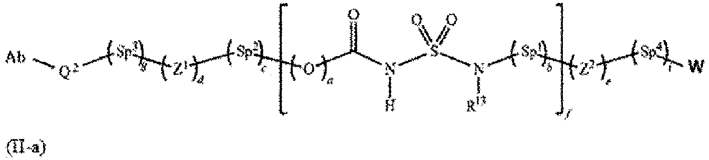 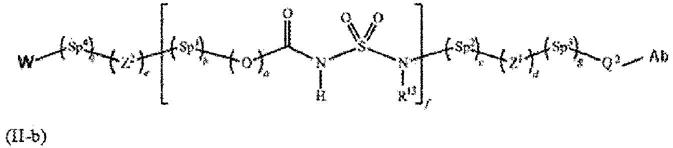 wherein Q¹ is cyclooctynyl or heterocyclooctynyl, wherein the cyclooctynyl or heterocyclooctynyl is optionally each independently fused with 1 or 2 rings selected from C₃-C₁₂ cycloalkyl, C₃-C₁₂ aryl and C₃-C₁₂ heteroaryl and is optionally substituted with hydroxy, C₁-C₈ alkyl, C₁-C₈ alkoxy, amino, ONH₂ or oxo,
R¹³ is selected from C₁-C₂₄ alkyl, C₃-C₂₄ cycloalkyl, C₃-C₂₄ aryl, C₃-C₂₄ heteroaryl, C₃-C₂₄ alkylaryl, C₃-C₂₄ alkylheteroaryl, C₃-C₂₄ arylalkyl and C₃-C₂₄ heteroarylalkyl, wherein the heteroaryl contains a heteroatom selected from O, S and NR¹⁴, wherein R¹⁴ is hydrogen or a C₁-C₄ alkyl group,
Sp¹, Sp², Sp³ and Sp⁴ are spacer moieties and are each independently selected from a single bond, or straight or branched C₁-C₂₀₀ alkylene, C₂-C₂₀₀ alkenylene, C₂-C₂₀₀ alkynylene, C₃-C₂₀₀ cycloalkylene, C₅-C₂₀₀ cycloalkenylene, C₈-C₂₀₀ cycloalkynylene, C₇-C₂₀₀ alkylarylene, C₇-C₂₀₀ arylalkylene, C₈-C₂₀₀ arylalkenylene and C₉-C₂₀₀ arylalkynylene, wherein the alkylene, alkenylene, alkynylene, cycloalkylene, cycloalkenylene, cycloalkynylene, alkylarylene, arylalkylene, aryl alkenylene and arylalkynylene are optionally substituted with or contain a heteroatom selected from O, S and NR¹⁴,
Z¹ and Z² are each independently selected from O, C(O) and N(R¹³), [139] [140] [141] [142] [143] [144] [145] [146]
   - a: is each independently 0 or 1,
   - b: is each independently 0 or 1,
   - c: is 0 or 1,
   - d: is 0 or 1,
   - e: is 0 or 1,
   - f: is an integer from 0 to 150,
   - g: is 0 or 1, and
   - i: is 0 or 1. For example, Q¹ may be selected from the following formulas: 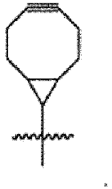 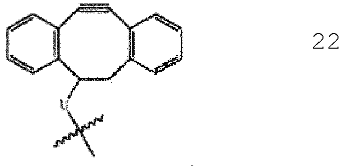
wherein U is O or NR¹⁵, wherein R¹⁵ is hydrogen, straight or branched C₁-C₁₂ alkyl, C₄-C₁₂ aryl or C₄-C₁₂ heteroaryl, and more specifically, R¹⁵ is hydrogen or C₁-C₄ alkyl.

Specifically, the linker L² in Formula (III) may have a structure represented by the following Formula (II-a) or (II-b) : 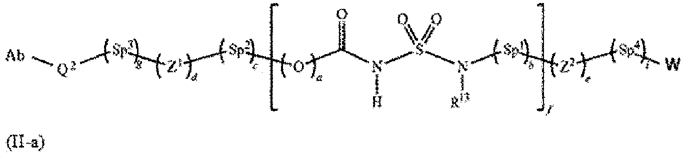 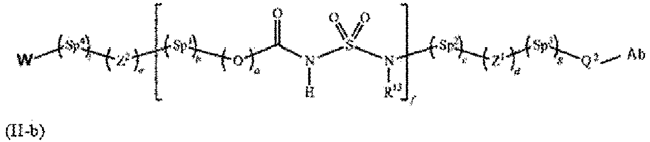 wherein Q² is cyclooctenyl fused with triazole or heterocyclooctenyl fused with triazole, wherein the cyclooctenyl or heterocyclooctenyl is optionally further fused with 1 or 2 rings each independently selected from C₃-C₁₂ cycloalkyl, C₃-C₁₂ aryl and C₃-C₁₂ heteroaryl, and is optionally substituted with hydroxy, C₁-C₈ alkyl, C₁-C₈ alkoxy, amino, ONH₂ or oxo, wherein Q² is linked to Ab through a nitrogen atom contained in the triazole;
Sp¹, Sp², Sp³ and Sp⁴ are spacer moieties and are each independently selected from a single bond, or straight or branched C₁-C₂₀₀ alkylene, C₂-C₂₀₀ alkenylene, C₂-C₂₀₀ alkynylene, C₃-C₂₀₀ cycloalkylene, C₅-C₂₀₀ cycloalkenylene, C₈-C₂₀₀ cycloalkynylene, C₇-C₂₀₀ alkylarylene, C₇-C₂₀₀ arylalkylene, C₈-C₂₀₀ arylalkenylene and C₉-C₂₀₀ arylalkynylene, wherein the alkylene, alkenylene, alkynylene, cycloalkylene, cycloalkenylene, cycloalkynylene, alkylarylene, arylalkylene, aryl alkenylene and arylalkynylene are optionally substituted with or containa heteroatom selected from O, S and NR¹⁴;
Z¹ and Z² are each independently selected from O, C(O) and N(R¹³) ;
   - a: is each independently 0 or 1;
   - b: is each independently 0 or 1;
   - c: is 0 or 1;
   - d: is 0 or 1;
   - e: is 0 or 1;
   - f: is an integer of 0 to 150;
   - g: is 0 or 1; and
   - i: is 0 or 1.

For example, Q² is cyclooctenyl fused with triazole, wherein the cyclooctenyl is further fused with C₃-C₆ cycloalkyl and/or C₃-C₆ aryl.

For example, Sp¹, Sp², Sp³ and Sp⁴, which are spacer moieties, are each independently selected from a single bond, or straight or branched C₁-C₂₀ alkylene, C₂-C₂₀ alkenylene, C₂-C₂₀ alkynylene, C₃-C₂₀ cycloalkylene, C₅-C₂₀ cycloalkenylene, C₈-C₂₀ cycloalkynylene, C₇-C₂₀ alkylarylene, C₇-C₂₀ arylalkylene, C₈-C₂₀ arylalkenylene and C₉-C₂₀ arylalkynylene, wherein the alkylene, alkenylene, alkynylene, cycloalkylene, cycloalkenylene, cycloalkynylene, alkylarylene, arylalkylene, aryl alkenylene and arylalkynylene are optionally substituted with or contain1 to 5 heteroatoms selected from O, S and NR¹⁴.

As used herein, the term "alkyl" refers to a monovalent moiety obtained by removing a hydrogen atom from a carbon atom of an aliphatic or alicyclic, saturated or unsaturated (unsaturated, fully unsaturated) hydrocarbon compound, and saturated alkyl, for example, includes methyl, ethyl, propyl, butyl, pentyl, hexyl, heptyl, or the like, saturated straight-chain alkyl, for example, includes methyl, ethyl, n-propyl, n-butyl, n-pentyl (amyl), n-hexyl, n-heptyl, or the like, and saturated branched-chain alkyl, for example, includes, isopropyl, isobutyl, sec-butyl, tert-butyl, isopentyl, neopentyl, or the like.

As used herein, the term "alkenylene" refers to a linear or branched chain monovalent hydrocarbon radical of a carbon atom having any length and having at least one unsaturated site, i.e., a carbon-carbon double bond, and the alkenyl radical may optionally be independently substituted with one or more of substituents described below and may include radicals having "cis" and "trans" orientations.

As used herein, the term "cycloalkyl" relates to a monovalent moiety obtained by removing a hydrogen atom from an alicyclic ring atom of a cyclic hydrocarbon compound. Examples of the cycloalkyl group include: saturated monocyclic hydrocarbon compounds such as cyclopropane, cyclobutane, cyclopentane, cyclohexane, cycloheptane, methylcyclopropane, dimethylcyclopropane, methylcyclobutane, dimethylcyclobutane, methylcyclopentane, dimethylcyclopentane and methylcyclohexane; and
unsaturated monocyclic hydrocarbon compounds such as cyclopropene, cyclobutene, cyclopentene, cyclohexene, methylcyclopropene, dimethylcyclopropene, methylcyclobutene, dimethylcyclobutene, methylcyclopentene, dimethylcyclopentene, and methylcyclohexene

As used herein, the term "heterocyclyl" refers to a monovalent moiety obtained by removing a hydrogen atom from a ring atom of a heterocyclic compound.

The prefix (e.g., C₁-₁₂, C₃-₈, or the like) used herein refers to the number of ring atoms or the range of the number of ring atoms, regardless of whether the substance after the prefix pertains to a carbon atom or a heteroatom. For example, the term "C₃₋₆ heterocyclyl" as used herein refers to a heterocyclyl group having 3 to 6 ring atoms.

Examples of the monocyclic heterocyclyl group include, but are not limited to, those derived from the following:
N₁: aziridine, azetidine, pyrrolidine, pyrroline, 2H- or 3H-pyrrole, piperidine, dihydropyridine, tetrahydropyridine, and azepine;
N₂: imidazolidine, pyrazolidine, imidazoline, pyrazoline, and piperazine;
O₁: oxirane, oxetane, oxolane, oxol, oxane, dihydropyran, pyran, and oxepin;
O₂: dioxolane, dioxane and dioxepane;
O₃: trioxane;
N₁O₁: tetrahydrooxazole, dihydrooxazole, tetrahydroisoxazole, dihydroisoxazole, morpholine, tetrahydrooxazine, dihydrooxazine, and oxazine;
S₁: thiirane, thietane, thiolane, thiane, and thiepane;
N₁S₁: thiazoline, thiazolidine, and thiomorpholine;
N₂O₁: oxadiazine;
O₁S₁: oxathiol and oxathiane; and
N₁O₁S₁: oxathiazine.

As used herein, the term "aryl" refers to a monovalent moiety obtained by removing a hydrogen atom from an aromatic ring atom of an aromatic compound having a ring atom. "C₆-C₂₀ aryl" refers to a moiety having 6 to 20 ring atoms, obtained by removing a hydrogen atom from an aromatic ring atom of an aromatic compound, and the prefix (C₆-C₂₀) refers to the number of ring atoms or the range of the number of ring atoms, regardless of whether the formula pertains to a carbon atom or a heteroatom, and means that the formula may include a carbon atom or at least one heteroatom.

As used herein, the term "heteroaryl" refers to an aryl containing one or more heteroatoms, and may for example include pyridine, pyrimidine, benzothiophene, furyl, dioxalanyl, pyrrolyl, oxazolyl, pyridyl, pyridazinyl, or pyrimidinyl, specifically C₉ having two fused rings derived from benzofuran, isobenzofuran, indole, isoindole, indolizine, indoline, isoindoline, purine (adenine or guanine), benzimidazole, indazole, benzoxazole, benzisoxazole, benzodioxol, benzofuran, benzotriazole, benzothiofuran, benzothiazole, or benzothiadiazole, C₁₀ having two fused rings derived from chromene, isochromene, chroman, isochroman, benzodioxane, quinoline, isoquinoline, quinolizine, benzoxazine, benzodiazine, pyridopyridine, quinoxaline, quinazoline, cinnoline, phthalazine, naphthyridine, or pteridine, C₁₁ having two fused rings derived from benzodiazepine, C₁₃ having three fused rings derived from carbazole, dibenzofuran, dibenzothiophene, carboline, perimidine, or pyridoindole, and C₁₄ having three fused rings derived from acridine, xanthene, thioxanthene, oxanthrene, phenoxathiin, phenazine, phenoxazine, phenothiazine, thianthrene, phenanthridine, phenanthroline, or phenazine.

As used herein, the term "alkoxy" means -OR [wherein R is an alkyl group], and examples thereof include methoxy, ethoxy, n-propoxy, isopropoxy, n-butoxy, sec-butoxy, isobutoxy, tert-butoxy, and the like.

As used herein, the term "alkylene" refers to a hydrocarbon compound containing a double bond, and may mean an alkylene group having 1 to 20 carbon atoms, 1 to 16 carbon atoms, 1 to 12 carbon atoms, 1 to 8 carbon atoms, or 1 to 4 carbon atoms. The alkylene group may be a straight-chain, branched, or cyclic alkylene group, and may optionally be substituted with one or more substituents.

As used herein, the term "pharmaceutically acceptable salt" may be an acid addition salt formed of a pharmaceutically acceptable free acid, and the free acid may be an organic acid or an inorganic acid.

The organic acid includes, but is not limited to, citric acid, acetic acid, lactic acid, tartaric acid, maleic acid, fumaric acid, formic acid, propionic acid, oxalic acid, trifluoroacetic acid, benzoic acid, gluconic acid, methanesulfonic acid, glycolic acid, succinic acid, 4-toluenesulfonic acid, glutamic acid, and aspartic acid. In addition, the inorganic acid includes, but is not limited to, hydrochloric acid, hydrobromic acid, sulfuric acid, and phosphoric acid.

For example, when the compound is an anion or has a functional group that may become an anion (e.g., -COOH may be converted to -COO-), it can form a salt with an appropriate cation. Examples of appropriate inorganic cations include, but are not limited to, alkali metal ions such as Na⁺ and K⁺, alkaline earth metal cations such as Ca²⁺ and Mg²⁺, and other cations such as Al³⁺. Examples of appropriate organic cations include, but are not limited to, an ammonium ion (i.e., NH₄⁺) and substituted ammonium ions (e.g., NH₃R⁺, NH₂R₂⁺, NHR₃⁺, NR₄⁺) .

Examples of some appropriate substituted ammonium ions include those derived from: ethylamine, diethylamine, dicyclohexylamine, triethylamine, butylamine, ethylenediamine, ethanolamine, diethanolamine, piperazine, benzylamine, phenylbenzylamine, choline, meglumine, and tromethamine, as well as amino acids such as lysine and arginine. A typical example of a quaternary ammonium ion is N(CH₃)⁴⁺.

When a compound is a cation or has a functional group that may become a cation (e.g., -NH₂ may be converted to - NH₃⁺) it can form a salt with an appropriate anion. Examples of appropriate inorganic anions include, but are not limited to, those derived from the following inorganic acids: hydrochloric acid, hydrobromic acid, hydroiodic acid, sulfuric acid, sulfurous acid, nitric acid, nitrous acid, phosphoric acid, and phosphorous acid.

Examples of appropriate organic anions include, but are not limited to, those derived from the following organic acids: 2-acetyloxybenzoic acid, acetic acid, ascorbic acid, aspartic acid, benzoic acid, camphorsulfonic acid, cinnamic acid, citric acid, edetic acid, ethanedisulfonic acid, ethanesulfonic acid, fumaric acid, gluheptonic acid, gluconic acid, glutamic acid, glycolic acid, hydroxymaleic acid, hydroxynaphthalene carboxylic acid, isethionic acid, lactic acid, lactobionic acid, lauric acid, maleic acid, malic acid, methanesulfonic acid, muconic acid, oleic acid, oxalic acid, palmitic acid, sulfamic acid, pantothenic acid, phenylacetic acid, phenylsulfonic acid, propionic acid, pyruvic acid, salicylic acid, stearic acid, succinic acid, sulfanilic acid, tartaric acid, toluenesulfonic acid, valeric acid, and the like. Examples of appropriate polymeric organic anions include, but are not limited to, those derived from the following polymeric acids: tannic acid, carboxymethyl cellulose, and the like.

As used herein, the term "solvate" refers to a molecular complex between the compound according to the present invention and solvent molecules, and for example, the solvate includes, but is not limited to, the compound according to the present invention combined with water, isopropanol, ethanol, methanol, dimethyl sulfoxide, ethyl acetate, acetic acid, ethanolamine, or a mixed solvent thereof.

Optionally, amino acids or peptides such as dipeptides may be further included. Specifically, the dipeptide such as Val-Cit, Phe-Cit, Phe-Lys, Val-Lys, Val-Glu, Val-Asp, Val-Ser or Val-Gly may be included. Val-Cit dipeptide linkers are preferred. When a single amino acid is included, for example, Cit, Glu, Lys or Ser may be included. A linker including such an amino acid or peptide is cleavable by cathepsin B.

Specifically, the compound of Formula (II) may be represented by the following formula:

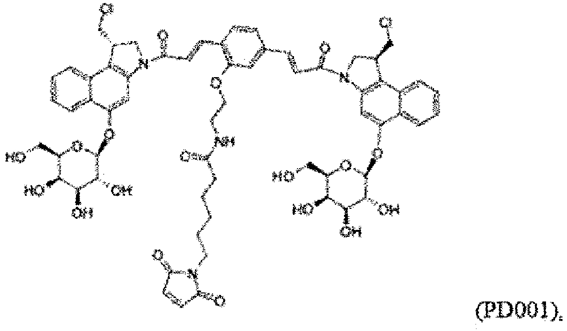

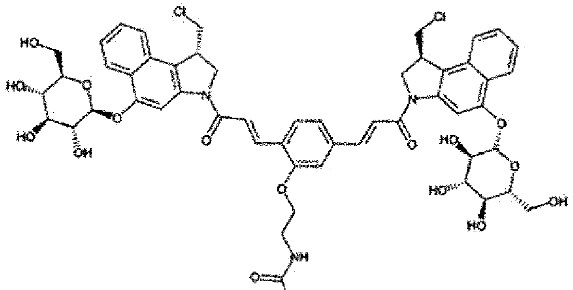

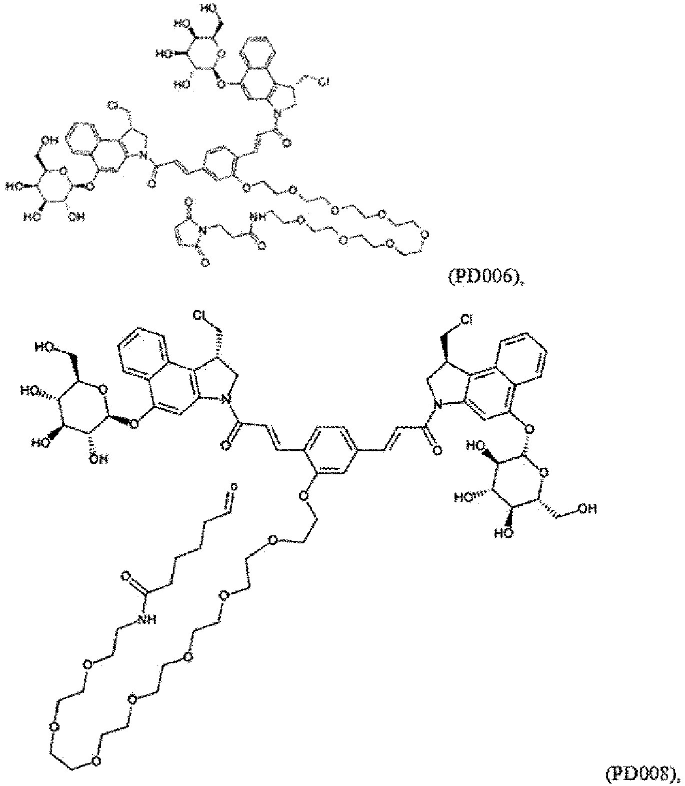

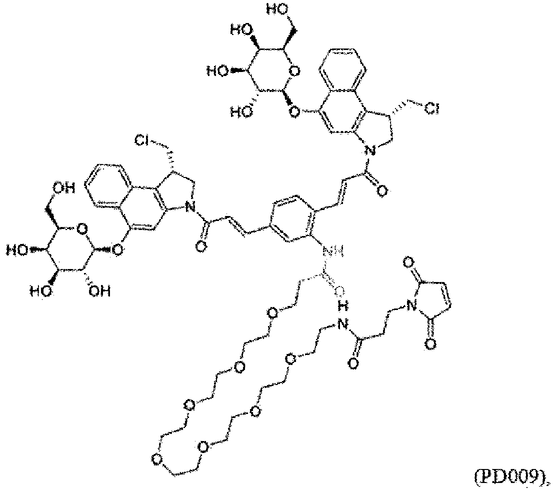

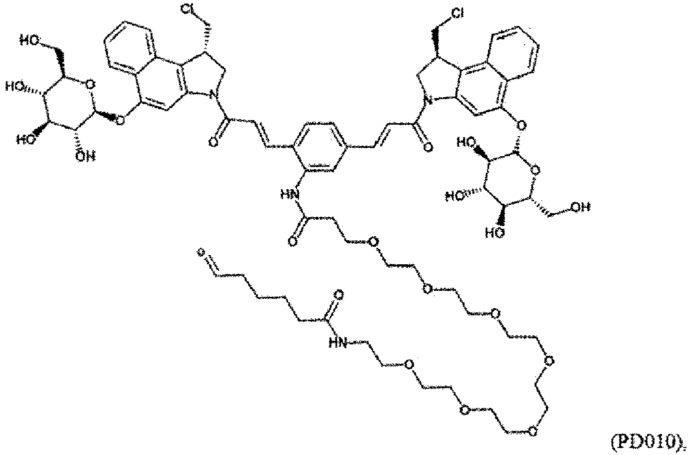

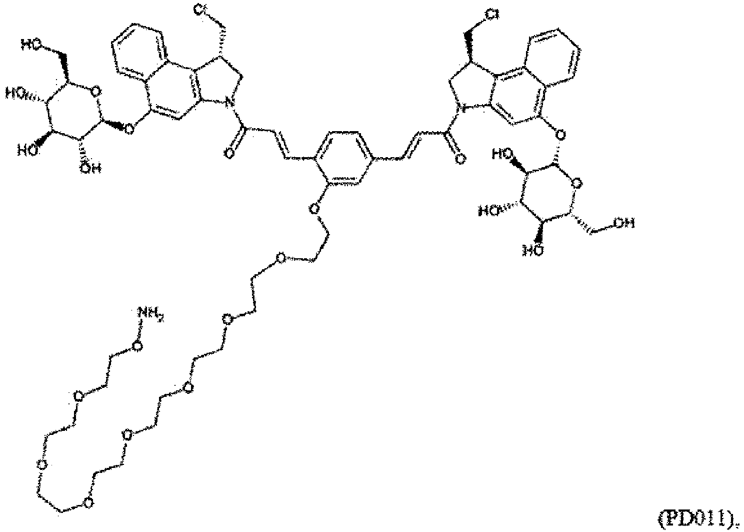

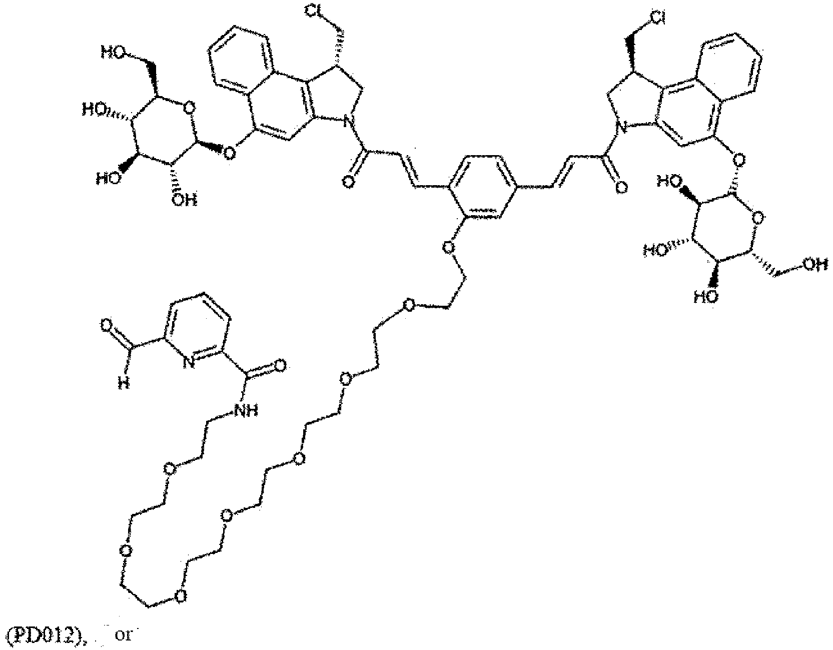

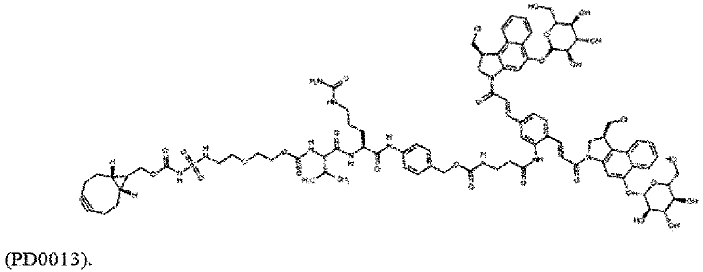

The compound of Formula (II) according to the present invention may be a linker-drug complex having a linker moiety that binds with an antibody to form an antibody-drug conjugate. In one embodiment, the linker may include a spacer.

The linker of the compound of Formula (II) according to the present invention may have a maleimide, aldehyde, aminooxyl, 2-PCA, or cyclooctyne group for binding to a drug, but is not limited thereto.

For example, when the linker of the compound of Formula (II) according to the present invention includes a maleimide group, the linker may be linked to the antibody through a cysteine amino acid introduced into the antibody for the formation of the antibody-drug conjugate according to the present invention. For example, the cysteine amino acid may be engineered and available for drug conjugation, but may be bound via THIOMAB^{™} antibodies, which are antibodies substituted with cysteines at positions that do not impede the folding of the antibody and do not alter antigen binding or effector functions. It may be conjugated to cytotoxic drugs via engineered cysteine thiol groups, so THIOMAB^{™} antibody-drug conjugates (TDCs) having a uniform stoichiometry (e.g., containing up to 2 drugs per antibody in an antibody with a single engineered cysteine) can be obtained.

In addition to the THIOMAB^{™} antibody, any antibody containing a cysteine amino acid can be bound to the compound of Formula (II), having a maleimide linker, through the following Michael reaction to produce an antibody-drug conjugate.

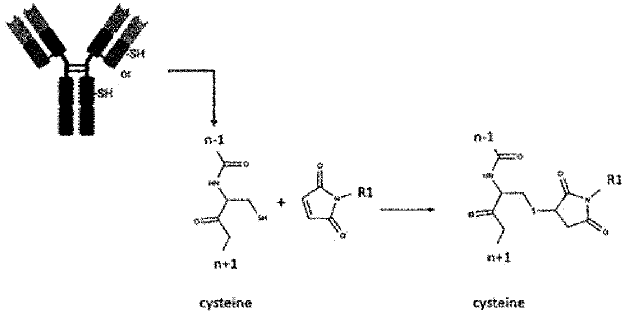

For example, engineered cysteines may be present at different positions for drug attachment, such as at specific amino acid positions within the light chain-Fab, heavy chain-Fab or heavy chain-Fc of the antibody.

Cysteine substitutions in the heavy chain are, for example, selected from the group consisting of Y33C, G162C, V184C, I195C, S420C, Y432C, Q434C, R19C, E46C, T57C, Y59C, A60C, M100cC, W103C, G162C, I195C, V258C, S420C, H425C, and N430C according to Kabat numbering. Cysteine substitutions in the light chain are, for example, selected from the group consisting of Y55C, G64C, T85C, T180C, N430C, T31C, S52C, G64C, R66C, A193C and N430C according to Kabat numbering, or may be optionally selected from the group consisting of LC-I106C, LC-R108C, LC-R142C, and LC-K149C.

For example, when the linker of the compound of Formula (II) according to the present invention includes an aldehyde group, the linker can be bound to the antibody through the following reductive alkylation between the N-terminus of the antibody protein or NH₂ of the lysine amino acid and the aldehyde in the linker for production of the antibody-drug conjugate according to the present invention.

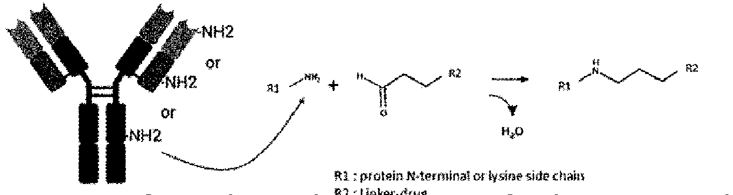

For example, when the linker of the compound of Formula (II) according to the present invention includes an aminooxyl group, the linker can be bound to the antibody through the following oxime ligation between the ketone group in the antibody amino acid and the aminooxyl group in the linker for the production of the antibody-drug conjugate according to the present invention.

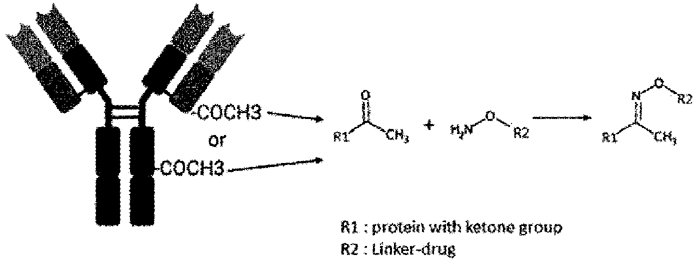

For example, when the linker of the compound of Formula (II) according to the present invention includes 2-pyridinecarboxaldehyde (2-PCA), the linker can be bound to the antibody through the following N-terminal imidazolidinone formation between NH₂ in the antibody amino acid and 2-PCA of the linker for the production of the antibody-drug conjugate according to the present invention.

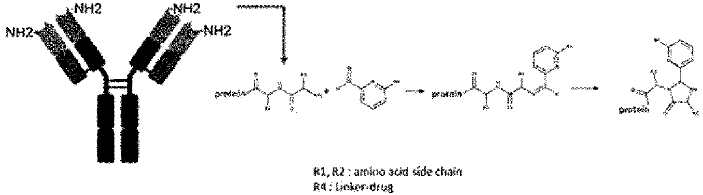

For example, when the linker of the compound of Formula (II) according to the present invention contains a cyclooctyne group, it is modified to have an azide group (-N₃) for production of the antibody-drug conjugate according to the present invention. The linker can be bound to the antibody through the following click-reaction between the azide group in the antibody and the cyclooctyne group in the linker.

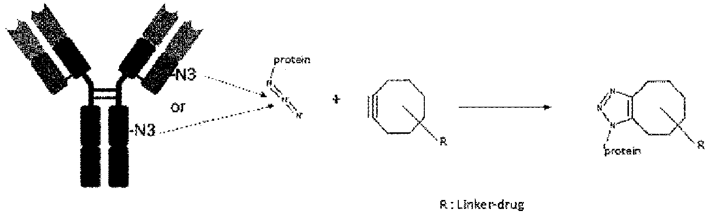

The types of linkers described above and the specific reaction for forming an antibody-drug conjugate by conjugating each linker with an antibody are already known in the art, and can be easily implemented by those skilled in the art without undue effort. In addition to the above linkers, various linkers which are known in the art and can be used by those skilled in the art may be used for the production of the antibody-drug conjugate of the present invention, and the structure of the antibody-drug conjugate bound with such a linker is also easily conceived by those skilled in the art.

The antibody used herein recognizes an antigen that is natively expressed or overexpressed by target cells, e.g., cancer cells, and can function as a targeting agent to deliver drug moieties to cancer cells with a high degree of specificity. When the antibody binds to the antigen, the antigen-conjugate forms a complex, is internalized, and ultimately enters the lysosome, and the linker between the drug moiety and the antibody is cleaved to release the drug moiety and thereby to provide a cytotoxic effect.

The antibody may bind, for example, to the following antigens, but is not limited thereto:
(1) BMPR1B (bone morphogenetic protein receptor-type IB, GenBank Accession No. NM_001203);
(2) E16 (LAT1, SLC7A5, GenBank Accession No. NM_003486);
(3) STEAP1 (Six-transmembrane epithelial antigen of prostate 1, GenBank Accession No. NM_012449);
(4) 0772P (CA125, MUC16, GenBank Accession No. AF361486);
(5) MPF (MPF, MSLN, SMR, megakaryocyte-enhancing factor, mesothelin, GenBank Accession No. NM-005823);
(6) Napi3b (NAPI-3B, NPTIIb, SLC34A2, solute carrier family 34 (sodium phosphate), member 2, type II sodium-dependent phosphate transporter 3b, GenBank Accession No. NM_006424);
(7) Sema 5b (FLJ10372, KIAA1445, Mm.42015, SEMA5B, SEMAG, semaphorin 5b Hlog, sema domain, 7 thrombospondin repeats (type 1 and pseudotype 1), transmembrane domain (TM) and a short cytoplasmic domain, (Semaphorin) 5B, GenBank Accession No. AB040878);
(8) PSCA hlg (2700050C12Rik, C530008016Rik, RIKEN cDNA 2700050C12, RIKEN cDNA 2700050C12 gene, GenBank Accession No. AY358628);
(9) ETBR (endothelin type B receptor, GenBank Accession No. AY275463);
(10) MSG783 (RNF124, hypothetical protein FLJ20315, GenBank Accession No. NM_017763);
(11) STEAP2 (HGNC_8639, IPCA-1, PCANAP1, STAMP1, STEAP2, STMP, prostate cancer-related gene 1, prostate cancer-related protein 1, prostate 6-transmembrane epithelial antigen 2, 6-transmembrane prostate protein, GenBank Accession No. AF455138);
(12) TrpM4 (BR22450, FLJ20041, TRPM4, TRPM4B, transient receptor potential cation channel, subfamily M, member 4, GenBank Accession No. NM_017636);
(13) CRIPTO (CR, CR1, CRGF, CRIPTO, TDGF1, teratocarcinoma-derived growth factor, GenBank Accession No. NP_003203 or NM_003212);
(14) CD21 (CR2 (Complement Receptor 2), C3DR (C3d/Epstein Barr Virus Receptor), or Hs.73792 GenBank Accession No. M26004);
(15) CD79b (CD79B, CD79β, IGb (immunoglobulin-associated beta), B29, GenBank Accession No. NM_000626);
(16) FcRH2 (IFGP4, IRTA4, SPAP1A (SH2 domain-containing phosphatase anchoring protein 1a), SPAP1B, SPAP1C, GenBank Accession No. NM_030764);
(17) HER2 (GenBank Accession No. M11730);
(18) ErbB receptor selected from EGFR, HER3 and HER4;
(19) NCA (GenBank Accession No. M18728);
(20) MDP (GenBank Accession No. BC017023);
(21) IL20Rα (GenBank Accession No. AF184971);
(22) Brevican (GenBank Accession No. AF229053);
(23) EphB2R (GenBank Accession No. NM_004442);
(24) ASLG659 (GenBank Accession No. AX092328);
(25) PSCA (GenBank Accession No. AJ297436);
(26) GEDA (GenBank Accession No. AY260763);
(27) BAFF-R (B-cell activating factor receptor, BLyS receptor 3, BR3, NP_443177.1);
(28) CD22 (B-cell receptor CD22-B isoform, NP-001762.1);
(29) CD79a (CD79A, CD79α, immunoglobulin-associated alpha, which are B cell-specific proteins that covalently interact with Ig beta (CD79B), form a complex on the surface with IgM, and transmit signals involved in B-cell differentiation, GenBank Accession No. NP_001774.1);
(30) CXCR5 (Burkitt's lymphoma receptor 1, which is a G-protein-coupled receptor activated by the CXCL13 chemokine, is considered to act on lymphocyte migration and humoral defense, participate in HIV-2 infection, and be related to the onset of AIDS, lymphoma, myeloma and leukemia, GenBank Accession No. NP_001707.1);
(31) HLA-DOB (beta subunit of MHC class II molecule (Ia antigen), which binds to peptide and presents to CD4+ T lymphocytes, GenBank Accession No. NP-002111.1);
(32) P2X5 (purinergic receptor P2X ligand-gated ion channel 5, which is an ion channel gated by extracellular ATP, may be involved in synaptic transmission and neurogenesis, and deficiency of which may contribute to the pathophysiology of idiopathic detrusor instability. GenBank Accession No. NP_002552.2);
(33) CD72 (B-cell differentiation antigen CD72, Lyb-2, GenBank Accession No. NP_001773.1);
(34) LY64 (lymphocyte antigen 64 (RP105), which is a type I membrane protein of the leucine-rich repeat (LRR) family, modulates B-cell activation and apoptosis, and loss of function of which is associated with increased disease activity of systemic lupus erythematosus patients), GenBank Accession No. NP_005573.1);
(35) FcRH1 (Fc receptor homolog protein 1, which is a putative receptor for an immunoglobulin Fc domain containing C2-type Ig-like and ITAM domains, and may be involved in B lymphocyte differentiation, GenBank Accession No. NP_443170.1);
(36) IRTA2 (immunoglobulin superfamily receptor translocation-associated 2, which is a putative immunoreceptor that may act on B-cell genesis and lymphomagenesis, and gene deregulation by translocation occurs in several B-cell malignancies, GenBank Accession No. NP_112571.1); and
(37) TENB2 (a putative transmembrane proteoglycan associated with the EGF/heregulin family of growth factors and follistatin, GenBank Accession No. AF179274);
(38) MAGE-C1/CT7 (protein overexpressed in testicular cancer);
(39) Androgen receptor, PTEN, human kallikrein-related peptidase 3 (protein overexpressed in prostate cancer);
(40) CD20;
(41) CD30;
(42) CD33;
(43) CD52;
(44) EpCam;
(45) CEA;
(46) gpA33;
(47) mucins;
(48) TAG-72;
(49) Carbonic anhydrase IX;
(50) PSMA;
(51) Folate receptor (which is a family of proteins expressed by the FOLR gene, having high affinity for folic acid, and transporting 5-methyltetrahydrofolate into cells);
(52) Gangliosides (GD2, GD3, GM2);
(53) glycohydrate Lewis-Y;
(54) VEGF;
(55) VEGFR;
(56) aVb3;
(57) a5b1;
(58) ERB3;
(59) c-MET;
(60) EphA3;
(61) TRAIL-R1 and TRAIL-R2;
(62) RANKL;
(63) FAP;
(64) tenascin;
(65) ROR1;
(66) BCMA; or
(67) CLL1.

The antibody may be, for example, selected from the group consisting of an anti-BCMA antibody, an anti-RORl antibody, an anti-Her2 antibody, an anti-NaPi2b antibody, and an anti-CLLl antibody, but is not limited thereto. In a specific example, the following antibodies were used as the anti-BCMA antibody and the anti-RORl antibody for ADC construction.

**[Table 1]**

| Type | | Sequence | | No. |
|---|---|---|---|---|
| Anti-BCMA antibo dy | B58 | L-CDR1 | SGSSSNIGSNSVS | 1 |
| | | L-CDR2 | ADSKRPS | 2 |
| | | L-CDR3 | GSWDYSLSGYV | 3 |
| | | VL | QSVLTQPPSASGTPGQRVTISCSGSSS NIGSNSVSWYQQLPGTAPKLLIYADSK RPSGVPDRFSGSKSGTSASLAISGLRS EDEADYYCGSWDYSLSGYVFGGGTKLT VLGQPKAAPSVTLFPPSSEELQANKAT LVCLISDFYPGAVTVAW(C)ADSSPVK AGVETTTPSKQSNNKYAASSYLSLTPE QWKSHRSYSCQVTHEGSTVEKTVAPTE CS | 4 |
| | | H-CDR1 | NYDMS | 5 |
| | | H - CDR2 | WIYPSDSSIYYADSVKG | 6 |
| | | H-CDR3 | RGPFANKYRQFDY | 7 |
| | | VH | EVQLLESGGGLVQPGGSLRLSCAASGF TFSNYDMSWVRQAPGKGLEWVSWIYPS DSSIYYADSVKGRFTISRDNSKNTLYL QMNSLRAEDTAVYYCARGPFANKYRQF DYWGQGTLVTVSSASTKGPSVFPLAPS SKSTSGGTAALGCLVKDYFPEPVTVSW NSGALTSGVHTFPAVLQSSGLYSLSSV VTVPSSSLGTQTYICNVNHKPSNTKVD KKVEPKSCDKTHTCPPCPAPELLGGPS VFLFPPKPKDTLMISRTPEVTCVVVDV SHEDPEVKFNWYVDGVEVHNAKTKPRE EQYNSTYRVVSVLTVLHQDWLNGKEYK CKVSNKALPAPIEKTISKAKGQPREPQ VYTLPPSREEMTKNQVSLTCLVKGFYP SDIAVEWESNGQPENNYKTTPPVLDSD GSFFLYSKLTVDKSRWQQGNVFSCSVM HEALHNHYTQKSLSLSPGK | 8 |
| Anti-ROR1 antibo dy | C2E3 | L-CDR1 | TGSSSNIGSNDVT | 9 |
| | | L-CDR2 | ADSKRPS | 10 |
| | | L-CDR3 | GTWDYSLSGYV | 11 |
| | | VL | QSVLTQPPSASGTPGQRVTISCTGSSS NIGSNDVTWYQQLPGTAPKLLIYADSK RPSGVPDRFSGSKSGTSASLAISGLRS EDEADYYCGTWDYSLSGYVFGGGTKLT VLGQPKAAPSVTLFPPSSEELQANKAT LVCLISDFYPGAVTVAW(C)ADSSPVK AGVETTTPSKQSNNKYAASSYLSLTPE QWKSHRSYSCQVTHEGSTVEKTVAPAE CS | 12 |
| | | H-CDR1 | NYAMS | 13 |
| | | H - CDR2 | SISHNSGSTYYADSVKG | 14 |
| | | H-CDR3 | FISARKSLGRSYSNGMDV | 15 |
| | | VH | EVQLLESGGGLVQPGGSLRLSCAASGF TFSNYAMSWVRQAPGKGLEWVSSISHN SGSTYYADSVKGRFTISRDNSKNTLYL | 16 |
| | | | QMNSLRAEDTAVYYCAKFISARKSLGR SYSNGMDVWGQGTLVTVSSASTKGPSV FPLAPSSKSTSGGTAALGCLVKDYFPE PVTVSWNSGALTSGVHTFPAVLQSSGL YSLSSVVTVPSSSLGTQTYICNVNHKP SNTKVDKKVEPKSCDKTHTCPPCPAPE LLGGPSVFLFPPKPKDTLMISRTPEVT CVVVDVSHEDPEVKFNWYVDGVEVHNA KTKPREEQYNSTYRVVSVLTVLHQDWL NGKEYKCKVSNKALPAPIEKTISKAKG QPREPQVYTLPPSREEMTKNQVSLTCL VKGFYPSDIAVEWESNGQPENNYKTTP PVLDSDGSFFLYSKLTVDKSRWQQGNV FSCSVMHEALHNHYTQKSLSLSPGK | |
| * It may be substituted with a cysteine at position 149 of the VL (according to Kabat numbering) | | | | |

The known sequence of trastuzumab was used for the anti-Her2 antibody, the known sequences of 10H1 antibody (10H1 VL: SEQ ID NO: 17, 10H1 VH: SEQ ID NO: 18) were used for the anti-NaPi2b antibody, and the known sequences of 6E7(N54A) antibody (6E7(N54A) VL: SEQ ID NO: 18, 6E7(N54A) VH: SEQ ID NO: 19) were used for the anti-CLL1 antibody.

As used herein, the term "antibody" refers to a polypeptide or protein that specifically binds to a specific antigen. The antibody includes not only a complete antibody specifically binding to an antigen but also an antigen-binding fragment of the antibody.

The term "complete antibody" refers to a structure having two full-length light chains and two full-length heavy chains, wherein each light chain is linked to a corresponding heavy chain by a disulfide bond. The heavy-chain constant region has gamma (γ), mu (µ) , alpha (α), delta (δ) and epsilon (ε) types, and is subclassified into gamma 1 (γ1), gamma 2 (γ2), gamma 3 (γ3), gamma 4 (γ4), alpha 1 (α1) and alpha 2 (α2). The light-chain constant region has kappa (κ) and lambda (λ) types.

The antigen-binding fragment of an antibody or antibody fragment is a fragment that has antigen-binding capacity and includes Fab, F(ab'), F(ab')2, Fv and the like. Among the antibody fragments, Fab refers to a structure including a variable region of each of the heavy chain and the light chain, the constant region of the light chain, and the first constant domain (CH1) of the heavy chain, each having one antigen-binding site. Fab' is different from Fab in that it further includes a hinge region including at least one cysteine residue at the C-terminus of the CH1 domain of the heavy chain. F(ab')2 is created by a disulfide bond between cysteine residues in the hinge region of Fab'. Fv is the minimal antibody fragment having only a heavy-chain variable region and a light-chain variable region. Two-chain Fv is a fragment wherein the variable region of the heavy chain and the variable region of the light chain are linked by a non-covalent bond, and single-chain Fv (scFv) is a fragment wherein the variable region of the heavy chain and the variable region of the light chain are generally linked by a covalent bond via a peptide linker therebetween, or are directly linked at the C-terminal, forming a dimer-shaped structure, like the two-chain Fv. Such antibody fragments may be obtained using proteases (e.g., Fab can be obtained by restriction-cleaving the complete antibody with papain, and the F(ab')2 fragment can be obtained by cleaving the complete antibody with pepsin), and may be also prepared using genetic recombination techniques.

The heavy-chain constant region may be selected from gamma (γ), mu (u), alpha (α), delta (δ), and epsilon (c) isotypes. For example, the constant region may be gamma 1 (IgG1), gamma 3 (IgG3), or gamma 4 (IgG4). The light-chain constant region may be kappa or lambda.

As used herein, the term "heavy chain" encompasses both a full-length heavy chain, which includes a variable domain (VH) containing an amino acid sequence having a variable region sequence sufficient to impart specificity to an antigen, three constant domains (CH1, CH2 and CH3), and a fragment thereof. As used herein, the term "light chain" encompasses both a full-length light chain, which includes a variable domain (VL) containing an amino acid sequence having a variable region sequence sufficient to impart specificity to an antigen, a constant domain (CL), and a fragment thereof.

The antibody of the present invention includes, but is not limited to, monoclonal antibodies, human antibodies, humanized antibodies, chimeric antibodies, single-chain Fvs (scFVs), single-chain antibodies, Fab fragments, F(ab') fragments, disulfide-bond Fvs (sdFVs), epitope-binding fragments of such antibodies, and the like.

The term "monoclonal antibody" refers to an identical antibody, which is obtained from a population of substantially homogeneous antibodies, that is, each antibody constituting the population, excluding possible naturally occurring mutations that may be present in trivial amounts. Monoclonal antibodies are highly specific and are thus induced against a single antigenic site. Unlike conventional (polyclonal) antibody preparations that typically include different antibodies directed against different determinants (epitopes), each monoclonal antibody is directed against a single determinant on the antigen.

The term "epitope" refers to a protein determinant to which an antibody can specifically bind. Epitopes usually consist of a group of chemically active surface molecules, such as amino acid or sugar side chains, and generally have not only specific three-dimensional structural characteristics but also specific charge characteristics. Three-dimensional epitopes are distinguished from non-three-dimensional epitopes in that a bond to the former is broken in the presence of a denatured solvent, while a bond to the latter is not broken.

The non-human (e.g., murine) antibody of the "humanized" form is a chimeric antibody containing minimal sequences derived from non-human immunoglobulins. In most cases, the humanized antibody is a human immunoglobulin (receptor antibody) in which a residue from the hypervariable region of a receptor is replaced with a residue from the hypervariable region of a non-human species (donor antibody) such as a mouse, rat, rabbit or non-human primate having the desired specificity, affinity and ability.

As used herein, the term "human antibody" refers to a molecule derived from human immunoglobulin, in which all of the amino acid sequences constituting the antibody including a complementarity-determining region and a structural region are composed of human immunoglobulins.

A part of the heavy chain and/or light chain is identical to or homologous with the corresponding sequence in an antibody derived from a particular species or belonging to a particular antibody class or subclass, while the remaining chain(s) includes "chimeric" antibodies (immunoglobulins) which are identical to or homologous with corresponding sequences in an antibody derived from another species or belonging to another antibody class or subclass, as well as fragments of such antibody exhibiting the desired biological activity.

As used herein, the term "antibody variable domain" refers to the light- and heavy-chain regions of an antibody molecule including the amino acid sequences of a complementarity-determining region (CDR; i.e., CDR1, CDR2, and CDR3) and a framework region (FR). VH refers to a variable domain of the heavy chain. VL refers to a variable domain of the light chain.

The term "complementarity-determining region" (CDR, that is, CDR1, CDR2, and CDR3), refers to an amino acid residue of the antibody variable domain, which is necessary for antigen binding. Each variable domain typically has three CDR regions, identified as CDR1, CDR2, and CDR3.

The term "framework region" (FR) refers to a variable domain residue other than a CDR residue. Each variable domain typically has four FRs, identified as FR1, FR2, FR3, and FR4.

The antibody or antigen-binding fragment of the present invention may include the sequence of the antibody mentioned herein as well as biological equivalents thereto. For example, additional variations can be made to the amino acid sequence of the antibody in order to further improve the binding affinity and/or other biological properties of the antibody. Such variations include, for example, deletions, insertions and/or substitutions of amino acid sequence residues of the antibody. Such amino acid variations are based on the relative similarity of amino-acid side-chain substituents, such as the hydrophobicity, hydrophilicity, charge, and size thereof. It can be seen through analysis of the size, shape and type of amino-acid side-chain substituents that all of arginine, lysine, and histidine are positively charged residues, alanine, glycine, and serine have similar sizes, and phenylalanine, tryptophan, and tyrosine have similar shapes. Thus, based on these considerations, arginine, lysine, and histidine are considered to be biologically functional equivalents, alanine, glycine, and serine are considered to be biologically functional equivalents, and phenylalanine, tryptophan, and tyrosine are considered to be biologically functional equivalents.

Taking into consideration variations having biologically equivalent activity, the antibody or a nucleotide molecule encoding the same according to the present invention is interpreted to include a sequence having substantial identity with the sequence set forth in the sequence number. The term "substantial identity" means that a sequence has a homology of at least 90%, most preferably a homology of at least 95%, at least 96%, at least 97%, at least 98%, or at least 99%, when aligning the sequence of the present invention with any other sequence so as to correspond thereto as closely as possible and analyzing the aligned sequence using algorithms commonly used in the art. Alignment methods for sequence comparison are well-known in the art. The NCBI Basic Local Alignment Search Tool (BLAST) is accessible through NCBI, and can be used in conjunction with sequence analysis programs such as BLASTP, BLASTM, BLASTX, TBLASTN and TBLASTX over the Internet. BLAST is available at www.ncbi.nlm.nih.gov/BLAST/. A method of comparing sequence homology using this program can be found at www.ncbi.nlm.nih.gov/BLAST/blast_help.html.

Based on this, the antibody or antigen-binding fragment thereof according to the present invention can have homology of 90%, 91%, 92%, 93%, 94%, 95%, 96%, 97%, 98%, 99%, or more with the sequence disclosed herein or the entirety thereof. Homology can be determined through sequence comparison and/or alignment by methods known in the art. For example, the percentage sequence homology of the nucleic acid or protein according to the present invention can be determined using a sequence comparison algorithm (i.e., BLAST or BLAST 2.0), manual alignment, or visual inspection.

In some cases, by isolating the nucleic acid encoding the antibody or antigen-binding fragment thereof according to the present invention, an antibody or antigen-binding fragment thereof can be produced through recombination. The nucleic acid is isolated and inserted into a replicable vector, followed by further cloning (amplification of DNA) or further expression. Based thereon, in another aspect, the present invention is directed to a vector including the nucleic acid.

The term "nucleic acid" is intended to encompass both DNA (gDNA and cDNA) and RNA molecules, and a nucleotide, which is the basic constituent unit of nucleic acids, includes naturally derived nucleotides as well as analogues thereof, in which sugar or base moieties are modified. The sequence of the nucleic acid encoding heavy- and light-chain variable regions of the present invention can vary. Such variations include additions, deletions, or non-conservative or conservative substitutions of nucleotides.

The DNA encoding the antibody can be easily separated or synthesized using conventional procedures (for example, using an oligonucleotide probe capable of specifically binding to DNA encoding heavy and light chains of the antibody) . A variety of vectors are obtainable. Vector components generally include, but are not limited to, one or more of the following components: signal sequences, replication origins, one or more marker genes, enhancer elements, promoters, and transcription termination sequences.

As used herein, the term "vector" refers to a means for expressing target genes in host cells, and includes plasmid vectors, cosmid vectors, and viral vectors such as bacteriophage vectors, adenovirus vectors, retroviral vectors, and adeno-associated viral vectors. The nucleic acid encoding the antibody in the vector is operably linked to a promoter.

The term "operably linked" means functional linkage between a nucleic acid expression regulation sequence (e.g., an array of the binding site of the promoter, signal sequence, or transcription regulator) and another nucleic acid sequence, and enables the regulation sequence to regulate transcription and/or translation of the other nucleic acid sequence.

When a prokaryotic cell is used as a host, it generally includes a potent promoter capable of conducting transcription (such as a tac promoter, lac promoter, lacUV5 promoter, lpp promoter, pLλ promoter, pRλ promoter, rac5 promoter, amp promoter, recA promoter, SP6 promoter, trp promoter, or T7 promoter), a ribosome-binding site for initiation of translation, and a transcription/translation termination sequence. In addition, for example, when a eukaryotic cell is used as a host, it includes a promoter derived from the genome of a mammalian cell (e.g., a metallothionein promoter, a β-actin promoter, a human hemoglobin promoter or a human muscle creatine promoter), or a promoter derived from a mammalian virus (e.g., an adenovirus late promoter, vaccinia virus 7.5K promoter, SV40 promoter, cytomegalovirus (CMV) promoter, HSV tk promoter, mouse mammary tumor virus (MMTV) promoter, HIV LTR promoter, Moloney virus promoter, Epstein-Barr virus (EBV) promoter, or Rous sarcoma virus (RSV) promoter), and generally has a polyadenylation sequence as a transcription termination sequence.

Optionally, the vector may be fused with another sequence in order to facilitate purification of the antibody expressed thereby. The sequence to be fused therewith may include, for example, glutathione S-transferase (Pharmacia, USA), maltose-binding protein (NEB, USA), FLAG (IBI, USA), 6x His (hexahistidine; Qiagen, USA) and the like.

The vector includes antibiotic resistance genes commonly used in the art as selectable markers, and examples thereof include genes conferring resistance to ampicillin, gentamycin, carbenicillin, chloramphenicol, streptomycin, kanamycin, geneticin, neomycin, and tetracycline.

In another aspect, the present invention is directed to a cell transformed with the above-mentioned vector. The cell used to produce the antibody of the present invention may be a prokaryote, yeast, or higher eukaryotic cell, but is not limited thereto.

Prokaryotic host cells such as *Escherichia coli,* strains of the genus *Bacillus,* such as *Bacillus subtilis* and *Bacillus thuringiensis, Streptomyces* spp., *Pseudomonas* spp. (for example, *Pseudomonas putida*)*, Proteus mirabilis* and *Staphylococcus* spp. (for example, *Staphylococcus carnosus*) may be used.

Interest in animal cells is the greatest, and examples of useful host cell lines include, but are not limited to, COS-7, BHK, CHO, CHOK1, DXB-11, DG-44, CHO/-DHFR, CV1, COS-7, HEK293, BHK, TM4, VERO, HELA, MDCK, BRL 3A, W138, Hep G2, SK-Hep, MMT, TRI, MRC 5, FS4, 3T3, RIN, A549, PC12, K562, PER.C6, SP2/0, NS-0, U20S, and HT1080.

The cells can be cultured in various media. Any commercially available medium can be used as a culture medium without limitation. All other essential supplements well-known to those skilled in the art may be included in appropriate concentrations. Culture conditions such as temperature and pH are those that are conventionally used with host cells selected for expression, as will be apparent to those skilled in the art.

The recovery of the antibody or antigen-binding fragment thereof can be carried out, for example, by centrifugation or ultrafiltration to remove impurities from and purify the resulting product using, for example, affinity chromatography. Other additional purification techniques, such as anion or cation exchange chromatography, hydrophobic interaction chromatography, and hydroxyapatite chromatography, may be used.

In another aspect, the present invention is directed to a composition for preventing or treating a proliferative disease, for example, a tumor or cancer, or a pharmaceutical composition containing the antibody-drug conjugate as an active ingredient.

In another aspect, the present invention is directed to a pharmaceutical composition for preventing or treating a proliferative disease, for example, a tumor or cancer, containing (a) a pharmaceutically effective amount of the antibody-drug conjugate, and (b) a pharmaceutically acceptable carrier. In another aspect, the present invention is directed to a method for preventing or treating tumors including administering the antibody-drug conjugate according to the present invention to a patient with a proliferative disease, for example, a tumor or cancer. In another aspect, the present invention is directed to the use of the antibody-drug conjugate for preventing or treating a proliferative disease, for example, a tumor or cancer.

With respect to such tumor or cancer, non-limiting examples of the tumor or cancer that can be treated include, but are not limited to, kidney cancer, pancreatic cancer, ovarian cancer, lymphoma, colon cancer, mesothelioma, gastric cancer, lung cancer, prostate cancer, adenocarcinoma, liver cancer, breast cancer, and the like. The tumor or cancer may include refractory or recurrent cancer.

The pharmaceutical composition may further contain a pharmaceutically acceptable carrier, and the pharmaceutically acceptable carrier may include those commonly used in the preparation of drugs, for example, one or more selected from the group consisting of lactose, dextrose, sucrose, sorbitol, mannitol, starch, gum acacia, calcium phosphate, alginate, gelatin, calcium silicate, microcrystalline cellulose, polyvinylpyrrolidone, cellulose, water, syrup, methylcellulose, methylhydroxybenzoate, propylhydroxybenzoate, talc, magnesium stearate, mineral oil, and the like, but is not limited thereto. The pharmaceutical composition may further contain one or more selected from the group consisting of diluents, excipients, lubricants, wetting agents, sweetening agents, flavors, emulsifiers, suspending agents, and preservatives.

The pharmaceutical composition may be administered orally or parenterally. Parenteral administration may be intravenous injection, subcutaneous injection, intramuscular injection, intraperitoneal injection, endothelial administration, topical administration, intranasal administration, pulmonary administration, rectal administration, or the like. Upon oral administration, proteins or peptides are digested, so an oral composition should be coated with an active drug or formulated so as to protect the same from degradation in the stomach. In addition, the pharmaceutical composition may be administered using any device capable of delivering the active substance to target cells.

The effective dose of the pharmaceutical composition according to the present invention may vary depending on factors such as the formulation method, administration method, and age, body weight, gender, pathological condition, diet, administration time, administration interval, administration route, excretion rate, and responsiveness of the patient. For example, the daily dose of the pharmaceutical composition may be within the range of 0.001 to 1,000 mg/kg, 0.01 to 100 mg/kg, 0.1 to 50 mg/kg, or 0.1 to 20 mg/kg, but is not limited thereto. The formulation may be prepared as a unit dose form containing the daily dose of the pharmaceutical composition, or a daily dose of the formulation may be divided into multiple doses or may be incorporated into a multi-dose container.

The pharmaceutical composition may be formulated in the form of a solution, a suspension, or an emulsion in an oil or aqueous medium, or may be formulated in the form of an extract, a powder, a suppository, a granule, a tablet, or a capsule. The composition may further contain a dispersant or a stabilizer for formulation.

### Example

Hereinafter, the present invention will be described in more detail with reference to examples. However, it will be obvious to those skilled in the art that these examples are provided only for illustration of the present invention, and should not be construed as limiting the scope of the present invention.

### Preparation example: drug synthesis

In the following Preparation Examples, all ¹H NMR spectra were recorded using a Bruker Avance 400 MHz.

LCMS was measured using a Shimadzu LCMS 2011 quadrupole mass spectrometer (column: Shim-pack XR-ODS (3.0*30 mm, 2.2 m)) operating in an ESI (+) ionization mode. Flow rate: 0.8 mL/min, acquisition time: 3 min or 1.5 min, Wavelength: UV220, oven temperature: 50°C.

Prep-HPLC was performed under the following conditions: Columns: Fuji C18 (300x25), YMC (250x20); Wavelength: 220 nm; Mobile phase: CH₃CN (0.05 % NH₃H₂O or 0.225 % FA); B water (0.1% NH₃·H₂O or 0.225% FA); Flow rate: 30 mL/min; Injection volume: 3 mL; Running time: 20 minutes; Equilibrium: 3 minutes

### Preparation Example 1: Synthesis of PD001

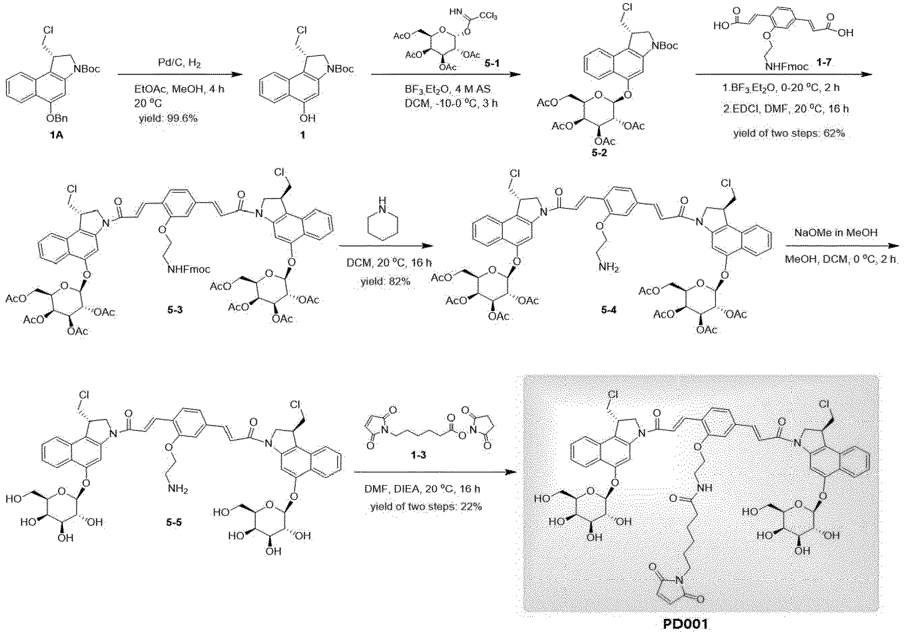

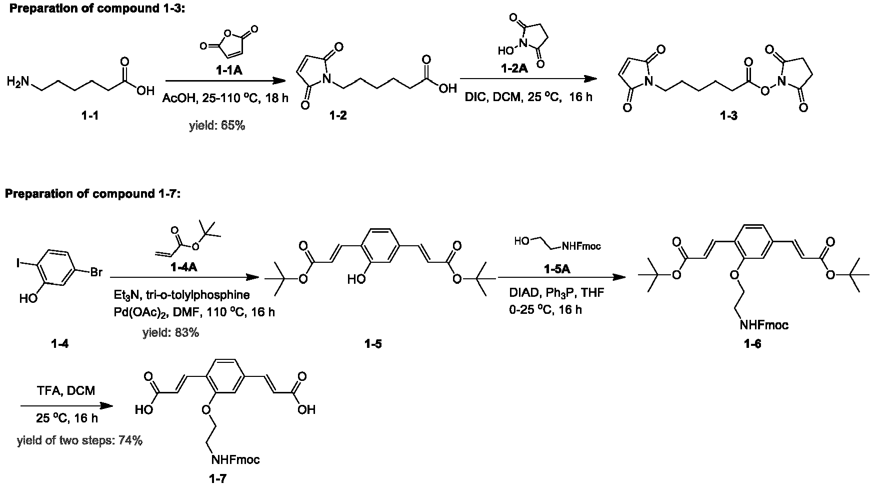

### 1) Preparation of Compound 1

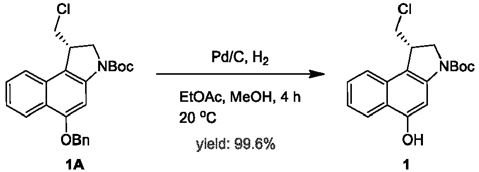

A mixture of Compound 1A (700 mg, 1.65 mmol) and 10% dry Pd/C (140 mg) in MeOH (7 mL) and EtOAc (7 mL) was degassed in a vacuum and purged several times with H₂. The mixture was stirred in H₂ (balloon, 15 psi) at 20°C for 4 hours. TLC showed that one new spot with greater polarity was formed. The mixture was filtered through celite, and the filtrate was concentrated and dried to yield Compound 1 (549 mg, yield 99.6%) as a white solid.

¹H NMR (400 MHz, CDCl₃) δ 1.56 (9H, s), 3.43 (1H, t, J = 10.4 Hz), 3.85-4.01 (2H, m), 4.07-4.16 (1H, m), 4.19-4.30 (1H, m), 6.24 (1H, brs), 7.34 (1H, t, *J* = 7.6 Hz), 7.50 (1H, t, *J* = 7.6 Hz), 7.59-7.80 (2H, m), 8.17 (1H, d, J = 8.0 Hz).

### 2) Preparation of Compound 5-2

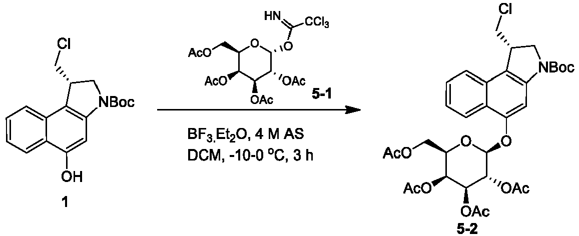

4A MS (2 g) was added to a stirred solution of Compound 1 (300 mg, 0.899 mmol) in anhydrous DCM (10 mL) at 20°C. Next, the mixture was stirred at 20°C for 30 minutes. After addition of Compound 5-1 (576 mg, 1.17 mmol), the resulting mixture was cooled to -10°C and BF₃·Et₂O (64 mg, 0.45 mmol) in DCM (5 mL) was added dropwise thereto. Then, the mixture was stirred at -10°C for 1 hour. The mixture was then heated at 0°C for 2 hours. TLC showed that a trace amount of starting material still remained, and that a new major spot with large polarity was formed. The mixture was filtered off, and the filtrate was quenched with *sat. aq.* NaHCO₃ (20 mL) . The mixed organic layer was separated. The remaining aqueous phase was extracted with DCM (15 mL * 2). The combined organic layer was dried over Na₂SO₄, concentrated and dried. The residue was mixed with batch 2 and purified in a silica gel column using PE:EtOAc/5:1 as an eluent.

4A MS (1.5 g) was further added to a stirred solution of Compound 1 (249 mg, 0.746 mmol) in DCM (10 mL) at 20°C. Then, the mixture was stirred at 20°C for 30 minutes. After addition of Compound 5-1 (478 mg, 0.970 mmol), the mixture was cooled to -10°C and BF₃·Et₂O (53 mg, 0.373 mmol) in DCM (5 mL) was added dropwise thereto. Then, the mixture was stirred at -10°C for 1 hour. The mixture was then heated at 0°C for 2 hours. TLC showed that a trace amount of starting material still remained, and that a new major spot with large polarity was formed. The mixture was filtered off, and the filtrate was quenched with *sat. aq.* NaHCO₃ (20 mL) . The organic layer was separated. The remaining aqueous phase was extracted with DCM (15 mL * 2). The combined organic layer was dried over Na₂SO₄, concentrated, and dried. The residue was mixed with batch 1 and purified in a silica gel column using PE:EtOAc/5:1 as an eluent to yield Compound 5-2 (1.19 g, crude) as a white solid. 35 mg of the starting material of Compound 1 was recovered as a white solid.

¹H NMR (400 MHz, CDCl₃) δ 1.61 (9H, s, overlap water signal), 2.02 (3H, s), 2.06 (3H, s, overlap EtOAc signal), 2.11 (3H, s), 2.24 (3H, s), 3.45 (1H, t, *J* = 10.8 Hz), 3.91-4.05 (2H, m), 4.10-4.19 (1H, m, overlap EtOAc signal), 4.20-4.43 (4H, m), 5.20 (1H, dd, *J* = 10.4, 3.6 Hz), 5.37 (1H, d, *J* = 8.0 Hz), 5.53 (1H, d, *J* = 3.2 Hz), 5.72 (1H, dd, *J* = 10.4, 7.6 Hz), 7.34-7.40 (1H, m), 7.49-7.57 (1H, m), 7.66 (1H, d, *J* = 8.0 Hz), 7.68 (1H, brs), 8.13 (1H, d, *J* = 8.0 Hz).

### 3) Preparation of Compound 5-3

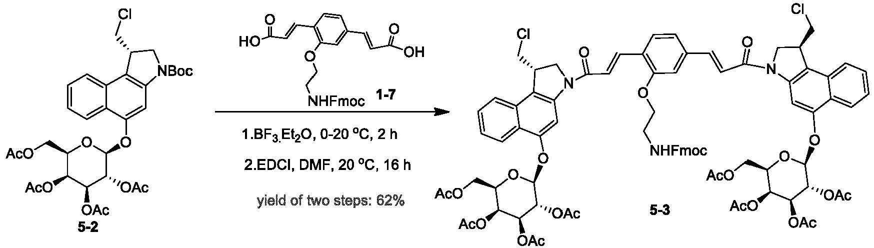

BF₃·Et₂O (890 mg, 6.27 mmol) was added dropwise to a stirred solution of Compound 5-2 (1.19 g, crude) in anhydrous DCM (15 mL) at 0°C. Then, the mixture was allowed to warm to 20°C and stirred for 2 hours. TLC showed that the reaction was completed well. The mixture was quenched with *sat. aq.* NaHCO₃ (15 mL). The organic layer was separated. The remaining aqueous phase was extracted with DCM (15 mL * 2). The combined organic layer was dried over Na₂SO₄, concentrated, and further dried. The residue was purified in a silica gel column using PE:EtOAc/2:1 to 1:1 as an eluent to obtain a de-Boc intermediate (782 mg, 84% yield of two steps) as a white solid. 28 mg of Compound 5-2 was recovered.

¹H NMR (400 MHz, CDCl₃) δ 2.06 (3H, s), 2.07 (3H, s, overlap EtOAc signal), 2.11 (3H, s), 2.24 (3H, s), 3.52 (1H, t, *J* = 10.8 Hz), 3.77-3.87 (2H, m), 3.88-3.94 (1H, m), 3.96-4.05 (1H, m), 4.12-4.25 (3H, m, overlap EtOAc signal), 4.31 (1H, dd, J = 10.8, 6.8 Hz), 5.11-5.20 (2H, m), 5.52 (1H, dd, *J* = 3.2, 0.8 Hz), 5.70 (1H, dd, *J* = 10.4, 8.0 Hz), 6.66 (1H, s), 7.20-7.20 (1H, m, overlap CDCl₃ signal), 7.45-7.52 (1H, m), 7.60 (1H, dd, *J* = 8.4, 1.2 Hz), 8.00 (1H, d, *J* = 8.0 Hz).

EDCI (219 mg, 1.14 mmol) was added to a mixture of a de-Boc intermediate (643 mg, 1.14 mmol) and Compound 1-7 (190 mg, 0.380 mmol) in anhydrous DMF (6 mL) at 20°C. Then, the mixture was stirred at 20°C for 16 hours. TLC showed that the starting material still remained and that the desired product was observed. The solvent was removed under reduced pressure. The mixture was quenched with water (15 mL) and extracted with EtOAc (15 mL * 3). The combined organic layer was washed with water (20 mL), concentrated over Na₂SO₄, and dried. The residue was purified in a silica gel column using PE:EtOAc/2:1 to 1:2 as an eluent to obtain Compound 5-3 (448 mg, yield: 74%) as a yellow solid. In addition, 163 mg of the de-Boc intermediate was recovered (LCMS: purity 75%).

¹H NMR (400 MHz, CDCl₃) δ 2.01-2.20 (18H, m, overlap EtOAc signal), 2.23 (6H, s), 3.43-3.57 (2H, m), 3.69-3.82 (2H, m), 3.95-4.05 (2H, m), 4.06-4.10 (1H, m, overlap EtOAc signal), 4.19-4.65 (15H, m), 5.12-5.27 (2H, m), 5.32-5.44 (2H, m, overlap CH₂Cl₂ signal), 5.50-5.62 (2H, m), 5.70-5.82 (2H, m), 6.92-7.13 (2H, m), 7.19-7.39 (7H, m, overlap CDCl₃ signal), 7.40-7.50 (2H, m), 7.51-7.64 (4H, m), 7.65-7.76 (5H, m), 7.84 (1H, d, *J* = 15.2 Hz), 8.13-8.27 (3H, m), 8.47 (2H, d, *J* = 18.4 Hz).

### 4) Preparation of Compound 5-4

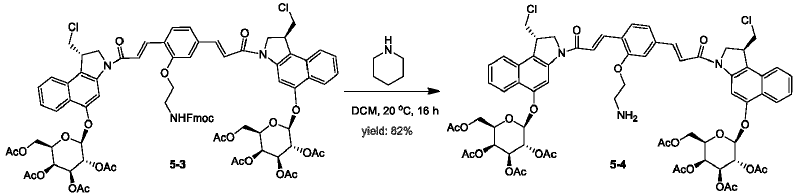

A solution of Compound 5-3 (250 mg, 0.157 mmol) and piperidine (134 mg, 1.57 mmol) in anhydrous DCM (4 mL) was stirred at 20°C for 16 hours. TLC showed that the reaction proceeded to completion. The solvent was removed under reduced pressure. Impurities were purified in a silica gel column using DCM:MeOH/50:1 to 25:1 as an eluent to obtain Compound 5-4 (176 mg, yield 82%) as a yellow solid.

¹H NMR (400 MHz, CDCl₃) δ 2.01-2.24 (24H, m), 3.35-3.59 (4H, m), 3.99-4.62 (16H, m), 5.20-5.30 (2H, m), 5.37-5.47 (2H, m), 5.56 (2H, d, *J* = 1.2 Hz), 5.75 (2H, dd, *J* = 8.0, 2.0 Hz), 6.94 (1H, d, *J* = 15.6 Hz), 7.10-7.20 (2H, m), 7.26-7.34 (1H, m, overlap CDCl₃ signal), 7.36-7.74 (7H, m), 7.80 (1H, d, *J* = 15.2 Hz), 8.05-8.20 (3H, m), 8.44 (2H, d, *J* = 7.6 Hz).

### 5) Preparation of Compound 5-5

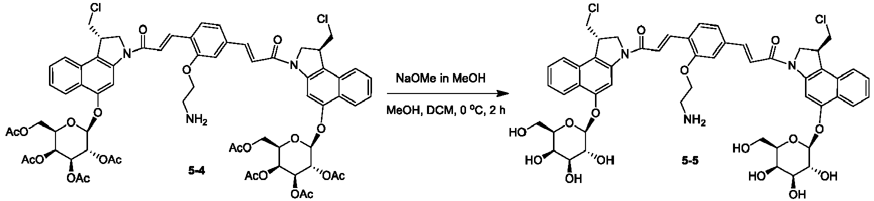

NaOMe (8.0 mg, 0.15 mmol) in MeOH (0.5 mL) was added dropwise to a solution of compound 5-4 (100 mg, 0.073 mmol) in MeOH (2 mL) and DCM (2 mL) at 0°C. Then, the mixture was stirred at 0°C for 2 hours. Crude LCMS showed that the desired product MS was observed. The mixture was quenched with AcOH (9.0 mg, 0.15 mmol). Next, the mixture was concentrated and dried at 25°C to obtain crude Compound 5-5 (79 mg) as a yellow solid. Next, 79 mg of crude Compound 5-5 was used directly in the next step.

### 6) Preparation of PD001

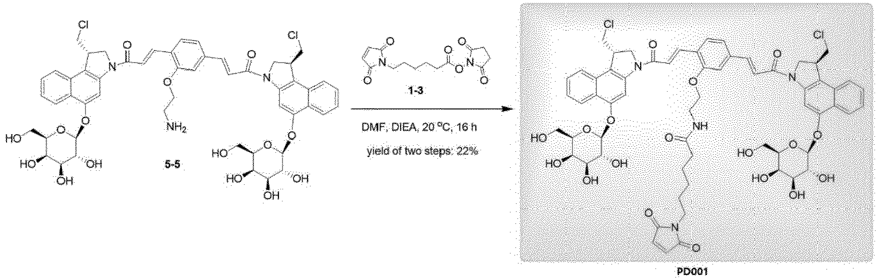

A mixture of Compound 5-5 (79 mg, crude), Compound 1-3 (24 mg, 0.076 mmol) and DIEA (20 mg, 0.15 mmol) in DMF (2 mL) was stirred at 20°C for 16 hours. Crude LCMS showed that the desired product MS was observed. The solvent was purified by prep-HPLC (0.05% NH₃·H₂O). Most of the MeCN was removed under reduced pressure. The remaining aqueous phase was lyophilized to obtain pure PD001 (20 mg, yield in 2 steps: 22%) as a yellow solid.

¹H NMR (400 MHz, DMSO-d₆) δ 1.15-1.27 (2H, m), 1.42-1.60 (4H, m), 2.13 (2H, t, *J* = 7.6 Hz), 3.48-3.74 (10H, m), 3.75-3.88 (4H, m), 3.89-4.08 (4H, m), 4.24-4.40 (4H, m), 4.48-4.74 (8H, m), 4.92-5.05 (4H, m), 5.38 (2H, d, *J* = 5.6 Hz), 6.96 (2H, s), 7.29-7.45 (4H, m), 7.48 (1H, d, *J* = 8.0 Hz), 7.52-7.63 (2H, m), 7.66 (1H, s), 7.90 (1H, d, *J* = 15.6 Hz), 7.86-8.01 (4H, m), 8.20-8.25 (1H, m), 8.33 (2H, dd, *J* = 8.4, 4.8 Hz), 8.38-8.48 (2H, m).

### 7) Preparation of Compound 1-2

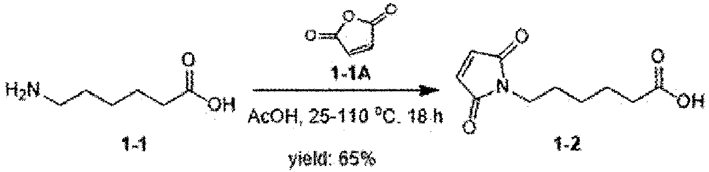

Furan-2,5-dione (374 mg, 3.81 mmol) was added to a solution of 6-aminohexanoic acid (500 mg, 3.81 mmol) in AcOH (5 mL) . The mixture was stirred under N₂ at 25°C for 2 hours. Next, the mixture was stirred at 110°C under N₂ for 16 hours. TLC showed that another spot was formed. The reaction mixture was concentrated in vacuum. Water (10 mL) was added to the residue, the resulting solution was extracted with EtOAc (20 mL * 3), and the mixed organic layer was dried over anhydrous Na₂SO₄, filtered, and then concentrated in vacuum. The residue was purified by silica gel chromatography using petroleum ether:ethyl acetate/1:1 to 1:2 to yield Compound 1-2 (525 mg, yield: 65%) as a white solid.

¹H NMR (400 MHz, DMSO-d₆) δ 1.11-1.29 (2H, m), 1.40-1.57 (4H, m), 2.18 (2H, t, *J* = 7.2 Hz), 3.36-3.41 (2H, m, overlap with water signal), 6.93-7.07 (2H, s), 12.00 (1H, brs) .

### 8) Preparation of Compound 1-3

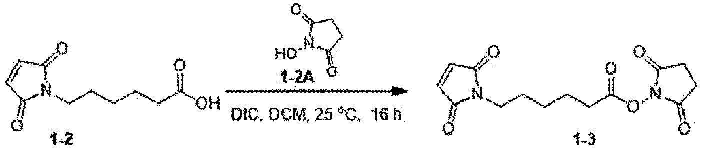

A solution of Compound 1-2 (525 mg, 2.49 mmol), 1-hydroxypyrrolidine-2,5-dione (300 mg, 2.61 mmol) and DIC (336 mg, 2.66 mmol) in DCM (5 mL) was stirred under N₂ at 25°C for 16 hours. TLC showed that another spot was formed. Water (10 mL) was added to the reaction mixture, and the resulting solution was extracted with EtOAc (15 mL * 4). The combined organic phase was dried over anhydrous Na₂SO₄, filtered, and concentrated in vacuum. The residue was purified by silica gel chromatography using petroleum ether:ethyl acetate/2:1 to 1:1 to 1:2 as an eluent to yield impure Compound 1-3 (643 mg) as a white solid.

¹H NMR (400 MHz, CDCl₃) δ 1.32-1.45 (2H, m), 1.61-1.65 (2H, m), 1.73-1.81 (2H, m), 2.59 (2H, t, *J* = 7.2 Hz), 2.82 (4H, s), 3.52 (2H, t, *J* = 7.2 Hz), 6.68 (2H, m).

### 9) Preparation of Compound 1-5

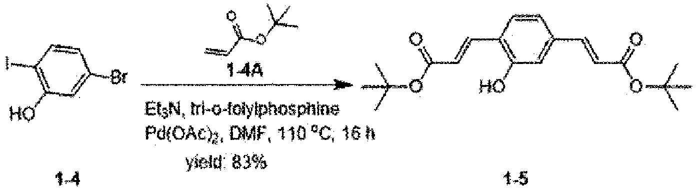

A solution of 5-bromo-2-iodine-phenol (1.0 g, 3.35 mmol), tert-butyl acrylate (1.50 g, 11.7 mmol), Pd(OAc)₂ (15 mg, 0.67 mmol), a tri-o-tolylphosphine compound (79 mg, 0.26 mmol) and Et₃N (1.02 g, 10.0 mmol) in DMF (10 mL) was stirred under N₂ at 110°C for 16 hours. TLC showed that the desired product was formed. The reaction mixture was quenched in 60 mL of water, and the resulting solution was extracted with EtOAc (40 mL * 4). The combined organic layer was washed with water (100 mL), dried over anhydrous Na₂SO₄, filtered, and then concentrated in a vacuum. The residue was purified in a CombiFlash apparatus using petroleum ether:ethyl acetate/24:1 to 20:1 to 6:1 as an eluent to yield Compound 1-5 (963 mg, yield: 83%) as a yellow solid.

¹H NMR (400 MHz, DMSO-d₆) δ 1.48 (18H, s), 6.41 (1H, d, *J* = 15.6 Hz), 6.56 (1H, d, *J* = 16.0 Hz), 7.07 (1H, s), 7.19 (1H, d, *J* = 8.0 Hz), 7.45 (1H, d, *J* = 15.6 Hz), 7.63 (1H, d, *J* = 8.0 Hz), 7.75 (1H, d, *J* = 16.4 Hz), 10.42 (1H, brs).

### 10) Preparation of Compound 1-6

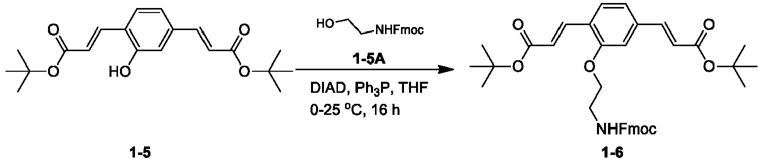

A solution of Compound 1-5 (2.00 g, 5.77 mmol) and Compound 1-5A (2.45 g, 8.66 mmol) in THF (20 mL) was stirred under N₂ at 0°C for 30 minutes. When the transparent solution was converted to a white emulsion, Ph3P (2.57 g, 9.81 mmol) was added at 0°C to the mixture and stirred under N₂ for 10 minutes. Next, DIAD (1.75 g, 8.66 mmol) was added dropwise to the mixture at 0°C (white emulsion was converted to an orange clear solution). Then, the obtained solution was allowed to warm to 25°C under N₂ for 1 hour. TLC showed that the desired product was formed. 1N *aq.* HCl (60 mL) was added to the reaction mixture, and the resulting solution was extracted with EtOAc (50 mL * 3). The combined organic layer was washed with brine (80 mL), dried over anhydrous Na₂SO₄, filtered, and concentrated in vacuum. The residue was purified in a Combiflash apparatus using petroleum ether and then petroleum ether:ethyl acetate/7:1 as eluents to yield impure Compound 1-6 (3.67 g) as a colorless oil.

### 11) Preparation of Compound 1-7

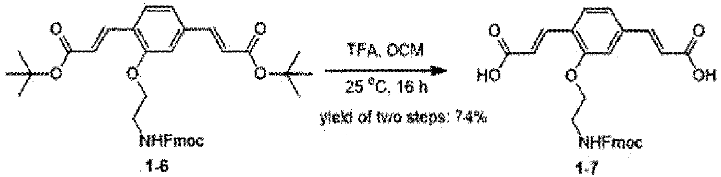

TFA (19.3 g, 169 mmol) was added to a solution of Compound 1-6 (3.67 g, impure) in DCM (80 mL). The mixture was stirred at 25°C for 16 hours without being monitored. The reaction mixture was concentrated in a vacuum. The residue was triturated with tert-butyl methyl ether (60 mL) and then filtered. The filter cake was washed with tert-butyl methyl ether (20 mL * 3). The filter cake was dried in a vacuum to give compound 1-7 (2.12 g, yield in 2 steps: 74%) as a white solid.

¹H NMR (400 MHz, DMSO-d₆) δ 3.43-3.46 (2H, m), 4.13-4.27 (3H, m), 4.31-4.43 (2H, m), 6.59 (1H, d, *J* = 16.2 Hz), 6.69 (1H, d, *J* = 16.2 Hz), 7.29-7.35 (3H, m), 7.36-7.48 (3H, m), 7.55-7.65 (2H, m), 7.66-7.77 (3H, m), 7.80-7.92 (3H, m), 12.40 (2H, brs).

### Preparation 2. Synthesis of PD005

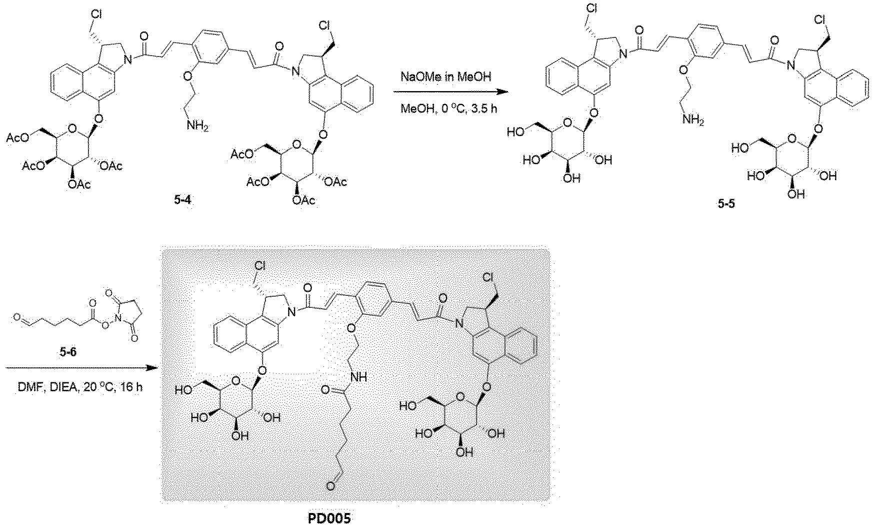

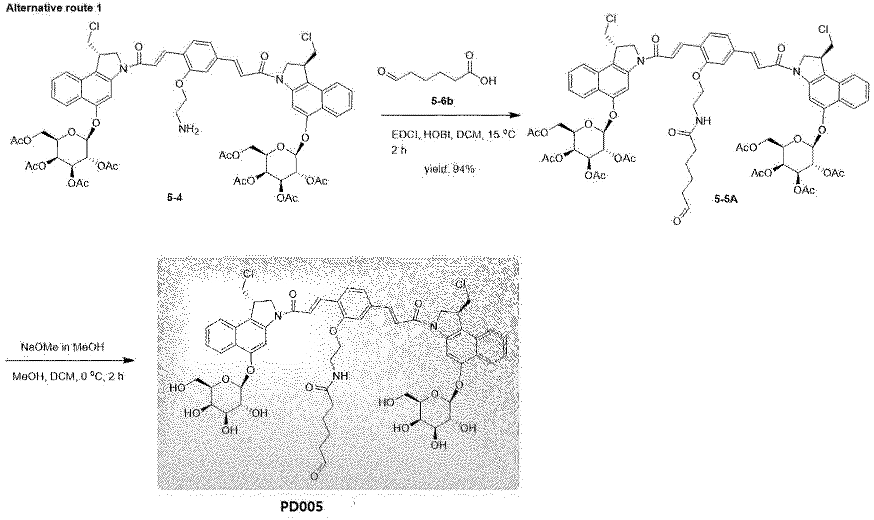

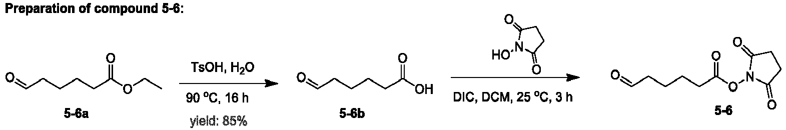

### 1) Preparation of Compound 5-5

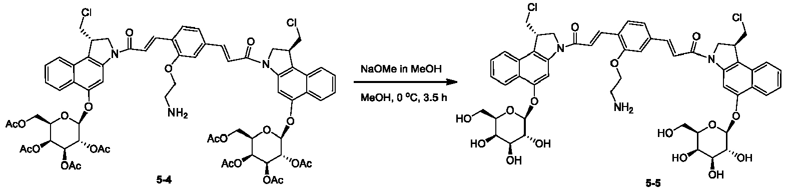

A solution of NaOMe (4.0 mg, 0.073 mmol) in MeOH 0.1 mL) was added dropwise at 0°C to a solution of Compound 5-4 (50 mg, 0.036 mmol) in MeOH (1 mL) and DCM (1 mL). Next, the mixture was stirred at 0°C for 3.5 hours. Crude LC-MS showed that the purity of the product was 93% at a retention time of 0.828 (MS Calcd: 1031; MS Found: 1034 [M+3H]⁺) . The mixture was quenched with AcOH (4.6 mg). Then, the mixture was concentrated to dryness at 25°C to yield crude Compound 5-5 (41 mg) as a yellow solid. 41 mg of crude Compound 5-5 was used directly in the next step.

### 2) Preparation of PD005

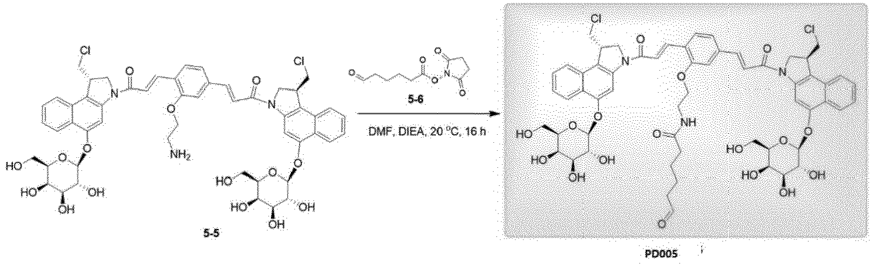

A mixture of Compound 5-5 (41 mg, crude), Compound 5-6 (11 mg, crude) and DIPEA (10 mg, 0.079 mmol) in DMF (1 mL) was stirred at 15°C for 16 hours. Crude LC-MS showed that the purity of the product was 89% at a retention time of 0.845 (MS Calcd: 1143; MS Found: 1149 [M+6H]⁺). The reaction mixture was purified by prep-HPLC (0.225% FA). Most of the MeCN was removed under reduced pressure. The remaining aqueous phase was lyophilized to yield crude PD005 (11 mg) as a yellow solid. In addition, HNMR showed that trace amounts of aldehyde signal protons were observed.

### 3) Preparation of Compound 5-5A

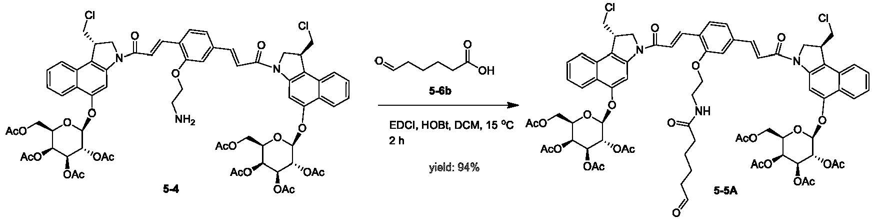

HOBt (9.5 mg, 0.070 mmol), EDCI (13 mg, 0.070 mmol) and DIEA (9.1 mg, 0.070 mmol) were added to a solution of Compound 5-6b (8.4 mg, 0.064 mmol) in DCM (2 mL). Next, the mixture was stirred at 15°C for 10 minutes. Compound 5-4 (80 mg, 0.058 mmol) was added to the reaction mixture. The prepared mixture was stirred at 15°C for 2 hours. TLC showed that the starting material was completely consumed. One new spot was formed. The mixture was quenched with water (10 mL) and extracted with DCM (6 mL * 3). The mixed organic layer was dried over Na₂SO₄ and concentrated to dryness. The residue was purified in a silica gel column using EtOAc as an eluent to obtain Compound 5-5A (81 mg, yield: 94 %) as a yellow solid.

¹H NMR (400 MHz, CDCl₃) δ 1.58-1.75 (4H, m), 1.95-2.05 (12H, m), 2.06-2.16 (12H, m), 2.24-2.31 (2H, m), 2.40-2.49 (2H, m), 3.42-3.55 (3H, m), 3.69-3.79 (2H, m), 3.92-4.05 (2H, m), 4.10-4.38 (9H, m, overlap EtOAc signal), 4.42-4.64 (4H, m), 5.12-5.26 (2H, m), 5.30-5.42 (2H, m), 5.50-5.60 (2H, m), 5.67-5.77 (2H, m), 6.91-7.06 (2H, m), 7.13-7.22 (2H, m, overlap CDCl₃ signal), 7.35-7.48 (3H, m), 7.50-7.60 (2H, m), 7.65-7.76 (3H, m), 7.77-7.84 (1H, m), 8.09-8.19 (2H, m), 8.20-8.30 (1H, m), 8.37-8.50 (2H, m), 9.71 (1H, s) .

### 4) Preparation of PD005

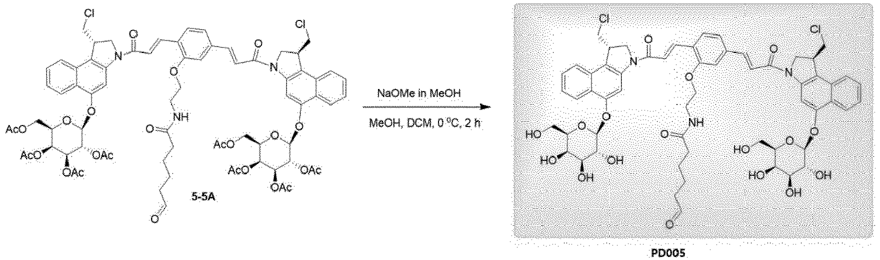

NaOMe (1.8 mg, 0.034 mmol) in MeOH (0.1 mL) was added dropwise at 0°C to a stirred solution of Compound 5-5A (25 mg, 0.017 mmol) in MeOH (0.5 mL) and DCM (0.5 mL). Then, the mixture was stirred at 0°C for 2 hours. Crude LC-MS showed that the purity of the product was 98% at a retention time of 0.834 (MS Calcd.: 1143; MS Found: 1144 [M+H]⁺). Next, the mixture was quenched with water (5 mL) and the solvent was removed under reduced pressure at 15°C. The produced yellow precipitate was collected by filtration. The cake was diluted with water (10 mL) and lyophilized to obtain crude PD005 (11 mg) as a yellow solid. About 6 mg of crude was used for the HNMR analysis, and 5 mg of crude was supplied.

¹H NMR (400 MHz, DMSO) δ 1.19-1.60 (4H, m), 2.05-2.22 (2H, m), 2.35-2.45 (2H, m, overlap DMSO signal), 3.45-4.76 (30H, m), 4.90-5.05 (4H, m), 5.35-5.53 (2H, m), 7.18-8.05 (13H, m), 8.15-8.50 (4H, m,), 9.61 (0.5H, m).

NaOMe (4.1 mg, 0.076 mmol) in MeOH (0.2 mL) was added dropwise at 0°C to a stirred solution of Compound 5-5A (56 mg, 0.038 mmol) in MeOH (1 mL) and DCM (1 mL) . Next, the mixture was stirred at 0°C for 2 hours. Crude LCMS showed that the desired product M was formed. Crude LC-MS showed that the purity of the product was 98% at a retention time of 0.857 (MS Calcd.: 1143; MS Found: 1169 [M+Na]⁺).

Next, the mixture was quenched with water (10 mL) and the solvent was removed under reduced pressure at 15°C. The produced yellow precipitate was collected by filtration. The cake was diluted with water (10 mL) and lyophilized to obtain crude PD005 (27 mg) as a yellow solid.

¹H NMR (400 MHz, DMSO) δ 1.19-1.60 (4H, m), 2.05-2.21 (2H, m), 2.35-2.45 (2H, m, overlap DMSO signal), 3.45-4.76 (30H, m), 4.90-5.05 (4H, m), 5.35-5.51 (2H, m), 7.21-8.01 (13H, m), 8.15-8.50 (4H, m,), 9.61 (0.4H, m).

### 5) Preparation of Compound 5-6b

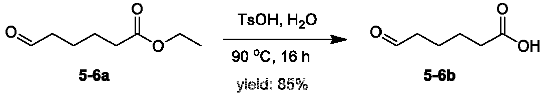

A solution of ethyl 6-oxohexanoate (546 mg, 3.45 mmol), TsOH (15 mg, 0.08 mmol), and H₂O (1.2 mL) was stirred under N₂ at 90°C for 16 hours. TLC showed that another spot was formed. The reaction mixture was quenched in water (10 mL), and the resulting solution was extracted with EtOAc (15 mL * 4). The combined organic layer was dried over anhydrous Na₂SO₄, filtered, and concentrated in a vacuum. The residue was purified in a silica gel column using petroleum ether:ethyl acetate/1:1 to 2:3 as eluents to obtain Compound 5-6b (383 mg, yield: 85%) as a colorless oil.

¹H NMR (400 MHz, DMSO-d₆) δ 1.49-1.53 (4H, m), 2.22 (2H, t, *J* = 6.8 Hz), 2.44 (2H, t, *J* = 7.2 Hz), 9.66 (1H, s), 12.01 (1H, brs).

### 6) Preparation of Compound 5-6

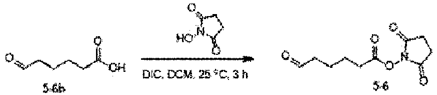

DIC (397 mg, 3.15 mmol) was added under N₂ to a solution of Compound 5-6b (383 mg, 2.94 mmol) and N-hydroxysuccinimide (356 mg, 3.09 mmol) in DCM (4 mL). The mixture was stirred at 25°C for 3 hours. TLC showed that another spot with lower polarity was formed. The reaction mixture was filtered, the filtrate was quenched with water (10 mL), and the organic layer was separated. The aqueous phase was then extracted with DCM (10 mL * 3). The combined organic layer was dried over anhydrous Na₂SO₄, filtered, and concentrated in a vacuum. The residue was purified in a silica gel column using petroleum ether:ethyl acetate/3:2 to 2:3 as eluents to obtain impure Compound 5-6 (204 mg) as a white solid.

¹H NMR (400 MHz, CDCl₃) δ 1.76-1.78 (4H, m), 2.49-2.51 (2H, m), 2.64 (2H, t, *J* = 6.8 Hz), 2.84 (4H, s), 9.77 (1H, s).

### Preparation Example 3. Synthesis of PD006

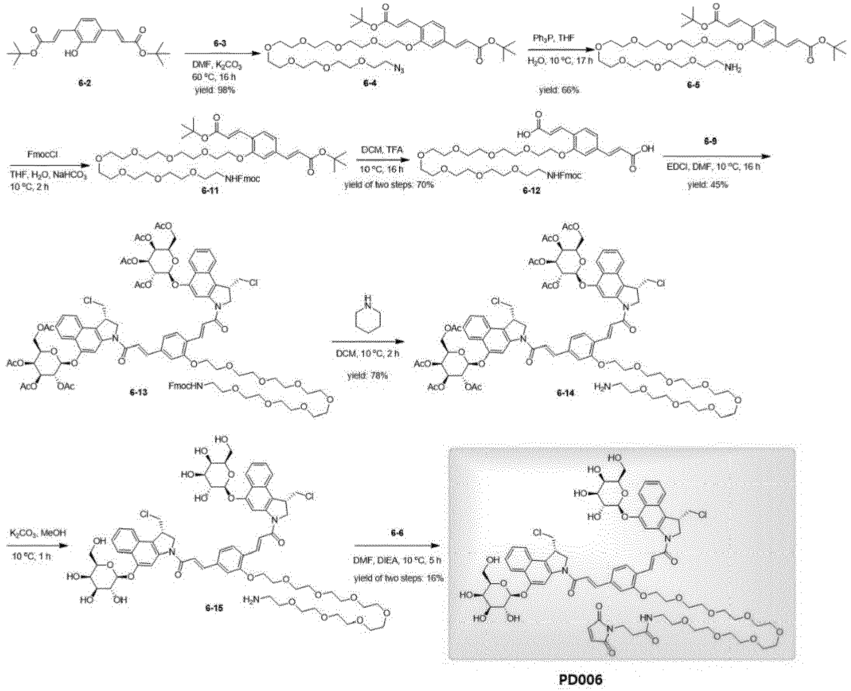

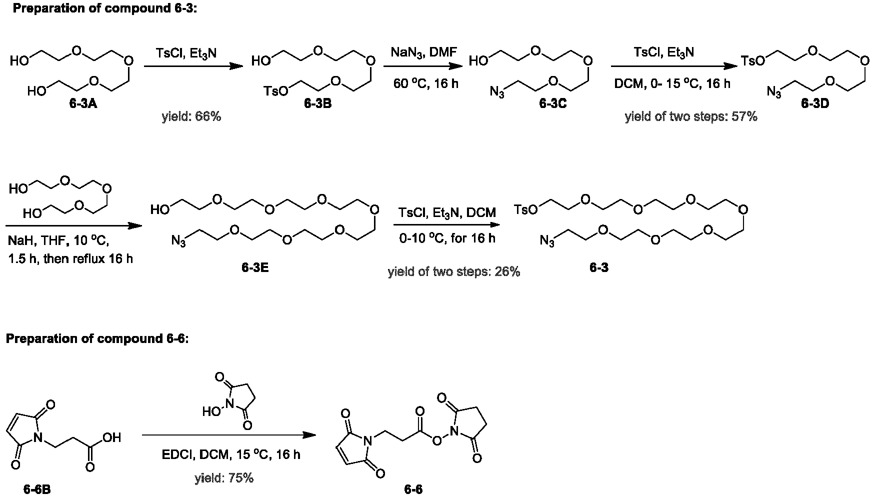

### 1) Preparation of Compound 6-4

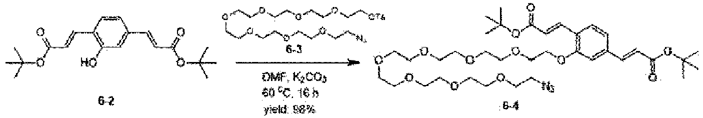

A mixture of Compound 6-3 (349 mg, 0.635 mmol) and Compound 6-2 (220 mg, 0.635 mmol) and K₂CO₃ (105 mg, 0.762 mmol) in DMF (3 mL) was heated to 60°C and stirred for 16 hours. Crude LC-MS showed that the purity of the product was 59% at a retention time of 1.088 (MS Calcd: 723.4; MS Found: 746.3 [M+Na]⁺) . Most of the solvent was removed under reduced pressure. The mixture was quenched with water (20 mL) and extracted with EtOAc (15 mL * 3). The combined organic layer was washed with water (20 mL * 2), dried over Na₂SO₄, concentrated, and further dried to obtain Compound 6-4 as a brown oil (0.45 g, yield 98%).

¹H NMR (400 MHz, CDCl₃) δ 1.51-1.56 (18H, m), 3.39 (2H, t, *J* = 4.8 Hz), 3.61-3.68 (24H, m), 3.69-3.72 (2H, m), 3.92 (2H, t, *J* = 4.8 Hz), 4.21 (2H, t, *J* = 4.8 Hz), 6.37 (1H, d, *J* = 16.0 Hz), 6.50 (1H, d, *J* = 16.0 Hz), 7.02 (1H, s), 7.10 (1H, d, *J* = 8.0 Hz), 7.47-7.55 (2H, m), 7.86 (1H, d, *J* = 16.0 Hz).

The synthesis procedure of Compound 6-2 is the same as in Example for PD001 described above.

### 2) Preparation of Compound 6-5

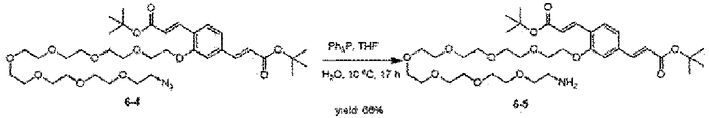

PPh₃ (196 mg, 0.746 mmol) was added to a solution of Compound 6-4 (450 mg, 0.622 mmol) in THF (4 mL) at 10°C, and the mixture was stirred at 1°C for 1 hour. Then, H₂O (1 mL) was added to the mixture, and the resulting mixture was stirred at 10°C for 16 hours. Crude LC-MS showed that the purity of the product was 21% at a retention time of 0.967 (MS Calcd: 697.4; MS Found: 698.4 [M+H]⁺) . The solvent was removed under reduced pressure. The residue was purified using EtOAc, DCM:MeOH/5:1, and then MeOH:NH₃-H₂O/100:1 as eluents to obtain Compound 6-5 (285 mg, yield 66%) as a brown oil.

¹H NMR (400 MHz, CDCl₃) δ 1.52-1.64 (18H, m, overlap water signal), 2.88 (2H, t, *J* = 5.2 Hz), 3.63 (2H, t, *J* = 5.2 Hz), 3.62-3.75 (22H, m), 3.76-3.80 (2H, m), 3.94 (2H, t, *J* = 5.2 Hz), 4.24 (2H, t, *J* = 5.2 Hz), 6.39 (1H, d, *J* = 16.0 Hz), 6.52 (1H, d, *J* = 16.4 Hz), 7.04 (1H, s), 7.12 (1H, d, *J* = 8.4 Hz), 7.49-7.57 (2H, m), 7.89 (1H, d, *J* = 16.4 Hz).

No two active protons were observed.

### 3) Preparation of Compound 6-11

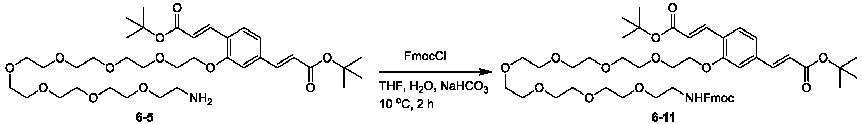

Fmoc-Cl (96 mg, 0.37 mmol) and NaHCO₃ (31 mg, 0.37 mmol) were added at 10°C to a solution of Compound 6-5 (235 mg, 0.337 mmol) in THF (1 mL) and H₂O (1 mL). Then, the mixture was stirred at 10°C for 2 hours. Crude LC-MS showed that the purity of the product was 84% at a retention time of 1.155 (MS Calcd: 919.5; MS Found: 942.5 [M+Na]⁺). The mixture was quenched with water (5 mL) and extracted with EtOAc (5 mL * 3). The mixed organic layer was dried over Na₂SO₄, concentrated, and further dried to obtain crude Compound 6-11 (325 mg) as a colorless oil, which contained FmocCl.

### 4) Preparation of Compound 6-12

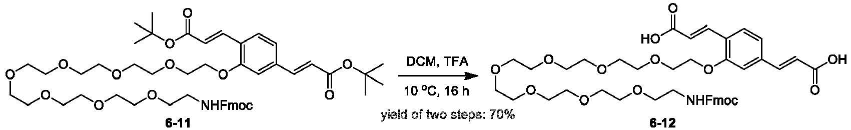

A mixture of Compound 6-11 (325 mg, crude) and DCM (3 mL) in TFA (2 mL) was stirred at 10°C for 16 hours. Crude LC-MS showed that the purity of the product was 87% at a retention time of 0.890 (MS Calcd: 807.4; MS Found: 830.3 [M+Na]⁺) . The solvent was removed under reduced pressure. The residue was triturated with EtOAc (5mL) to obtain Compound 6-12 (191 mg, yield in 2 steps: 70%) as a white solid.

¹H NMR (400 MHz, DMSO) δ 3.10-3.17 (2H, m), 3.30-3.43 (2H, m, overlap water signal), 3.45-3.58 (24H, m), 3.59-3.64 (2H, m), 3.78-3.84 (2H, m), 4.18-4.32 (5H, m), 4.42 (2H, d, *J* = 7.2 Hz), 5.46 (1H, brs), 6.65 (2H, dd, *J* = 16.0, 14.4 Hz), 7.27-7.36 (4H, m), 7.38-7.46 (3H, m), 7.57 (1H, d, *J* = 16.0 Hz), 7.66-7.74 (3H, m), 7.81 (1H, d, *J* = 16.4 Hz), 7.89 (2H, d, *J* = 7.6 Hz), 12.4 (2H, brs).

### 5) Preparation of Compound 6-13

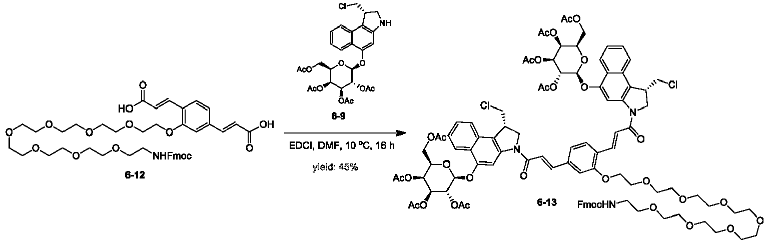

A mixture of Compound 6-12 (90 mg, 0.11 mmol), Compound 6-9 (188 mg, 0.334 mmol), and EDCI (64 mg, 0.33 mmol) in DMF (2 mL) was stirred at 10°C for 16 hours. TLC showed that the desired product was formed. The solvent was removed under greatly reduced pressure. The residue was purified in a CombiFlash apparatus using PE:EtOAc/2:1 and then EtOAc:MeOH/50:1 as eluents to obtain Compound 13 (96 mg, yield 45%) as a yellow solid.

¹H NMR (400 MHz, CDCl₃) δ 2.04-2.09 (12H, m, overlap EtOAc signal), 2.16 (6H, d, *J* = 10.4 Hz), 2.24 (6H, s), 3.35-3.43 (2H, m), 3.47-3.84 (30H, m), 3.98-4.09 (4H, m), 4.11-4.62 (15H, m, overlap EtOAc signal), 5.17-5.27 (2H, m), 5.34-5.43 (2H, m), 5.46-5.62 (3H, m), 5.69-5.80 (2H, m), 6.97 (1H, d, *J* = 14.8 Hz), 7.17 (1H, s), 7.25-7.36 (4H, m, overlap CDCl₃ signal), 7.37-7.50 (4H, m), 7.53-7.66 (5H, m), 7.69-7.80 (4H, m), 7.83 (1H, d, *J* = 15.2 Hz), 8.00 (1H, d, *J* = 15.2 Hz, overlap DMF signal), 8.17 (2H, dd, *J* = 8.4, 2.0 Hz), 8.46 (2H, d, *J* = 12.0 Hz).

The preparation process for Compound 6-9 is the same as in Example for PD001 described above.

### 6) Preparation of Compound 6-14

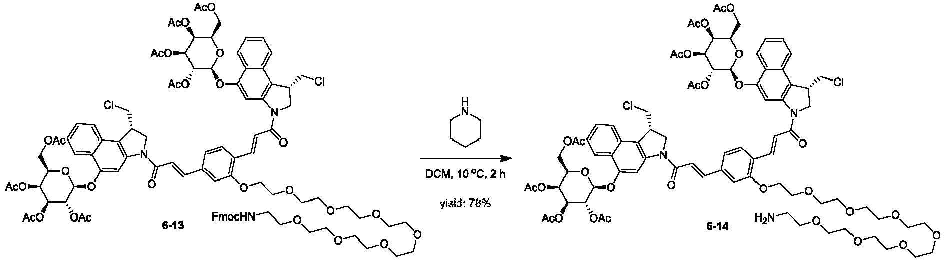

A solution of piperidine (86 mg, 1.0 mmol, 0.1 mL) and compound 6-13 (96 mg, 0.051 mmol) in DCM (1 mL) was stirred at 10°C for 3 hours. TLC showed that the starting material was completely consumed and that one new major site was formed. The solvent was removed under reduced pressure. The residue was purified in a CombiFlash apparatus using DCM:MeOH/100:1 to 9:1 as an eluent to obtain Compound 6-14 (75 mg, yield: 78 %) as a yellow solid.

¹H NMR (400 MHz, CDCl₃) δ 2.03-2.09 (12H, m, overlap EtOAc signal), 2.17 (6H, d, *J* = 10.4 Hz), 2.24 (6H, s), 3.11-3.18 (2H, m), 3.48-3.87 (30H, m), 3.97-4.70 (18H, m, overlap EtOAc signal), 5.16-5.26 (2H, m), 5.36-5.46 (2H, m), 5.51-5.63 (2H, m), 5.68-5.79 (2H, m), 7.13 (1H, d, *J* = 15.2 Hz), 7.24-7.37 (3H, m, overlap CDCl₃ signal), 7.40-7.49 (2H, m), 7.53-7.65 (3H, m), 7.73 (2H, d, *J* = 8.0 Hz), 7.83 (1H, d, *J* = 15.2 Hz), 8.02 (1H, d, *J* = 15.2 Hz), 8.16 (2H, d, *J* = 8.4 Hz), 8.46 (2H, d, *J* = 8.4 Hz).

### 7) Preparation of Compound 6-15

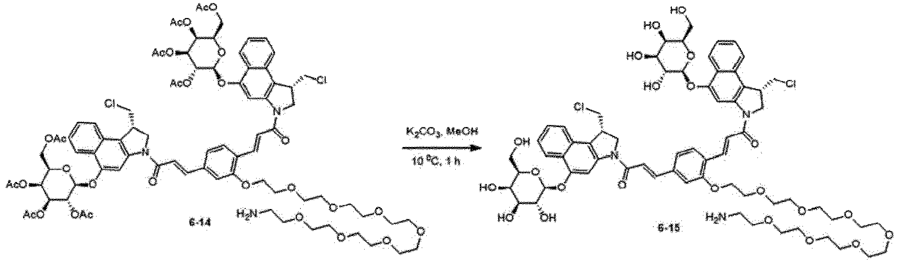

K₂CO₃ (14 mg, 0.10 mmol) was added at 10°C to a solution of Compound 6-14 (87 mg, 0.052 mmol) in MeOH (2 mL) and DCM (1 mL). Then, the mixture was stirred at 10°C for 1 hour. Crude LC-MS showed that the purity of the product was 90% at a retention time of 0.836 (MS Calcd.: 1339.5; MS Found: 1340.9 [M+H]⁺) . TLC showed that the starting material was completely consumed. The mixture was quenched with AcOH (10 mg). The mixture was concentrated to dryness to yield crude Compound 6-15 (74 mg) as a yellow solid, which was used directly in the next step.

### 8) Preparation of PD006

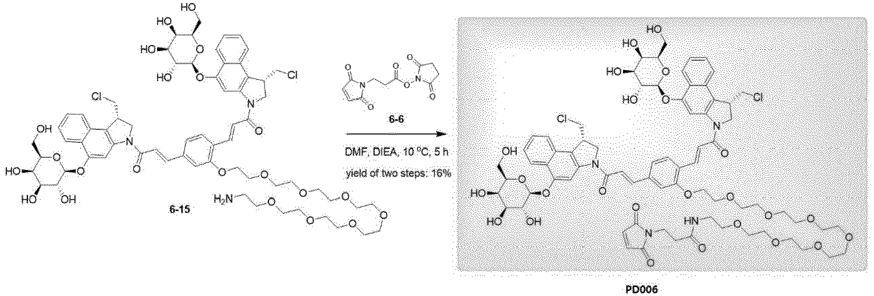

A mixture of Compound 6-15 (74 mg, crude), Compound 6-6 (16 mg, 0.061 mmol), and DIEA (8.6mg, 0.066 mmol) in DMF (1 mL) was stirred at 10°C for 5 hours. Crude LC-MS showed that the purity of the product was 94% at a retention time of 0.842 (MS Calcd: 1490.5; MS Found: 1493.5 [M+2H]⁺). The solvent was removed under reduced pressure. The residue was purified by prep-HPLC (0.225% FA). The remaining aqueous solution was lyophilized to obtain PD006 (8 mg) and 7 mg of impurities. The impure PD006 was further purified by prep-HPLC (0.225% FA). The remaining aqueous solution was lyophilized to obtain PD006 (4 mg) as a yellow solid. A total of 12 mg of PD006 was obtained, and the yield in both steps was 16%.

¹H NMR (400 MHz, DMSO) δ 2.24-2.37 (2H, m, overlap DMSO signal), 3.08-3.21 (2H, m), 3.24-4.10 (46H, m, overlap water signal), 4.26-4.77 (12H, m), 4.88-5.02 (4H, m), 5.34-5.47 (2H, m), 6.99 (1.1H, s), 7.30-7.64 (8H, m), 7.74 (1H, d, *J* = 15.2 Hz), 7.83-7.94 (4H, m), 7.98-8.04 (1H, m), 8.29-8.46 (4H, m).

### 9) Preparation of Compound 6-3B

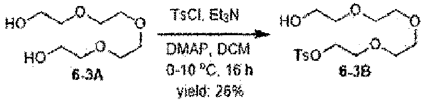

TsCl (14.7 g, 77.2 mmol) was added at 0°C to a mixture of tetraethylene glycol (30.0 g, 154 mmol), EtN (11.7 g, 116 mmol) and DMAP (944 mg, 7.72 mmol) in DCM (300 mL) . The reaction mixture was stirred at 10°C for 16 hours. TLC showed two new spots and showed that the starting material was completely consumed. The reaction mixture was quenched with water (100 mL) and the organic layer was separated. Then, the remaining mixture was extracted with DCM (100 mL * 4). The combined organic layer was washed with brine (400 mL). The organic layer was dried over Na₂SO₄, filtered, and concentrated in a vacuum. The residue was purified on a silica gel column using EtOAc and then DCM:MeOH/20:1 as eluents to obtain colorless oily Compound 6-3B (9.33g) and colorless oily Compound 6-3B (7.16g). Then, 7.16 g of the impure oil was purified in a CombiFlash apparatus using DCM:MeOH/50:1 as an eluent to 20:1 to obtain a colorless oily Compound 6-3B (4.89 g). Thus, a total of 14.2 g of compound 6-3B was obtained, and the yield was 26%.

¹H NMR (400 MHz, CDCl₃) δ 2.45 (3H, s), 2.48-2.62 (1H, brs), 3.58-3.71 (14H, m), 4.17 (2H, t, *J* = 4.8 Hz), 7.34 (2H, d, *J* = 8.0 Hz), 7.80 (2H, dd, *J* = 6.4 Hz, 1.6 Hz).

### Condition 2:

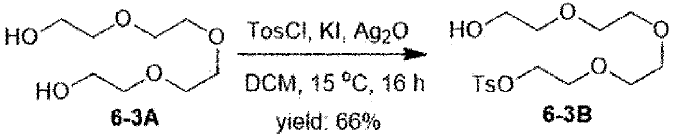

A mixture of TsCl (9.82 g, 51.5 mmol), KI (855 mg, 5.15 mmol) and Ag₂O (14.3 g, 61.8 mmol) was added at 15°C to a solution of tetraethylene glycol (10.0 g, 51.5 mmol) in DCM (300 mL). The reaction mixture was stirred at 15°C for 16 hours. TLC showed another spot with greater polarity and showed that the starting material was completely consumed. The reaction mixture was filtered through Celite, and the filtrate was concentrated in a vacuum. The residue was purified in a Combiflash apparatus using petroleum ether:ethyl acetate/1:1 and MeOH as eluents to obtain Compound 6-3B (11.8 g, yield: 66%) as a colorless oil.

¹H NMR (400 MHz, CDCl₃) δ 2.32-2.44 (1H, brs), 2.45 (3H, s), 3.60-3.71 (14H, m), 4.17 (2H, t, *J* = 4.4 Hz), 7.33 (2H, d, *J* = 8.0 Hz), 7.80 (2H, d, *J* = 8.0 Hz).

### 10) Preparation of Compound 6-3C

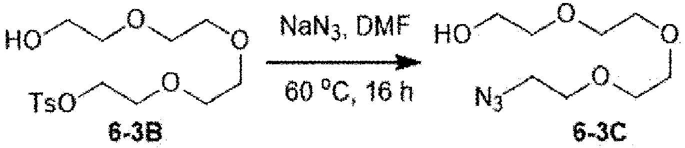

NaN₃ (465 mg, 7.15 mmol) was added to a solution of Compound 6-3B (1.66 g, 4.76 mmol) in DMF (20 mL). The reaction mixture was stirred at 60°C for 16 hours. TLC showed another spot with lower polarity and showed that the starting material was completely consumed. The reaction mixture was quenched with water (50 mL), and the resulting solution was extracted with EtOAc (50 mL * 4). The combined organic layers were dried over anhydrous Na₂SO₄, filtered, and concentrated in a vacuum. The residue was purified in a silica gel column using DCM:MeOH/40:1 to 30:1 as an eluent to obtain a pale yellow oily impure Compound 6-3C (800 mg).

¹H NMR (400 MHz, CDCl₃) δ 2.46-2.72 (1H, brs), 3.34-3.45 (2H, m), 3.59-3.64 (2H, m), 3.66-3.71 (10H, m), 3.71-3.75 (2H, m).

### 11) Preparation of Compound 6-3D

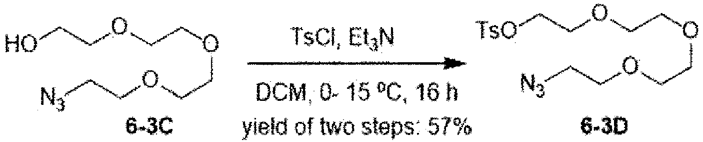

EtN (517 mg, 5.11 mmol) and TsCl (974 mg, 5.11 mmol) were added at 0°C to a solution of Compound 6-3C (800 mg, impure) in DCM (16 mL), and were then allowed to warm to 15°C for 16 hours. TLC showed another spot with low polarity. The mixture was quenched with water (20 mL) and the organic layer was separated. Then, the remaining mixture was extracted with DCM (15 mL * 3). The combined organic layer was dried over anhydrous Na₂SO₄, filtered, and concentrated in a vacuum. The residue was purified in a silica gel column using petroleum ether:ethyl acetate/2:1 to 1:1 as an eluent to obtain Compound 6-3D (1.01 g, yield of 2 steps: 57 %) as a colorless oil.

¹H NMR (400 MHz, CDCl₃) δ 2.47 (3H, s), 3.35-3.46 (2H, m), 3.62-3.72 (12H, m), 4.18 (2H, t, *J* = 4.8 Hz), 7.36 (2H, d, *J* = 8.0 Hz), 7.82 (2H, d, *J* = 8.4 Hz).

### 12) Preparation of Compound 6-3E

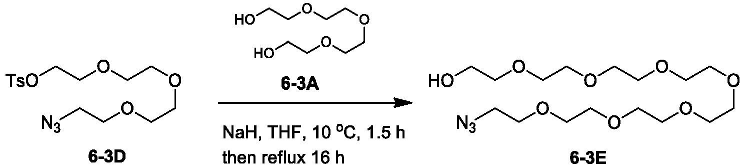

Compound 6-3A (3.41 g, 17.6 mmol) in THF (3 mL) was added dropwise at 0°C under N₂ to a suspension of NaH (780 mg, purity of 60% in 19.5 mmol mineral oil) in THF (33 mL). The reaction was stirred at 10°C for 1.5 hours. Then, Compound 6-3D (3.28 g impure) in THF (3 mL) was added dropwise to a refluxing solution of sodium alcoholate. Then, the mixture was refluxed (70°C) for 16 hours. TLC showed that a new spot was formed. After the reaction mixture was cooled to room temperature, THF was removed in a vacuum. The residue was quenched with water (30 mL), and the resulting solution was extracted with a mixed solvent (DCM : MeOH / 10 : 1) (50 mL * 5). The mixed organic layer was dried over anhydrous Na₂SO₄, filtered, and concentrated in a vacuum to obtain crude Compound 6-3E (3.47 g, crude), which was used directly in the next step.

### 13) Preparation of Compound 6-3

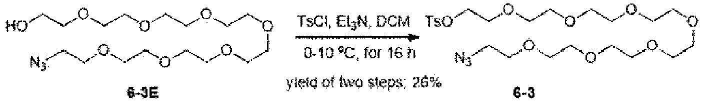

EtN (1.12 g, 11.1 mmol) was added to a solution of Compound 6-3E (3.03 g, crude) in DCM (30 mL) and TsCl (2.12 g, 11.1 mmol) was added at 0°C to the mixture. The reaction mixture was warmed to 10°C and stirred for 16 hours. TLC showed that another new spot was formed. The reaction mixture was quenched with water (40 mL) and the organic layer was separated. Then, the remaining aqueous phase was extracted with DCM (30 mL * 5). The combined organic layer was then dried over anhydrous Na₂SO₄, filtered, and concentrated in a vacuum. The residue was purified in a silica gel column using petroleum ether:ethyl acetate (EtOAc)/3:1 as an eluent to obtain impure Compound 6-3 (1.82 g) . The impure solid was purified using a CombiFlash apparatus using petroleum ether:ethyl acetate/2:1 and then EtOAc as eluents to obtain Compound 6-3 (1.26g, yield of 2 steps: 26%) as a yellow oil.

¹H NMR (400 MHz, CDCl₃) δ 2.47 (3H, s), 3.40-3.53 (2H, m), 3.54-3.83 (28H, m), 4.14-4.24 (2H, m), 7.36 (2H, d, *J* = 8.4 Hz), 7.81 (2H, d, *J* = 8.0 Hz).

### 14) Preparation of Compound 6-6

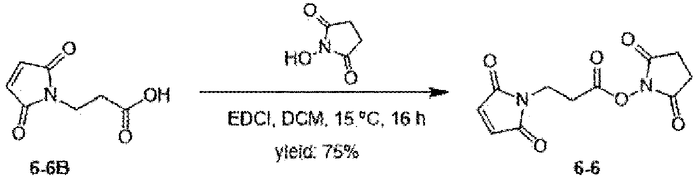

EDCI (1.21 g, 6.33 mmol) was added to a solution of 3-maleimidepropionic acid (1.00 g, 5.91 mmol) and N-hydroxysuccinimide (714 mg, 6.21 mmol) in DCM (10 mL) and the resulting mixture was stirred in the presence of N₂ at 15°C for 16 hours. TLC showed another spot having lower polarity was formed. The reaction was quenched with water (15 mL) and the organic layer was separated. Then, the remaining aqueous phase was extracted with DCM (20 mL * 3). The combined organic layer was dried over anhydrous Na₂SO₄, filtered, and concentrated in a vacuum. The residue was purified in a silica gel column using petroleum ether:ethyl acetate/1:2 as an eluent to obtain Compound 6-6 (1.18 g, yield: 75%) as a white solid.

¹H NMR (400 MHz, CDCl₃) δ 2.84 (4H, s), 3.04 (2H, t, *J* = 14.0 Hz), 3.95 (2H, t, *J* = 7.2 Hz), 6.76 (2H, s).

### Preparation Example 4: Synthesis of PD008

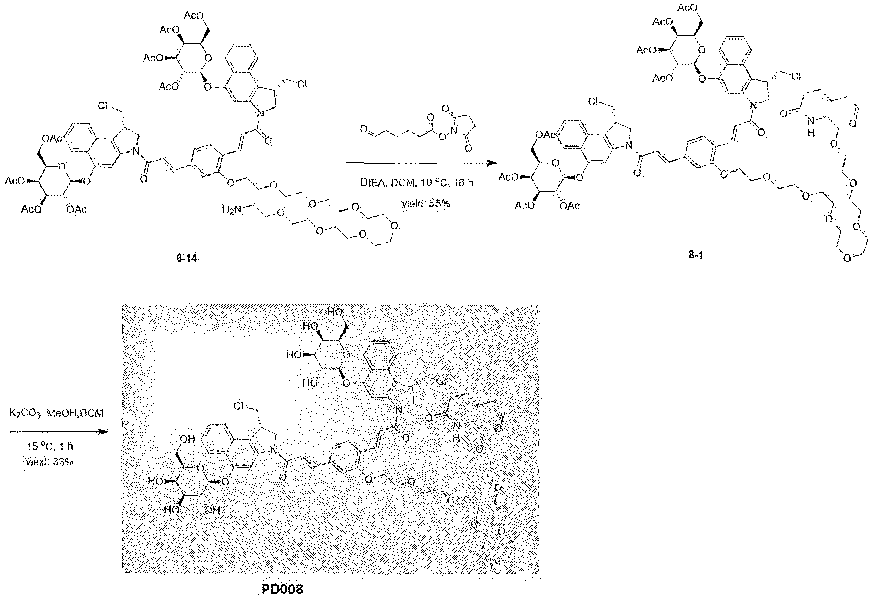

### 1) Preparation of Compound 8-1

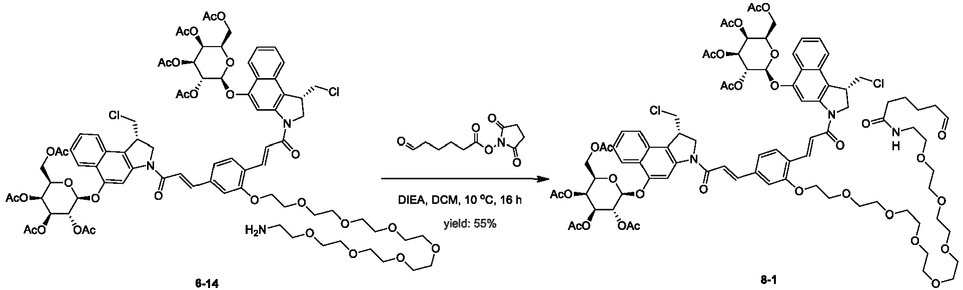

A mixture of (2,5-dioxopyrrolidin-1-yl) 6-oxohexanoate (16 mg, 0.072 mmol), DIPEA (15 mg, 0.12 mmol), and Compound 6-14 (100 mg, 0.0596 mmol) in DCM (1 mL) was stirred at 10°C for 16 hours. TLC showed that a new spot with lower polarity was formed. The mixture was quenched with water (8 mL) and extracted with DCM (8 mL * 3). The combined organic layer was dried over Na₂SO₄ and concentrated to dryness. The residue was purified through prep-TLC using EtOAc:MeOH/10:1 as an eluent to obtain Compound 8-1 (59 mg, yield: 55%) as a yellow solid.

¹H NMR (400 MHz, CDCl₃) δ 1.55-1.76 (4H, m, overlap water signal), 2.03-2.10 (12H, m), 2.13-2.27 (14H, m), 2.45-2.50 (2H, m), 3.42-3.48 (2H, m), 3.49-3.85 (30H, m), 3.99-4.63 (16H, m), 5.22 (2H, dd, *J* = 10.4, 3.2 Hz), 5.40 (2H, dd, *J* = 7.2, 2.0 Hz), 5.57 (2H, d, *J* = 2.8 Hz), 5.70-5.79 (2H, m), 6.41 (1H, brs), 6.98 (1H, d, *J* = 15.2 Hz), 7.18 (1H, s), 7.26-7.36 (2H, m, overlap CDCl ₃ signal), 7.41-7.48 (2H, m), 7.54-7.65 (3H, m), 7.70-7.78 (2H, m), 7.84 (1H, d, *J* = 15.2 Hz), 8.01 (1H, d, *J* = 15.6 Hz), 8.14-8.20 (2H, m), 8.46 (2H, d, *J* = 11.6 Hz), 9.77 (1H, t, *J* = 1.6 Hz).

The synthesis of (2,5-dioxopyrrolidin-1-yl) 6-oxohexanoate and Compound 6-14 is the same as in the preparation example of PD006.

### 2) Preparation of Compound PD008

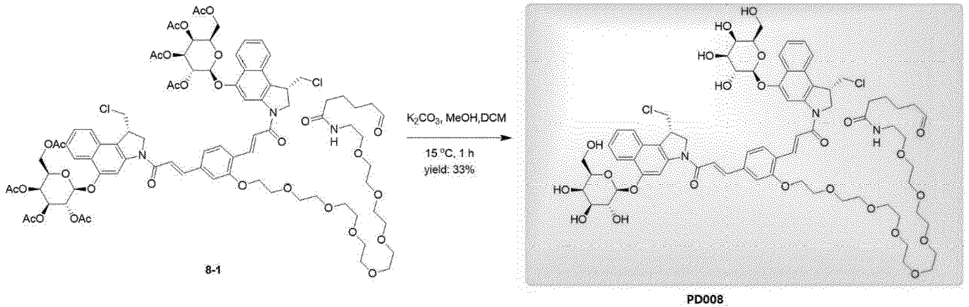

A mixture of Compound 8-1 (59 mg, 0.033 mmol) and K₂CO₃ (9.1 mg, 0.065 mmol) in MeOH (1 mL) and DCM (0.5 mL) was stirred at 15°C for 1 hour. Crude LC-MS showed that the purity of the product was 99.9% at a retention time of 0.796 (MS Calcd: 1451.5; MS Found: 1455.1 [M+3H]⁺). The mixture was quenched with AcOH (4 mg). The resulting mixture was purified by prep-HPLC (0.225% FA). Most of the MeCN was removed under reduced pressure. The remaining mixture was lyophilized to obtain PD008 (16 mg, yield: 33%) as a yellow solid.

¹H NMR (400 MHz, DMSO) δ 1.36-1.56 (4H, m), 1.95-2.12 (2H, m), 2.33-2.44 (2H, m, overlap DMSO-d₆ signal), 3.08-3.22 (2H, m, overlap water signal), 3.24-4.11 (44H, m, overlap water signal), 4.24-4.74 (12H, m), 4.86-5.04 (4H, m), 5.24-5.57 (2H, m), 7.27-7.64 (8H, m), 7.68-7.94 (6H, m), 8.28-8.49 (4H, m), 9.64 (0.6H, s).

### Preparation Example 5: Synthesis of PD009

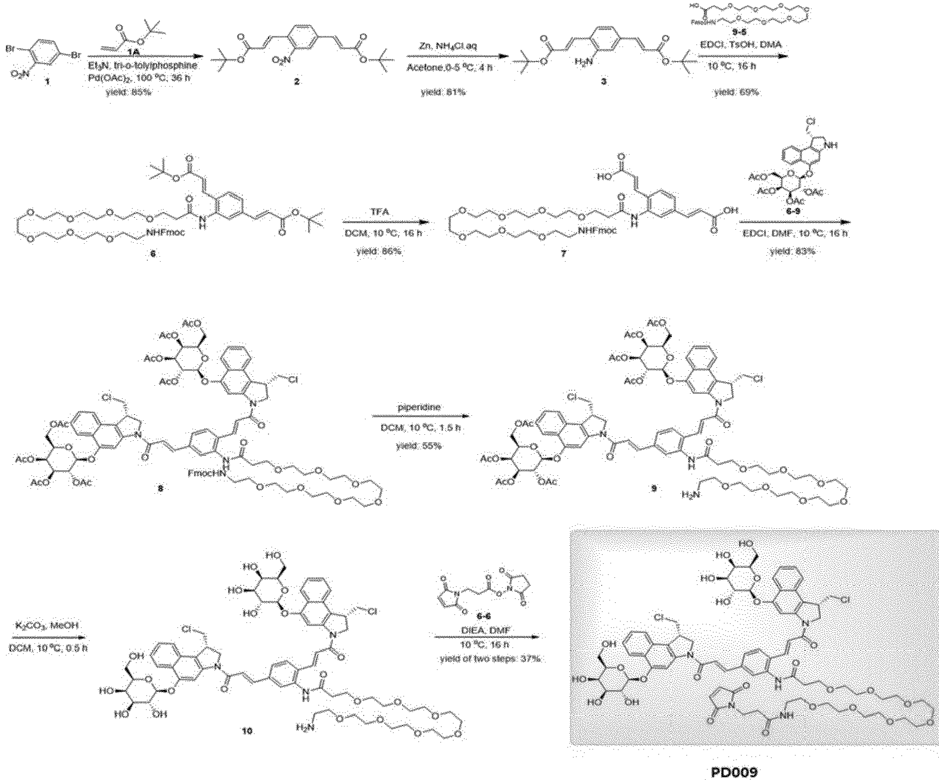

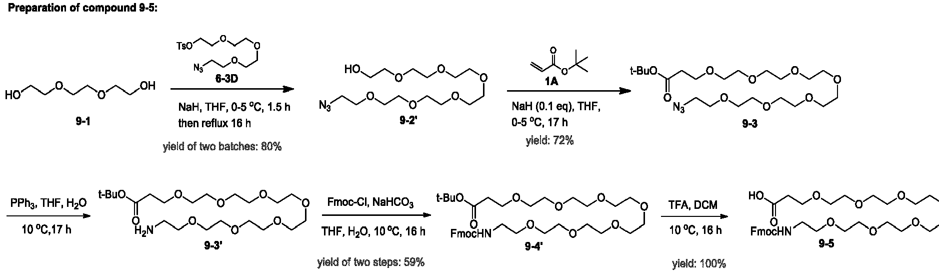

### 1) Preparation of Compound 2

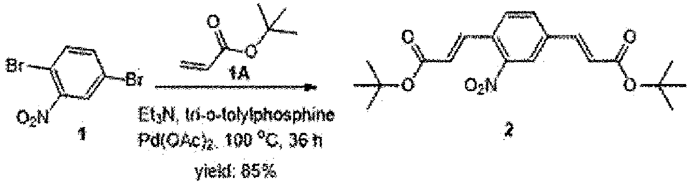

Tris(o-tolyl)-phosphine (433 mg, 1.42 mmol) and diacetoxypalladium (80 mg, 0.36 mmol) were added under N₂ to a solution of 2,5-dibromobenzene (5.00 g, 17.8 mmol) and tert-butyl acrylate (6.84 g, 53.4 mmol). The mixture was stirred at 100°C for 16 hours. TLC showed that Compound 1 was not completely consumed. Next, tert-butyl acrylate (4.00 g), tri(o-tolyl)-phosphine (224 mg) and diacetoxypalladium (80 mg) were added to the reaction mixture. Then, the mixture was stirred at 100°C for 20 hours. TLC showed that another spot having greater polarity was formed. The reaction mixture was concentrated in a vacuum, EtOAc (50 mL) was added to the residue, the mixture was filtered, and the filtrate was concentrated in a vacuum. The residue was purified in a CombiFlash apparatus using PE:EtOAc / 50:1 as an eluent to obtain Compound 2 (5.72 g, yield: 85 %) as a yellow solid.

¹H NMR (400 MHz, CDCl₃) δ 1.54 (18H, s), 6.34 (1H, d, *J* = 15.6 Hz), 6.48 (1H, d, *J* = 16.0 Hz), 7.56 (1H, d, *J* = 16.0 Hz), 7.65 (1H, d, *J* = 8.0 Hz), 7.72 (2H, dd, *J* = 8.0 Hz, 1.6Hz), 7.98 (1H, d, *J* = 16.0 Hz), 8.12 (1H, d, *J* = 1.6 Hz).

### 2) Preparation of Compound 3

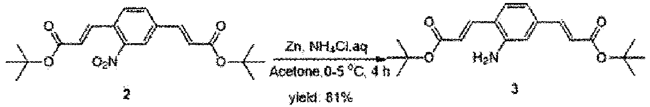

Zn (9.73 g, 149 mmol) was added to a solution of compound 2 (6.98 g, 18.6 mmol) in acetone (70 mL) cooled in an ice bath, and then a solution of NH₄Cl (3.98 g, 74.4 mmol) in H₂O (35 mL) was added thereto. The mixture was stirred at 5°C for 4 hours. TLC showed another spot having greater polarity was formed and that the starting material was completely consumed. EtOAc (50 mL*4) was added to the reaction mixture, and the clear solution present as an uppermost layer was collected. The combined organic layer was dried over anhydrous Na₂SO₄, filtered, and concentrated in a vacuum. The residue was purified in a CombiFlash apparatus using PE:EtOAc / 16:1 as an eluent to obtain Compound 3 (5.22 g, yield: 81 %) as a yellow solid.

¹H NMR (400 MHz, CDCl₃) δ 1.54 (18H, s, overlap water signal), 3.99 (2H, brs), 6.26-6.38 (2H, m), 6.81 (1H, s), 6.93 (1H, d, *J* = 9.6 Hz), 7.37 (1H, d, *J* = 8.4 Hz), 7.46 (1H, d, *J* = 16.0 Hz), 7.68 (1H, d, *J* = 16.0 Hz).

### 3) Preparation of Compound 6

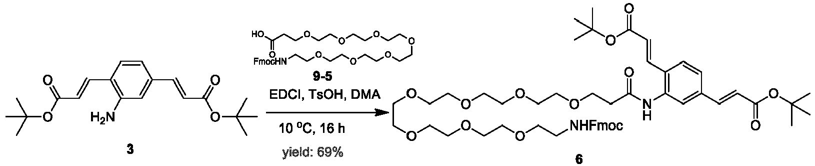

A mixture of Compound 3 (300 mg, 0.484 mmol), TsOH (8 mg, 0.05 mmol), Compound 9-5 (251 mg, 0.726 mmol), and EDCI (464 mg, 2.42 mmol) in DMA (6 mL) was stirred at 10°C for 16 hours. TLC showed that another spot having greater polarity was formed. DMA was removed in a vacuum. The residual solution was quenched in water (10 mL), and the resulting solution was extracted with EtOAc : MeOH = 3 : 1 (30 mL * 6). The combined organic layer was dried over anhydrous Na₂SO₄, filtered, and concentrated in a vacuum. The residue was purified in a silica gel column using EtOAc and then EtOAc:MeOH/10:1 as eluents to obtain Compound 6 (319 mg, yield: 69%) as a yellow oil.

¹H NMR (400 MHz, CDCl₃) δ 1.52 (18H, s), 2.71 (2H, t, *J* = 5.6 Hz), 3.44-3.67 (26H, m), 3.71-3.75 (2H, m), 3.82-3.89 (2H, m), 4.16-4.24 (1H, m), 4.38-4.40 (2H, m), 5.45 (1H, brs), 6.33-6.45 (2H, m), 7.26-7.32 (3H, m, overlap CDCl ₃ signal), 7.39 (2H, t, *J* = 7.6 Hz), 7.51-7.61 (4H, m), 7.69-7.76 (3H, m), 8.00 (1H, s), 8.76 (1H, brs).

### 4) Preparation of Compound 7

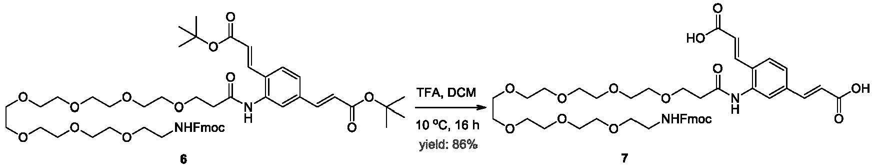

TFA (1.09 g, 9.52 mmol) was added to a solution of Compound 6 (319 mg, 0.337 mmol) in DCM (3 mL). The mixture was stirred at 10°C for 16 hours. Crude LC-MS showed that the product had a purity of 95% at a retention time of 0.801 (MS Calcd.: 834.3; MS Found: 835.0 [M+H]⁺). The reaction mixture was concentrated in a vacuum. The residue was triturated with EtOAc (10 mL) and filtered. The filter cake was washed with EtOAc (5mL) to obtain Compound 7 (244 mg, yield: 86%) as a white solid.

¹H NMR (400 MHz, DMSO-d₆) δ 2.51-2.68 (2H, m, overlap DMSO-d₆ signal), 3.09-3.17 (2H, m), 3.44-3.58 (26H, m, overlap water signal), 3.62-3.79 (2H, m), 4.16-4.21 (1H, m), 4.22-4.42 (2H, m), 6.48-6.61 (2H, m), 7.28-7.34 (3H, m), 7.36-7.49 (2H, m), 7.54-7.58 (2H, m), 7.66-7.72 (4H, m), 7.84-7.90 (3H, m), 9.88 (1H, brs), 12.48 (2H, brs).

### 5) Preparation of Compound 8

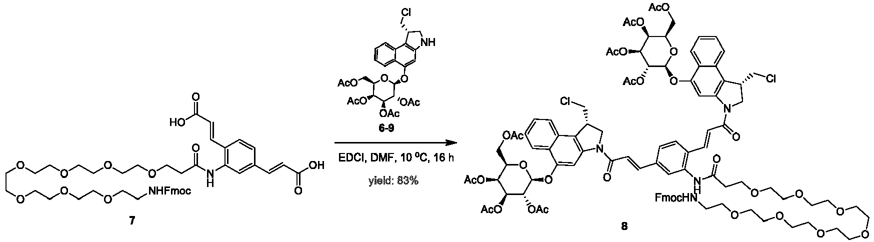

EDCI (168 mg, 0.877 mmol) was added at 10°C to a solution of compound 7 (244 mg, 0.292 mmol) and Compound 6-9 (494 mg, 0.877 mmol) in DMF (4 mL). Next, the mixture was stirred at 10°C for 16 hours. TLC showed that another spot having greater polarity was formed. The DMF was removed in a vacuum and the residue was quenched with water (10 mL). The resulting solution was extracted with EtOAc : MeOH = 20 : 1 (15mL * 6). The combined organic layers were dried over anhydrous Na₂SO₄, filtered, and concentrated in a vacuum. The residue was purified in a silica gel column using PE:EtOAc / 1:1 to 0:1 and then EtOAc : MeOH / 20 : 1 as eluents to obtain Compound 8 (469 mg, yield: 83%) as a yellow solid.

¹H NMR (400 MHz, CDCl₃) δ 1.97-2.04 (12H, m, overlap water signal), 2.11-2.14 (6H, m), 2.15-2.23 (6H, m), 2.71-2.82 (2H, m), 3.39-3.62 (26H, m), 3.67-3.73 (2H, m), 3.75-3.83 (2H, m), 3.92-4.06 (4H, m), 4.11-4.57 (15H, m) (overlap EtOAc signal), 5.22 (2H, d, *J* = 7.6 Hz), 5.35-5.40 (2H, m), 5.50-5.56 (3H, m), 5.74 (2H, t, *J* = 8.0 Hz), 6.95-7.03 (2H, m), 7.27-7.36 (2H, m, overlap CDCl₃ signal), 7.37-7.46 (5H, m), 7.54-7.61 (4H, m), 7.71-7.76 (5H, m), 7.84 (1H, d, *J* = 15.6 Hz), 7.98-8.04 (1H, m), 8.17 (3H, d, *J* = 8.0 Hz), 8.43 (2H, d, *J* = 8.0 Hz), 8.95 (1H, brs).

The synthesis of Compound 6-9 was the same as in Preparation Example of PD005.

### 6) Preparation of Compound 9

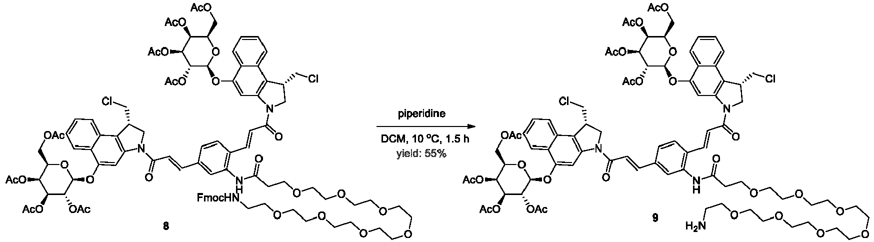

Piperidine (0.3 mL) was added at 10°C to a solution of Compound 8 (197 mg, 0.102 mmol) in DCM (3 mL). The mixture was stirred at 10°C for 1.5 hours. TLC showed that another spot having greater polarity was formed. The reaction mixture was concentrated in a vacuum, and the residue was quenched with water (10 mL). The resulting solution was extracted in DCM (15 mL * 4). The combined organic layer was dried over anhydrous Na₂SO₄, filtered, and concentrated in a vacuum. The residue was purified in a silica gel column using EtOAc and then DCM:MeOH/50:1 as eluents to 10:1 to obtain Compound 9 (96 mg, yield: 55 %) as a yellow solid.

¹H NMR (400 MHz, CDCl₃) δ 2.03-2.10 (12H, m, overlap water signal), 2.11-2.18 (6H, m), 2.19-2.28 (6H, m), 2.95-3.06 (2H, m), 3.16-3.16 (2H, m), 3.48-3.76 (28H, m), 3.76-3.82 (2H, m), 3.84-3.90 (2H, m), 3.91-4.06 (4H, m), 4.13-4.39 (8H, m), 4.46-4.63 (4H, m), 5.23 (2H, d, *J* = 10.8 Hz), 5.40 (2H, d, *J* = 8.0 Hz), 5.52-5.61 (2H, m), 5.68-5.80 (2H, m), 6.98 (2H, t, *J* = 15.6 Hz), 7.38-7.49 (3H, m), 7.56 (2H, t, *J* = 7.2 Hz), 7.67-7.89 (4H, m), 7.83 (2H, d, *J* = 15.2 Hz), 7.93-8.04 (2H, m), 8.12-8.20 (2H, m), 8.34-8.51 (2H, m), 10.04 (1H, brs).

### 7) Preparation of Compound 10

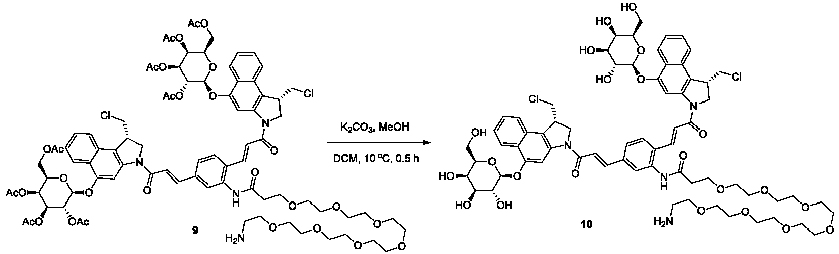

K₂CO₃ (19 mg, 0.14 mmol) was added to a solution of Compound 9 (120 mg, 0.0704 mmol) in DCM (1 mL) and MeOH (2 mL). The mixture was stirred at 10°C for 30 minutes. Crude LC-MS showed a new peak, and showed that compound 9 was completely consumed (MS Calcd.: 1702.5) (MS of compound 10 exceeded 1500 and thus could not be detected) . CH₃COOH (19 mg) was added to the reaction mixture. The mixture was stirred at 10°C for 5 minutes and concentrated in a vacuum to obtain Compound 10 (92 mg, crude) as a yellow solid.

### 8) Preparation of PD009

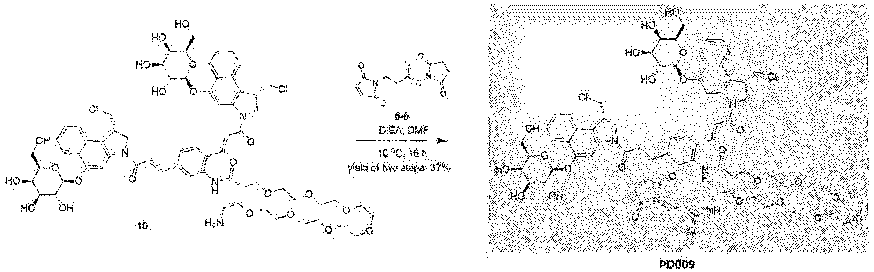

Compound 6-6 (28 mg, 0.11 mmol) was added at 10°C to a solution of Compound 10 (92 mg, crude) and DIEA (14 mg, 0.11 mmol) in DMF (1 mL). The mixture was stirred at 10°C for 16 hours. Crude LC-MS showed a new peak, and showed that Compound 10 was completely consumed (MS Calcd.: 1366.5) (MS of PD009 exceeded 1500 and could not be detected). The resulting mixture was purified by prep-HPLC (0.225 % FA). Most of the MeCN was removed under reduced pressure. The remaining mixture was lyophilized to obtain PD009 (40 mg, yield in 2 steps: 37%) as a yellow solid.

¹H NMR (400 MHz, DMSO-d₆) δ 2.32 (2H, t, *J* = 7.2 Hz), 2.64-2.74 (2H, m) 3.09-3.18 (2H, m), 3.46-3.71 (33H, m, overlap water signal), 3.73-4.12 (12H, m), 4.23-4.70 (11H, m) 4.91-5.00 (4H, m), 5.34-5.44 (2H, m), 6.99 (1.4H, s), 7.27 (2H, d, *J* = 15.2 Hz), 7.43 (2H, t, *J* = 8.0 Hz), 7.57 (2H, t, *J* = 7.6 Hz), 7.66-7.81 (2H, m), 7.84-7.91 (4H, m), 7.98-8.10 (2H, m), 8.32-8.41 (4H, m), 10.00 (1H, brs)

The synthesis of compound 6-6 was the same as in the preparation example of PD006.

### 9) Preparation of Compound 9-2'

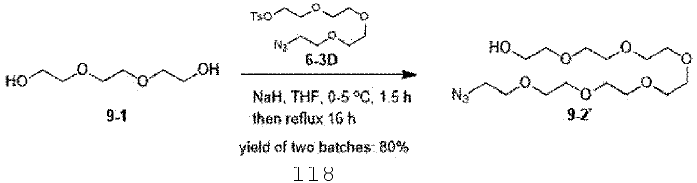

### Batch 1

Triethylene glycol (2.41 g, 16.1 mmol) in THF (5 mL) was added dropwise at 0°C under N₂ to a suspension of NaH (536 mg, 60% in 13.4 mmol mineral oil) in THF (20 mL). The reaction was stirred at 5°C for 1.5 hours. Next, Compound 6-3D (2.00 g, 5.36 mmol) in THF (5 mL) was added dropwise to a refluxing solution of sodium alcoholate. Next, the mixture was refluxed (70°C) under N₂ for 16 hours. TLC showed another new position, and showed that Compound 6-3D was completely consumed. The reaction mixture was mixed with batch 2.

### Batch 2

Triethylene glycol (2.41 g, 16.1 mmol) in THF (5 mL) was added dropwise under N₂ at 0°C to a suspension of NaH (536 mg, 60% in 13.4 mmol mineral oil) in THF (20 mL). The reaction was stirred at 5°C for 1.5 hours. Next, Compound 6-3D (2.00 g, 5.36 mmol) in THF (5 mL) was added dropwise to a refluxing solution of sodium alcoholate. Next, the mixture was refluxed (70°C) under N₂ for 16 hours. TLC showed another new position, and showed that compound 6-3D was completely consumed. The reaction mixture was quenched with water (50 mL) along with batch 1. The organic layer was separated, and then the remaining mixture was extracted with DCM: MeOH = 10: 1 (50 mL * 4). The combined organic layer was dried over anhydrous Na₂SO₄, filtered, and concentrated in a vacuum. The residue was purified in a silica gel column using DCM:MeOH/100:1 to 50:1 as an eluent to obtain Compound 9-2' (3.03 g, 2-batch yield: 80%) as a pink oil.

¹H NMR (400 MHz, CDCl₃) δ 3.41 (2H, t, *J* = 5.2 Hz), 3.62-3.76 (26H, m).

The synthesis of Compound 6-3D was the same as in the Preparation Example of PD006.

### 10) Preparation of Compound 9-3

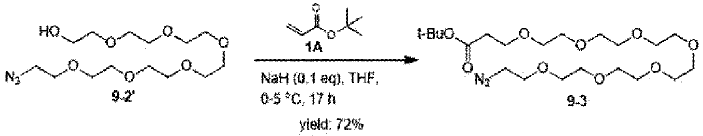

NaH (4.0 mg, 0.093 mmol, 60 % in mineral oil) was added at 0°C to a solution of Compound 9-2' (328 mg, 0.933 mmol) in THF (5 mL) and the mixture was stirred at 0°C for 1 hour. Next, tert-butyl acrylate (239 mg, 1.87 mmol) was added to the mixture. The reaction mixture was stirred at 5°C for 16 hours. TLC showed another spot having low polarity. A trace amount of Compound 9-2' remained. The reaction was quenched with water (10 mL), and the resulting solution was extracted with DCM : MeOH = 10 : 1 (15 mL * 4). The combined organic layer was dried over anhydrous Na₂SO₄, filtered, and concentrated in a vacuum. The residue was purified in a silica gel column using PE:EtOAc/1:1 to 1:4 as an eluent to obtain Compound 9-3 (322 mg, yield: 72%) as a colorless oil.

¹H NMR (400 MHz, CDCl₃) δ 1.45 (9H, s), 2.50 (2H, t, *J* = 6.8 Hz), 3.39 (2H, t, *J* = 5.2 Hz), 3.62-3.72 (28H, m).

### 11) Preparation of Compound 9-3'

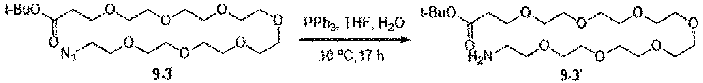

PPh₃ (896 mg, 3.42 mmol) was added under N₂ to a solution of compound 9-3 (1.49 g, 3.11 mmol) in THF (10 mL). The mixture was stirred at 10°C for 1 hour and then H₂O (5 mL) was added to the mixture. Next, the mixture was stirred under N₂ at 10°C for 16 hours. Crude LC-MS showed that the product had a purity of 3% at a retention time of 0.742 (MS Calcd.: 453.2; MS Found: 454.2 [M+H]⁺). THF was removed under vacuum. The remaining solution was quenched with water (10 mL), and the resulting solution was extracted with EtOAc : PE = 3 : 1 (30 mL * 2). The remaining aqueous layer was concentrated in a vacuum to prepare Compound 9-3' (1.52 g, crude) (containing water) as a colorless oil.

### 12) Preparation of Compound 9-4'

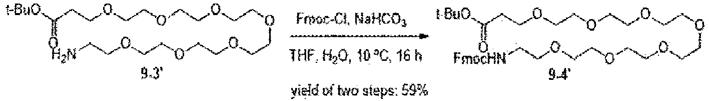

Fmoc-Cl (897 mg, 3.47 mmol) and NaHCO₃ (291 mg, 3.47 mmol) were added to a solution of Compound 9-3' (1.52 g) (crude, water) in THF (10 mL) and H₂O (10 mL). After the mixture was stirred at 10°C for 16 hours, TLC showed another less polar spot. THF was removed under vacuum and the remaining solution was quenched with water (20 mL). The resulting solution was extracted with EtOAc (40 mL * 5). The combined organic layers were dried over anhydrous Na₂SO₄, filtered, and concentrated in a vacuum. The residue was purified in a silica gel column using PE: EtOAc/1:1 to 0:1 as an eluent to obtain Compound 9-4' (1.25 g, yield of 2 steps: 59 %) as a colorless oil.

¹H NMR (400 MHz, CDCl₃) δ 1.46 (9H, s), 2.51 (2H, t, J = 6.8 Hz), 3.39-3.43 (2H, m), 3.62-3.73 (28H, m), 4.22-4.26 (1H, m), 4.42 (2H, d, J =7.2 Hz), 5.45 (1H, brs), 7.31-7.35 (2H, m), 7.38-7.47 (2H, m), 7.62 (2H, d, J = 7.2 Hz), 7.78 (2H, d, J = 7.2 Hz).

### 13) Preparation of Compound 9-5

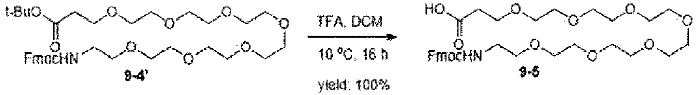

TFA (5.95 g, 52.2 mmol) was added to a solution of compound 9-4' (1.25 g, 1.85 mmol) in DCM (12 mL). The mixture was stirred at 10°C for 16 hours. Crude LC-MS showed that the product was 95% pure at a retention time of 0.816 (MS Calcd.: 619.3; MS Found: 642.0 [M+Na]+). The reaction mixture was concentrated in a vacuum to obtain Compound 9-5 (1.17 g, yield: 100%) as a colorless oil.

¹H NMR (400 MHz, DMSO-d₆) δ 2.43 (2H, t, J = 6.4 Hz), 3.13 (2H, dd, J = 11.6 Hz, 5.6 Hz), 3.40 (2H, t, J = 6.0 Hz), 3.48-3.49 (24H, m), 3.59 (2H, t, J = 6.4 Hz), 4.19-4.23 (1H, m), 4.29 (2H, d, J =6.4 Hz), 7.29-7.39 (3H, m), 7.42 (2H, t, J = 7.6 Hz), 7.69 (2H, d, J = 7.2 Hz), 7.89 (2H, d, J = 7.2 Hz). No active protons were observed.

### Preparation Example 6. Synthesis of PD010

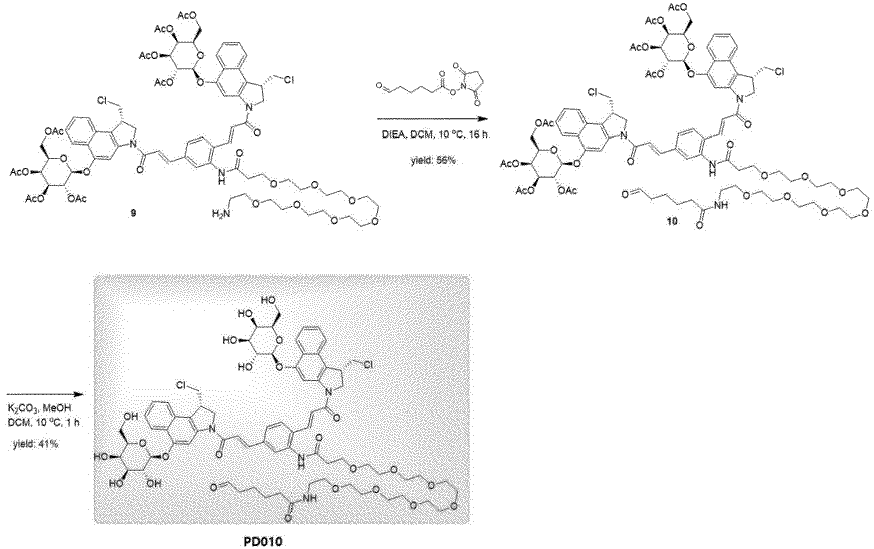

### 1) Preparation of Compound 10

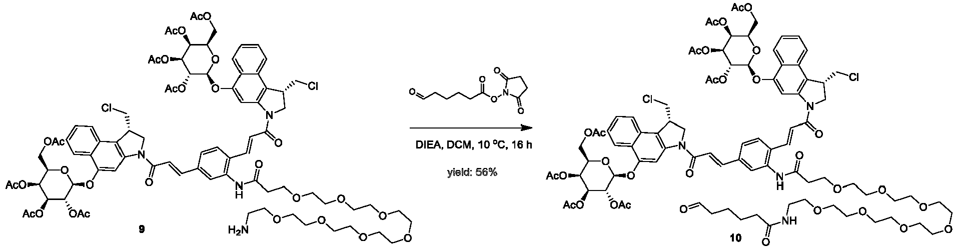

A mixture of Compound 9 (120 mg, 0.0704 mmol), (2,5-dioxopyrrolidin-1-yl) 6-oxohexanoate (24 mg, 0.11 mmol), and DIEA (18 mg, 0.14) in DCM (2 mL) mmol) was stirred at 10°C for 16 hours. TLC showed one major spot having lower polarity. The reaction mixture was purified by prep-TLC (EtOAc: MeOH of 20:1 as an eluent) to obtain compound 10 (72 mg, yield: 56 %) as a yellow solid.

¹H NMR (400 MHz, CDCl₃) δ 1.57-1.76 (4H, m), 2.01-2.10 (12H, m), 2.11-2.30 (14H, m), 2.41-2.50 (2H, m), 2.77-2.88 (2H, m), 3.36-3.76 (28H, m), 3.78-3.86 (2H, m), 3.90-4.07 (4H, m), 4.12-4.39 (8H, m), 4.45-4.63 (4H, m), 5.23 (2H, d, J = 10.0 Hz), 5.35-5.45 (2H, m), 5.56 (2H, s), 5.74 (2H, d, J = 9.2 Hz), 6.42 (1H, brs), 6.93-7.07 (2H, m), 7.40-7.50 (3H, m), 7.52-7.63 (2H, m), 7.69-7.78 (3H, m), 7.85 (1H, d, J = 15.6 Hz), 8.02 (1H, d, J = 14.8 Hz), 8.13-8.21 (3H, m), 8.43 (2H, d, J = 7.2 Hz), 9.10 (1H, brs), 9.75 (1H, s).

The synthesis of the (2,5-dioxopyrrolidin-1-yl) 6-oxohexanoate compound is the same as in Preparation Example of PD006. The synthesis of Compound 9 is the same as in Preparation Example of PD009.

### 2) Preparation of PD010

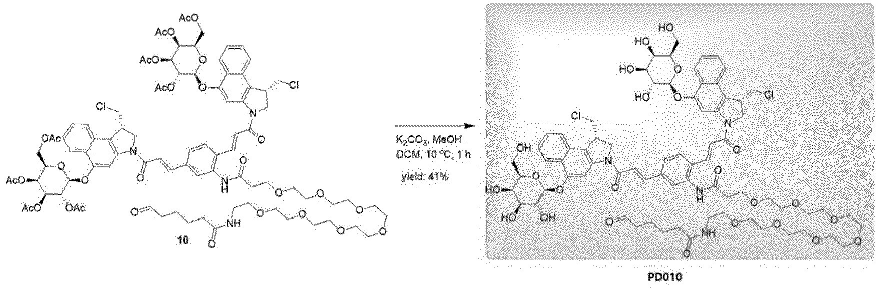

A mixture of Compound 10 (72 mg, 0.040 mmol) and K₂CO₃ (11 mg, 0.079 mmol) in DCM (1 mL) and MeOH (2 mL) was stirred at 10°C for 1 hour. Crude LC-MS showed that the product had a purity of 91% at a retention time of 0.831 (MS Calcd.: 1478.5; MS Found: 1481.1 [M+3H]⁺). The reaction mixture was quenched with AcOH (12 mg). The resulting mixture was purified by prep-HPLC (0.225 % FA). Most of the MeCN was removed under reduced pressure. The remaining mixture was lyophilized to obtain PDO10 (24 mg, yield: 41%) as a yellow solid. This batch (24 mg) was mixed with a batch of es8455-193-p1 (2 mg). Then, a total of 26 mg of PDO10 was obtained.

1H NMR (400 MHz, DMSO) δ 1.30-1.54 (4H, m), 1.95-2.10 (2H, m), 2.63-2.78 (2H, m, overlap DMSO signal), 3.08-3.24 (2H, m, overlap water signal), 3.25-4.11 (46H, m), 4.26-4.42 (2H, m), 4.45-4.77 (8H, m), 4.81-5.10 (4H, m), 5.29-5.54 (2H, brs), 7.18-7.33 (2H, m), 7.36-7.49 (2H, m), 7.51-7.62 (2H, m), 7.64-7.96 (7H, m), 8.04-8.14 (1H, m), 8.26-8.47 (4H, m), 9.64 (1H, s), 10.00 (1H, s).

### Preparation Example 7: Synthesis of PD011

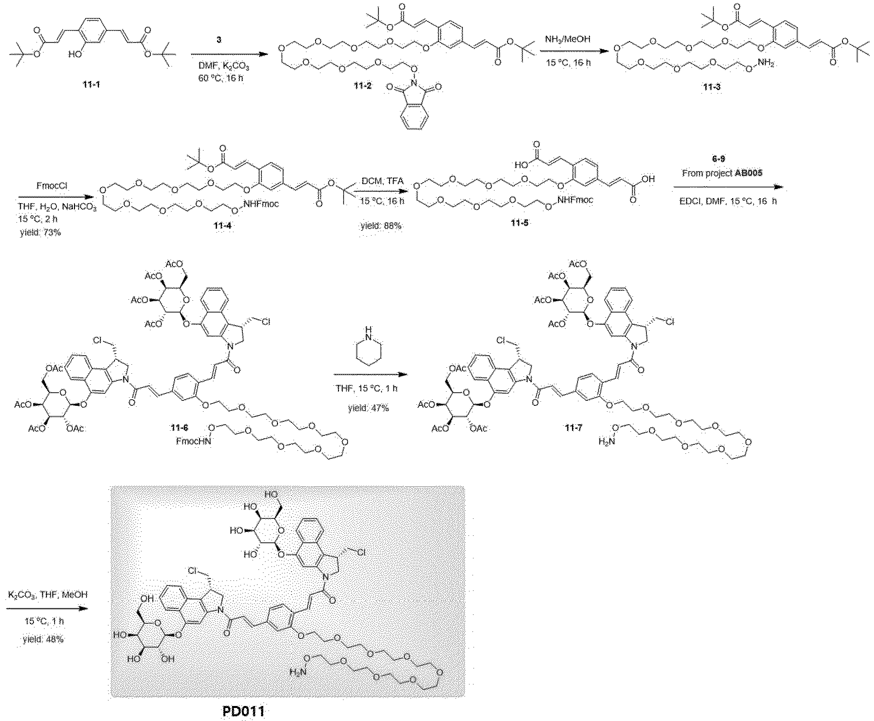

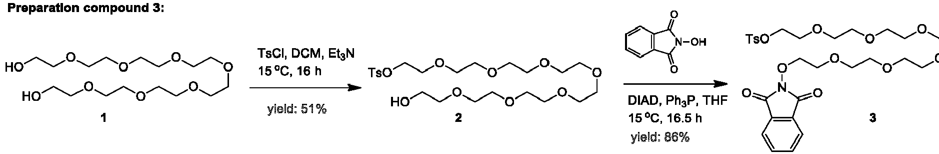

### 1) Preparation of Compound 11-2

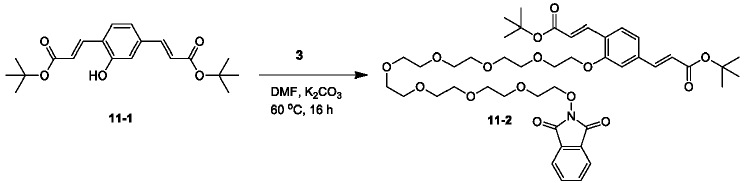

A mixture of compound 11-1 (1.30 g, 1.94 mmol), compound 3 (672 mg, 1.94 mmol), and K₂CO₃ (322 mg, 2.33 mmol) in DMF (6 mL) was stirred at 60°C for 16 hours. TLC showed that the starting material of Compound 11-1 still remained. A new spot was observed. The mixture was quenched with water (50 mL) and extracted with DCM (20 mL * 3). The combined organic layer was washed with water (50 mL), dried over Na₂SO₄, and concentrated to dryness. The residue was purified in a CombiFlash eluents having EtOAc : PE / 1:1 and EtOAc to obtain crude Compound 11-2 (710 mg, containing DMF) as a colorless oil.

¹H NMR (400 MHz, CDCl₃) δ 1.55 (18H, d, *J* = 4.0 Hz), 3.56-3.73 (22H, m), 3.75-3.80 (2H, m), 3.86-3.90 (2H, m), 3.92-3.96 (2H, m), 4.21-4.26 (2H, m), 4.37-4.42 (2H, m), 6.30 (1H, d, *J* = 16.0 Hz), 6.52 (1H, d, *J* = 16.0 Hz), 7.04 (1H, s), 7.12 (1H, d, *J* = 8.4 Hz), 7.48-7.57 (2H, m), 7.75-7.79 (2H, m), 7.84-7.91 (3H, m).

The synthesis of compound 11-1 was the same as in Example of PD001.

### 2) Preparation of Compound 11-3

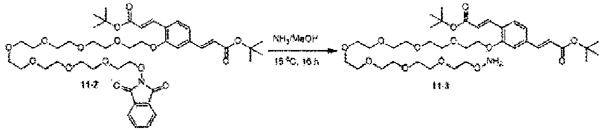

A solution of NH₃ and Compound 11-2 (738 mg, crude) in MeOH (7 M, 10 mL) was stirred at 15°C for 16 hours. A white precipitate was formed, and was then filtered. The filtrate was concentrated and dried to obtain Compound 11-3 (582 mg) as a colorless oil.

¹H NMR (400 MHz, DMSO) δ 1.48 (18H, d, *J* = 4.4 Hz), 3.46-3.54 (22H, m), 3.55-3.66 (6H, m), 3.79-3.85 (2H, m), 4.24-4.29 (2H, m), 4.37-4.42 (2H, m), 5.98 (2H, brs), 6.60-6.71 (2H, m), 7.28 (1H, d, *J* = 7.6 Hz), 7.45 (1H, s), 7.53 (1H, d, *J* = 16.0 Hz), 7.73 (1H, t, *J* = 8.0 Hz), 7.81 (1H, d, *J* = 18.4 Hz).

### 3) Preparation of Compound 11-4

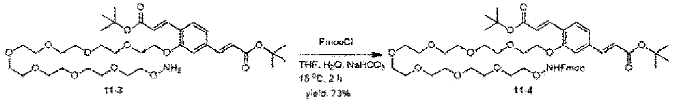

A mixture of compound 11-3 (580 mg, 0.812 mmol), Fmoc-Cl (231 mg, 0.894 mmol), and NaHCO₃ (75 mg, 0.89 mmol) in H₂O (5 mL) and THF (5 mL) was stirred at 15°C for 2 hours. Crude LC-MS showed that the product had a purity of 43% at a retention time of 1.003 (MS Calcd: 935.5; MS Found: 958.5 [M+Na]⁺) . The mixture was extracted with EtOAc (6 mL * 4). The combined organic layer was dried over Na₂SO₄, concentrated, and further dried. The residue was purified in a silica gel column using PE: EtOAc / 1:1, EtOAc, and then EtOAc: MeOH / 10:1 as eluents to obtain compound 11-4 (558 mg, yield: 73%) as a colorless oil.

¹H NMR (400 MHz, CDCl₃) δ 1.55 (18H, d, *J* = 4.8 Hz), 3.61-3.79 (26H, m), 3.90-3.96 (2H, m), 4.01-4.06 (2H, m), 4.19-4.30 (3H, m), 4.50 (2H, d, *J* = 6.8 Hz), 6.38 (1H, d, *J* = 16.0 Hz), 6.52 (1H, d, *J* = 16.0 Hz), 7.03 (1H, s), 7.12 (1H, d, *J =* 7.6 Hz), 7.31-7.36 (2H, m), 7.42 (1H, t, *J* = 7.6 Hz), 7.48-7.57 (2H, m), 7.62 (1H, d, *J* = 7.6 Hz), 7.78 (1H, d, *J* = 7.2 Hz), 7.88 (1H, d, *J* = 16.0 Hz), 8.27 (1H, brs).

### 4) Preparation of compound 11-5

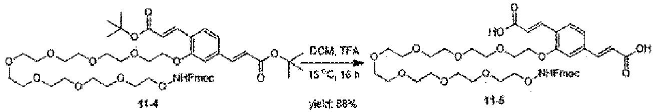

A solution of Compound 11-4 (558 mg, 0.596 mmol) and TFA (3.08 g, 27.0 mmol, 2 mL) in DCM (5 mL) was stirred at 15°C for 16 hours. Crude LC-MS showed that the product had a purity of 85% at a retention time of 0.827 (MS Calcd.: 823.3; MS Found: 846.3 [M+Na]⁺). The solvent was removed under reduced pressure. The residue was triturated with PE:EtOAc/1:1 (10 mL) to obtain Compound 11-5 (433 mg, yield: 88%) as a white solid.

¹H NMR (400 MHz, DMSO) δ 3.41-3.87 (30H, m, overlap water signal), 4.21-4.32 (3H, m), 4.40 (2H, d, *J* = 6.8 Hz), 6.59-6.72 (2H, m), 7.26-7.37 (3H, m), 7.38-7.47 (3H, m), 7.58 (1H, d, *J* = 16.0 Hz), 7.65-7.75 (3H, m), 7.80 (1H, d, *J* = 16.4 Hz), 7.89 (2H, d, *J* = 7.6 Hz), 10.46 (1H, brs), 12.43 (2H, brs).

### 5) Preparation of compound 11-6

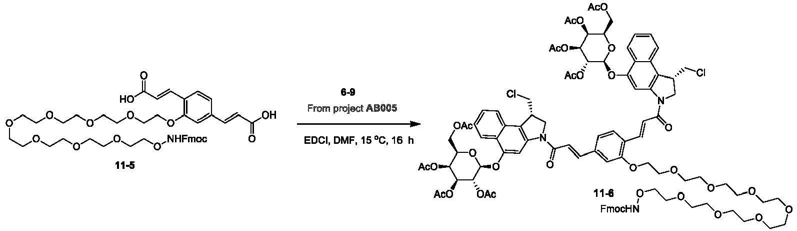

EDCI (168 mg, 0.874 mmol) was added at 15°C to a mixture of Compound 11-5 (240 mg, 0.291 mmol) and Compound 6-9 (493 mg, crude) in DMF (3 mL). Next, the mixture was stirred at 15°C for 16 hours. TLC showed one major yellow spot. Most of the DMF was removed under reduced pressure. The residue was purified in a silica gel column using EtOAc and then EtOAc : MeOH / 25 : 1 as eluents to obtain the product (541 mg) as a yellow solid. About 165 mg of the crude product was used in the next step (de-Fmoc reaction), but no positive results were obtained. Then, the residue (375 mg) was dissolved in EtOAc (40 mL) and washed with water (40 mL * 3). The organic layer was separated, dried over Na₂SO₄, and concentrated to dryness to obtain pure Compound 11-6 (340 mg).

¹H NMR (400 MHz, CDCl₃) δ 2.00-2.11 (12H, m, overlap EtOAc signal), 2.17 (6H, d, *J* = 11.2 Hz), 2.24 (6H, s), 3.45-3.84 (28H, m), 3.97-4.40 (19H, m, overlap EtOAc signal), 4.42-4.63 (5H, m), 5.18-5.26 (2H, m), 5.36-5.44 (2H, m), 5.52-5.60 (2H, m), 5.70-5.80 (2H, m), 6.97 (1H, d, *J* = 15.2 Hz), 7.17 (1H, s), 7.25-7.37 (4H, m, overlap CDCl₃ signal), 7.38-7.49 (4H, m), 7.53-7.64 (5H, m), 7.69-7.79 (4H, m), 7.83 (1H, d, *J* = 15.2 Hz), 8.00 (1H, d, *J* = 15.6 Hz), 8.14-8.20 (2H, m), 8.34 (1H, brs), 8.46 (1H, d, *J* = 11.6 Hz).

### 6) Preparation of Compound 11-7

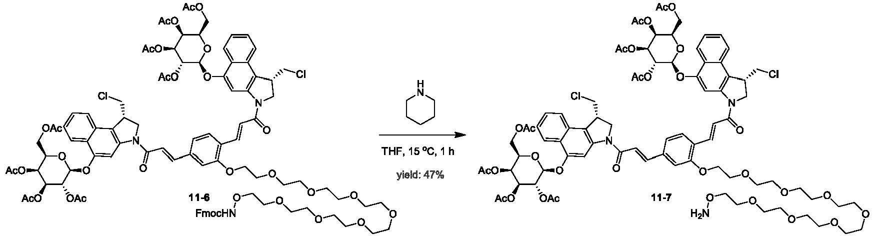

A solution of compound 11-6 (280 mg, 0.146 mmol) and piperidine (172 mg, 2.03 mmol, 0.2 mL) in THF (2 mL) was stirred at 15°C for 1 hour. TLC showed that one new spot was observed. The starting material was completely consumed. The solvent was removed under reduced pressure. The residue was purified by prep-TLC using EtOAc:MeOH/10:1 as an eluent to obtain Compound 11-7 (116 mg, yield: 47 %) as a yellow solid.

¹H NMR (400 MHz, CDCl₃) δ 2.06 (12H, d, *J* = 4.4 Hz), 2.17 (6H, d, *J* = 10.4 Hz), 2.24 (6H, s), 3.47-3.85 (30H, m), 3.97-4.63 (18H, m), 5.17-5.27 (2H, m), 5.35-5.44 (2H, m), 5.52-5.62 (2H, m), 5.70-5.80 (2H, m), 6.98 (1H, d, *J* = 15.2 Hz), 7.18 (1H, s), 7.26-7.37 (2H, m, overlap CDCl ₃ signal), 7.40-7.49 (2H, m), 7.52-7.65 (3H, m), 7.73 (2H, d, *J* = 7.6 Hz), 7.84 (1H, d, *J* = 15.2 Hz), 8.00 (1H, d, *J* = 15.2 Hz), 8.13-8.21 (2H, m), 8.45 (2H, d, *J* = 11.6 Hz).

### 7) Preparation of PD011

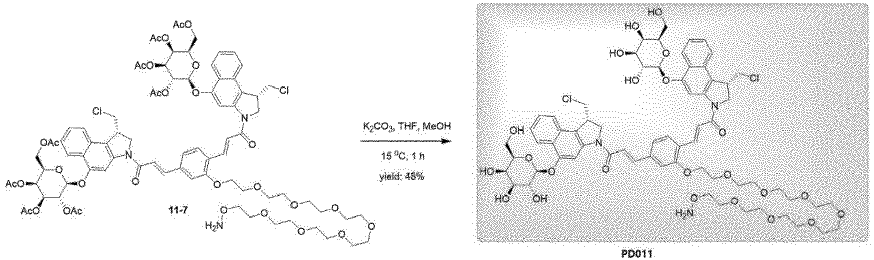

A mixture of Compound 11-7 (116 mg, 0.068 mmol) and K₂CO₃ (19 mg, 0.146 mmol) in MeOH (1 mL) and THF (1 mL) was stirred at 15°C for 1 hour. Crude LC-MS showed that the product had a purity of 86% at a retention time of 0.772 (MS Calcd: 1355.5; MS Found: 1357.2 [M+2]⁺). The mixture was quenched with AcOH (50 mg). The mixture was purified by prep-HPLC (0.225% FA). Most of the MeCN was removed under reduced pressure, and the remaining aqueous solution was lyophilized to obtain PD011 (45 mg, yield: 48%) as a yellow solid.

¹H NMR (400 MHz, DMSO) δ 3.18-4.10 (48H, m, overlap water signal), 4.27-4.72 (12H, m), 4.87-5.02 (2H, m), 5.39 (2H, brs), 7.30-7.64 (8H, m), 7.74 (1H, d, *J* = 15.2 Hz), 7.81-7.97 (4H, m), 8.28-8.49 (4H, m).

### 8) Preparation of Compound 2

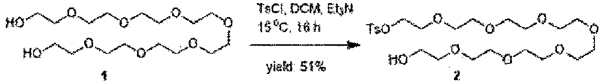

TosCl (2.57 g, 13.5 mmol) was added at 15°C to a solution of octaethylene glycol (5.00 g, 13.5 mmol) and Et₃N (1.37 g, 13.5 mmol) in DCM (50 mL). Next, the mixture was stirred at 15°C for 16 hours. TLC showed that the starting material was consumed and that two new spots were formed. The mixture was quenched with water (50 mL) and the organic layer was separated. The remaining aqueous phase was extracted with DCM (30 mL * 2). The mixed organic layer was dried over Na₂SO₄, concentrated, and further dried. The residue was purified in a CombiFlash apparatus using EtOAc and then EtOAc : MeOH / 9:1 as eluents to obtain Compound 2 (3.59 g, yield: 51 %) as a colorless oil.

¹H NMR (400 MHz, CDCl₃) δ 3.59-3.76 (30H, m), 4.18 (2H, t, *J* = 4.8 Hz), 7.36 (2H, d, *J* = 8.0 Hz), 7.82 (2H, d, *J* = 8.4 Hz).

### 9) Preparation of Compound 3

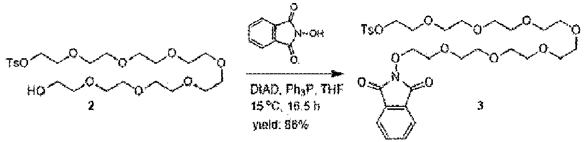

2-hydroxyisoindoline-1,3-dione (1.11 g, 6.82 mmol) and PPh3 (2.33 g, 8.87 mmol) were added in the presence of N₂ at 0°C to a solution of Compound 2 (3.58 g, 6.82 mmol) in THF (30 mL). Next, the mixture was stirred at 0°C for 30 minutes. Then, DIAD (1.66 g, 8.19 mmol) at 0°C was added to the mixture. The resulting mixture was stirred in the presence of N₂ at 15°C for 16 hours. TLC showed a new spot. Then, the solvent was removed under reduced pressure. The residue was purified in a CombiFlash apparatus using PE:EtOAc/1:1 and EtOAc as eluents to obtain Compound 3 (3.91 g, yield: 86%) as a colorless oil.

¹H NMR (400 MHz, CDCl₃) δ 2.47 (3H, s), 3.56-3.73 (26H, m), 3.86-3.91 (2H, m), 4.16-4.20 (2H, m), 4.37-4.42 (2H, m), 7.36 (2H, d, *J* = 7.6 Hz), 7.75-7.88 (6H, m).

### Preparation 8: Synthesis of PD012

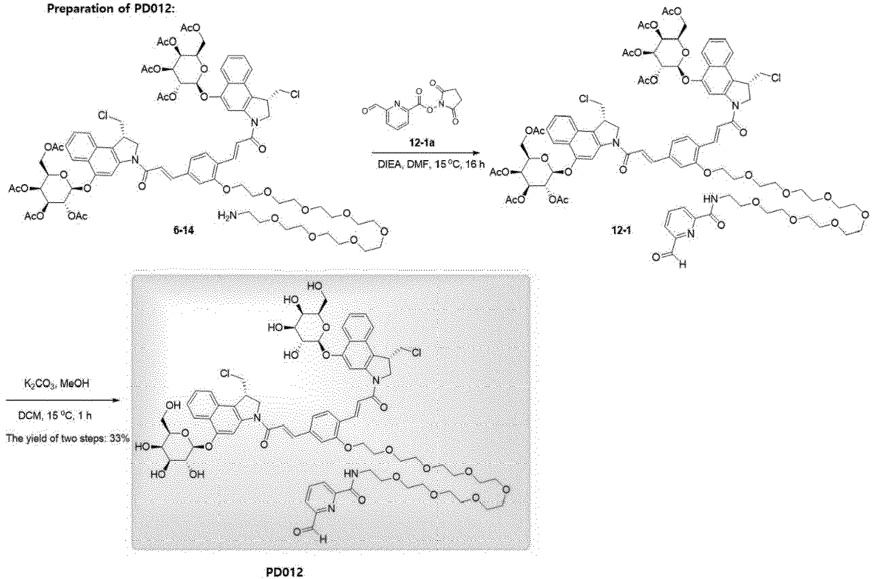

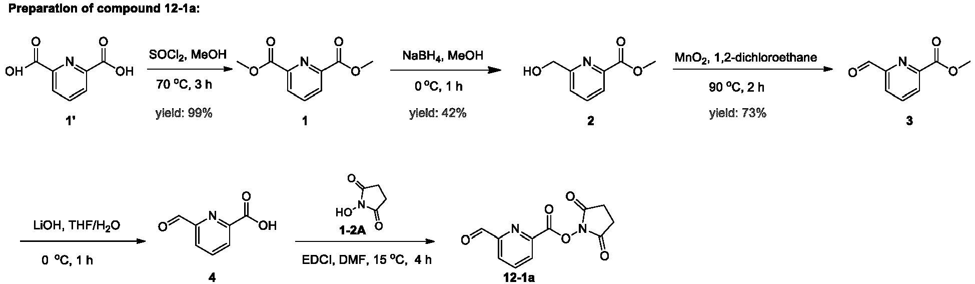

### 1) Preparation of compound 12-1

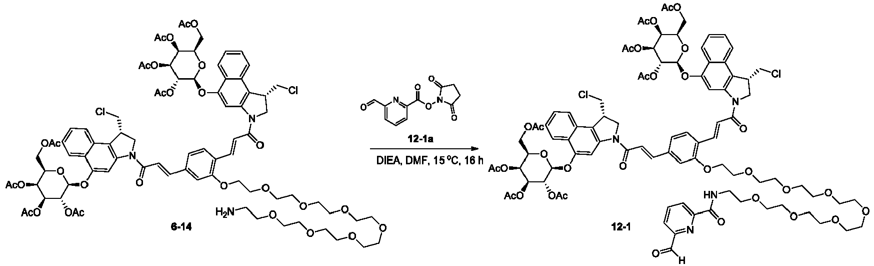

DIEA (13 mg, 0.10 mmol) was added to a solution of Compound 6-14 (107 mg, 0.0638 mmol) and Compound 12-la (25 mg, crude oil) in DMF (2 mL). The mixture was stirred at 15°C for 16 hours. TLC showed another yellow spot having lower polarity. DMF was removed in a vacuum. The residue was quenched in water (10 mL). The resulting solution was extracted in DCM (15 mL * 4). The combined organic layer was washed with water (50 mL), and the organic layer was dried over anhydrous Na₂SO₄, filtered, and then concentrated in a vacuum. The residue was purified through prep-TLC using EtOAc:MeOH/10:1 as an eluent to obtain Compound 12-1 (81 mg, crude oil) as a yellow solid.

¹H NMR (400 MHz, CDCl₃) δ 1.94-2.08 (12H, m), 2.09-2.24 (12H, m), 3.50-3.84 (32H, m), 3.98-4.37 (13H, m), 4.43-4.62 (4H, m), 5.20 (2H, d, *J* = 10.0 Hz), 5.38 (2H, d, *J* = 7.2 Hz), 5.50-5.57 (2H, m), 5.72 (2H, t, *J* = 8.8 Hz), 6.96 (1H, d, *J* = 15.2 Hz), 7.15 (1H, s), 7.27-7.35 (2H, m, overlap CDCl ₃ signal), 7.39-7.47 (2H, m), 7.51-7.62 (4H, m), 7.71 (2H, d, *J* = 8.0 Hz), 7.81 (1H, d, *J* = 15.2 Hz), 7.94-8.09 (2H, m), 8.11-8.20 (2H, m), 8.36-8.48 (3H, m), 10.07 (1H, s).

The synthesis of compound 6-14 was the same as in Example of PD006.

### 2) Preparation of PD012

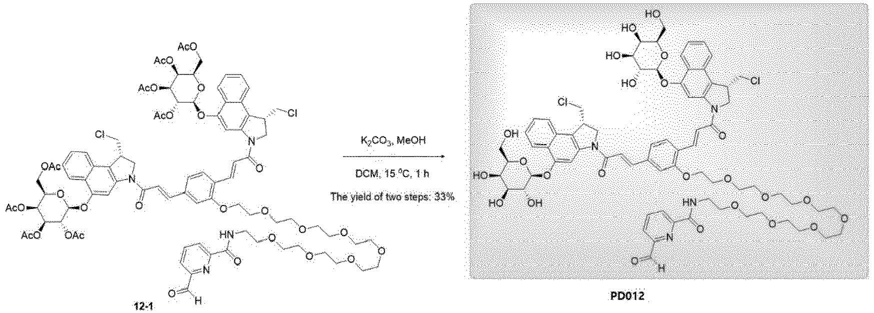

K₂CO₃ (12 mg, 0.090 mmol) was added to a solution of compound 12-1 (81 mg, crude) in DCM (0.2 mL) and MeOH (0.4 mL). The mixture was stirred at 15°C for 1 hour. Crude LC-MS showed that the product had a purity of 99% at a retention time of 0.787 (MS Calcd.: 1474.2; MS Found: 1496.2 [M+Na]⁺). The reaction mixture was concentrated in a vacuum. The residue was purified by prep-HPLC (0.225% FA). Most of the MeCN was removed under reduced pressure. The remaining mixture was lyophilized to obtain PD012 (31 mg, yield of 2 steps: 33%) as a yellow solid.

¹H NMR (400 MHz, DMSO-d₆) δ 3.44-3.68 (28H, m), 3.71-4.16 (15H, m), 4.26-4.82 (15H, m), 4.88-5.07 (4H, m), 5.39 (2H, brs), 7.25-7.64 (8H, m), 7.57 (1H, d, *J* = 5.2 Hz), 7.81-8.02 (4H, m), 8.07 (1H, d, *J* = 7.6 Hz), 8.17-8.36 (4H, m), 8.37-8.46 (2H, m), 8.81 (1H, brs), 10.02 (1H, s).

### 3) Preparation of Compound 1

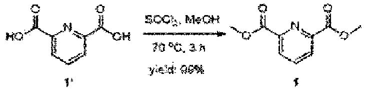

2,6-pyridinedicarboxylic acid (2.00 g, 12.0 mmol) was added at 0°C to a solution of SOCl₂ (8.83 g, 74.2 mmol) in MeOH (25 mL). The mixture was stirred at 70°C for 3 hours. Crude LC-MS showed that the product had a purity of 99% at a retention time of 0.752 (MS Calcd.: 195.1; MS Found: 195.9 [M+H]⁺) . The reaction mixture was quenched with *sat. aq.* NaHCO₃ (30 mL), and the resulting solution was extracted with EtOAc (30 mL*6). The combined organic layer was dried over anhydrous Na₂SO₄, filtered, and then concentrated in a vacuum to give compound 1 (2.33 g, yield: 99 %) as a white solid.

¹H NMR (400 MHz, CDCl₃) δ 4.03 (6H, s), 8.03 (1H, t, *J* = 8.0 Hz), 8.32 (2H, d, *J* = 7.2 Hz).

### 4) Preparation of Compound 2

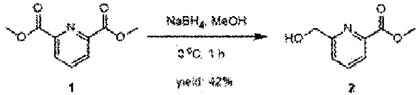

NaBH₄ (1.71 g, 45.1 mmol) was added portionwise at 0°C to a stirred solution of Compound 1 (2.33 g, 11.9 mmol) in MeOH (35 mL), and the mixture was stirred at 0°C for 1 hour. Crude LC-MS showed that the product had a purity of 97% at a retention time of 0.297 (MS Calcd.: 167.1; MS Found: 167.7 [M+H]⁺) . The reaction mixture was quenched with *sat. aq.* NaHCO₃ (200 mL), and the resulting solution was extracted with DCM (100 mL*6). The combined organic layers were dried over anhydrous Na₂SO₄, filtered, and concentrated in a vacuum. The residue was purified in a silica gel column using PE: EtOAc / 1:1 and then EtOAc as eluents to obtain Compound 2 (832 mg, Yield: 42 %) as a white solid.

¹H NMR (400 MHz, CDCl₃) δ 3.45 (1H, brs), 4.02 (3H, s), 4.88 (2H, d, *J* = 4.8 Hz), 7.55 (1H, d, *J* = 7.6 Hz), 7.87 (1H, t, *J* = 7.6 Hz), 8.06 (1H, d, *J* = 7.2 Hz).

### 5) Preparation of compound 3

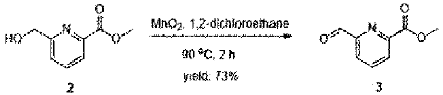

MnO₂ (4.33 g, 50.0 mmol) was added at 15°C to a solution of compound 2 (832 mg, 4.98 mmol) in 1,2-dichloroethane (25 mL), and the mixture was heated at 90°C for 2 hours. Crude LC-MS showed that the product had a purity of 98% at a retention time of 0.386 (MS Calcd.: 165.1; MS Found: 165.7 [M+H]⁺). The mixture was filtered through celite and the filtrate was evaporated to obtain Compound 3 (606 mg, yield: 73%) as a white solid.

¹H NMR (400 MHz, CDCl₃) δ 4.10 (3H, s), 8.08 (1H, t, *J* = 7.6 Hz), 8.18 (1H, d, *J* = 6.8 Hz), 8.38 (1H, d, *J* = 7.6 Hz), 10.22 (1H, s).

### 6) Preparation of compound 4

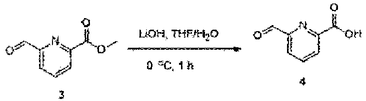

LiOH (132 mg, 5.50 mmol) was added at 0°C to a solution of compound 3 (606 mg, 3.67 mmol) in THF (6 mL) and H₂O (6 mL) and the mixture was stirred at 0°C for 1 hour. TLC showed another new spot with greater polarity, and showed that the starting material was completely consumed. The reaction mixture was concentrated in a vacuum, and the remaining solution was quenched with 1N aq. HCl. The resulting solution was extracted with EtOAc : THF = 10 : 1 (20mL * 8) to obtain an impure product (466 mg) as a white solid. The impure product was triturated with TBME: PE = 1:1 (10 mL) and filtered, and the filter cake was dried under vacuum to obtain Compound 4 (444 mg, crude oil) as a white solid.

¹H NMR (400 MHz, DMSO-d₆) δ 8.09 (1H, dd, *J* = 7.6 Hz, 1.2 Hz), 8.20 (1H, t, *J =* 7.6 Hz), 8.29 (1H, dd, *J* = 7.6 Hz, 1.2 Hz), 10.03 (1H, s). (Note: No active proton was observed)

### 7) Preparation of Compound 12-la

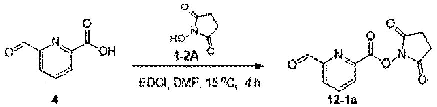

EDCI (136 mg, 0.708 mmol) was added in the presence of N₂ at 15°C to a solution of Compound 4 (100 mg, crude) and N-hydroxysuccinimide (80 mg, 0.695 mmol) in DMF (1.5 mL), and the mixture was stirred at 15°C for 4 hours. Crude LC-MS showed that the product had a purity of 96% at a retention time of 1.289 (MS Calcd.: 248.1; MS Found: 248.9 [M+H]⁺). DMF was removed in a vacuum, and the residue was purified in a silica gel column using PE:EtOAc/2:3 as an eluent to obtain Compound 12-la (51 mg impure) as a white solid.

### Preparation Example 9: Synthesis of PD013

A linker was prepared according to the method described in US Patent No. 9,636,421 of Synaffix. A linker-drug complex was prepared using the prepared linker in the same manner as in Preparation Example 8.

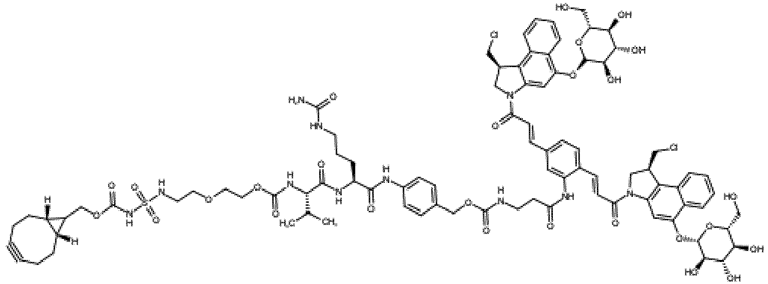

(PD013)

### Example 1. Overexpression of beta-galactosidase in tumor cell lines compared to normal cells

Beta-galactosidase expression in tumor cell lines was identified by Western blotting. 15 cell lines (1x10⁷ each) were washed with PBS and then centrifuged to obtain cell pellets. The cells were lysed with an M-PER^{®} mammalian protein extract reagent (Thermo, 78501), and proteins contained in the cells were extracted. 200 µl of the M-PER^{®} mammalian protein extract reagent was added to the cell pellets, pipetted, and allowed to react for 10 minutes. The reaction solution was centrifuged and the supernatant was collected. The concentration of proteins in the supernatant was measured using a BCA protein assay kit (Pierce, 23225). 10 ug of the protein extract of each cell line was subjected to SDS-PAGE electrophoresis. After electrophoresis, the protein was transferred to the PVDF membrane (Invitrogen, IB401002) using an iBlot transfer system. The membrane was blocked with 4% BSA (PBS) at room temperature for 2 hours, treated with anti-beta-galactosidase antibody (1/10000, abcam, ab128993), and allowed to react at 4°C for 15 hours. The reaction product was washed 3 times with 1X PBST (1XPBS + 0.05% Tween20), treated with Anti-Rabbit IgG HRP (1/3000, pierce, 65-6120), and allowed to react at room temperature for 1 hour. The resulting product was washed 3 times with 1X PBST and treated with ECL solution (GE healthcare, RPN2232), and the protein was detected. The membrane that had been used to identify the expression of beta-galactosidase was stripped to identify the expression of β-actin. The stripped membrane was blocked with 4% BSA (PBS) at room temperature for 2 hours and then allowed to react with an anti-actin antibody (1/1000, Santacruz, SC-47778) at 4°C for 15 hours. The membrane was washed 3 times with 1X PBST (1XPBS + 0.05% Tween20), treated with anti-mouse IgG HRP (1/10000, Pierce, 31432), and allowed to react at room temperature for 1 hour. The reaction product was washed 3 times with 1X PBST and treated with ECL solution, and the protein was detected.

The result of Western blotting showed that beta-galactosidase was expressed in 15 tumor cell lines. In addition, the result of analysis of the TCGA (The Cancer Genome Atlas) data showed that expression of beta-galactosidase was high in a mRNA level in tumor tissues compared to normal tissues (FIG. 2). FIG. 2 shows the mRNA expression proportion of beta-galactosidase gene GLB1 in tumor tissues and normal tissues, and indicates that beta-galactosidase is highly expressed in tumor tissues compared to normal tissues. In particular, in the case of uterine cancer, the expression of GLB1 was 2 times higher in tumor tissues than in normal tissues, and the breast cancer tissues also overexpressed GLB1 by more than 68%. This demonstrated that beta-galactosidase, which is highly expressed in tumor cells, can be used as a tumor-selective activating enzyme.

### Example 2. Evaluation of efficacy of linker-drug complex

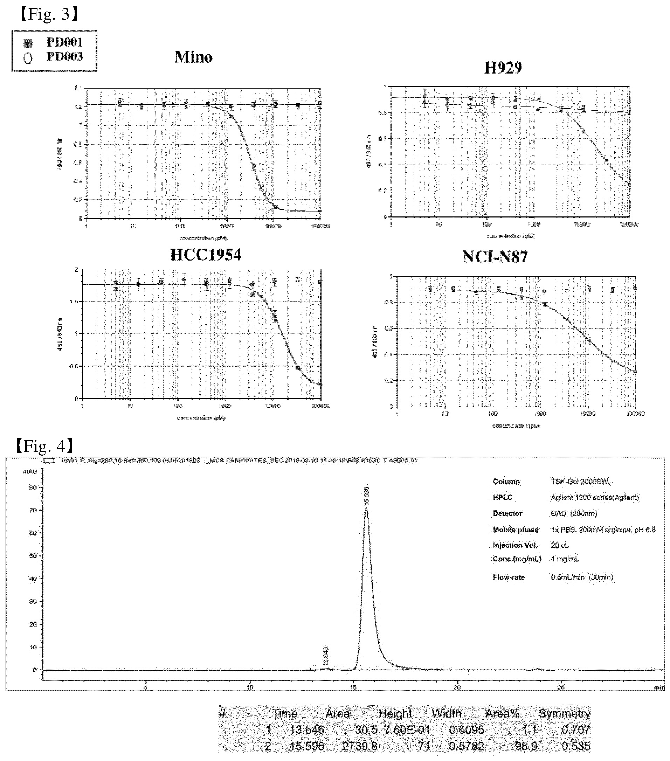

The cytotoxicity of the linker-drug complex itself prepared to contain beta-galactosidase was evaluated. Specifically, each of the linker-drug complexes dissolved in DMSO was serially diluted 3x from 200 nM to 30 pM in a medium for cell culture to prepare a sample, and various cancer cell lines were treated with the sample to finally expose the cells to 100 nM to 15 pM the linker-drug complexes. The cells were cultured for 6 days, cell viability was observed using a CCK-8 cell-counting kit and cytotoxicity was evaluated. The results of evaluation are shown in brief in Table 2, and a representative reaction curve is shown in FIG. 3. As can be seen in Table 2 and in FIG. 3, PD003 was not cytotoxic to most cells, and as a result, IC50 could not be determined. On the other hand, PD001 was cytotoxic to multiple cells, and the IC50 is shown in Table 2.

These experimental results indicated that sufficient beta-galactosidase, which degrades beta-galactose, was present in cancer cells and thus PD001 was able to be activated by beta-galactosidase in various cancer cells and was able to exhibit cytotoxicity. The fact that PD003 was inactive indirectly suggests that the beta-glucose moiety cannot be used as a substrate for beta-glucosidase. In addition, the examples given below show that PD001 prepared in the form of ADC was more cytotoxic than a single substance, which supports the notion that the ADC form is much more efficient for drug delivery and activation.

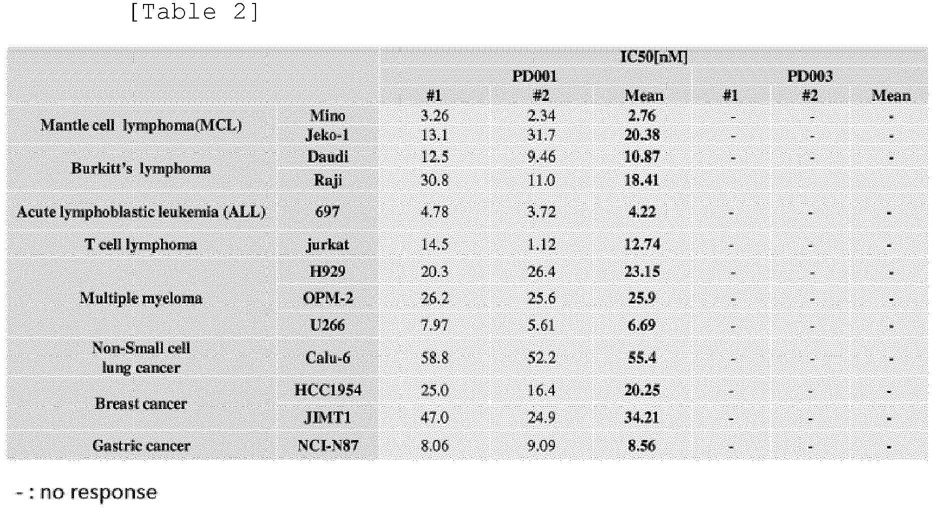

### Example 3. Preparation of antibody-drug conjugate (ADC) in which antibody is bound to drug

For the preparation of ADC in which an antibody is bound to a drug, lysine (K) at position 149 (according to Kabat numbering, which also applies below) of the existing antibody light chain is mutated to cysteine (C) and allowed to react with a reducing agent such as dithiothreitol (DTT) to generate a thiol group on the antibody light chain K153C (K153C T), and the antibody was conjugated with the drug through the generated disulfide bond between the thiol group and the drug. Specifically, the antibody was prepared at a concentration of 5 mg/ml or more through ultrafiltration/diafiltration (UF/DF), and 1M tris(hydroxymethyl)aminomethane (Tris-HCl), and pH 8.8, 500 mM ethylene diaminetetraacetic acid (EDTA) were added thereto to adjust the final antibody concentration to 5 mg/ml, and obtain 75 mM Tris-HCl, and 2 mM EDTA. 100 mM dithiothreitol (DTT) was added to the prepared antibody such that the molar ratio of the antibody to DTT was adjusted to 1:20, and allowed to react at 25°C for 16.5 hours to remove free cysteine linked through a disulfide bond at cysteine 149 of the antibody light chain. This process is called "decapping", and was followed by cation exchange chromatography (CEX) as a purification method to isolate the decapped antibody. The reaction product was eluted on a HiTrap SPHP column (GE Healthcare) equilibrated with SPHP-A buffer [10 mM succinate, pH 5.0] with SPHP-B buffer [50 mM Tris(hydroxymethyl)aminomethane (Tris-HCl), pH 7.5, 0.5M sodium chloride]. The antibody to be oxidized was prepared in 75 mM Tris-HCl (pH7.5) using 1M tris(hydroxymethyl)aminomethane (Tris-HCl, pH7.5) to re-bind the decapped antibody, dehydroascorbic acid (DHAA), an oxidized vitamin C, was added at a ratio of antibody to DHAA of 1:20 and reoxidized in the dark at 25°C for 2 hours. Then, to isolate the reoxidized antibody, the result was eluted with SPHP-C buffer [10 mM succinate pH 5.0, 0.5 M sodium chloride] using a cation exchange chromatography (CEX) purification method. The purified antibody was concentrated to 5 mg/ml or more through ultrafiltration/diafiltration (UF/DF). Then, to produce an antibody-drug conjugate, the antibody to be reacted was prepared in 100 mM tris(hydroxymethyl)aminomethane (Tris-HCl, a final concentration: 5 mg/ml, pH 8.0), with 1M tris(hydroxymethyl)aminomethane (Tris-HCl, pH 8.0), was added such that the molar ratio of the antibody to the drug was 1:10, and was then allowed to react at 25°C for 16.5 hours. Then, to isolate the antibody-drug conjugate, elution was performed with an SPHP-C buffer [10 mM succinate pH 5.0, 0.5 M sodium chloride] using a cation exchange chromatography (CEX) purification method. Then, hydrophobic interaction chromatography (HIC) was used to isolate the DAR2 antibody-drug conjugate. For this purpose, a HiTrap butyl HP column (GE Healthcare) was equilibrated with HIC-A buffer [50 mM potassium phosphate, pH 7.0, 1.0 M ammonium sulfate] and was eluted with HIC-B buffer [50 mM potassium phosphate, pH 7.0, 30% isopropyl alcohol (2-propanol)]. Then, the antibody was exchanged with HA buffer [20 mM Histidine, pH 5.5, 240 mM sucrose) through ultrafiltration/diafiltration (UF/DF) to remove isopropyl alcohol (2-propanol) and thereby prepare a final antibody-drug conjugate.

Table 3 shows names and specific configurations of the ADC according to the present invention, the ADC using D-glucose β-pyranose or glucuronide as a trigger, and the ADCs linked to different drugs. The VH and VL sequences of each antibody are as shown in Table 1.

**[Table 3]**

| ADC name | Antibody | Linker-drug |
|---|---|---|
| D20103 (B58-PD001) | B58 (anti-BCMA antibody) | PD001 |
| D20204 (C2E3-PD001) | C2E3 (anti-ROR1 Ab) | PD001 |
| D20001 (T-PD001) | Trastuzumab (anti-HER2 Ab) | PD001 |
| D20002 (10H1-PD001) | 10H1 (anti-NaPi2b antibody) | PD001 |
| D20106 (B58-PD006) | B58 (anti-BCMA antibody) | PD006 |
| D20109 (B58-PD009) | B58 (anti-BCMA antibody) | PD009 |
| D20502 (6E7-PD009) | 6E7 (anti-CLL1 antibody) | PD009 |
| C2E3-PD003 | C2E3 (anti-ROR1 Ab) | PD003 (trigger: D-glucose β-pyranose) |
| C2E3-CAAX-MMAE | C2E3 (anti-ROR1 Ab) | MMAE |
| C2E3-mc-MMAF | C2E3 (anti-ROR1 Ab) | MMAF |
| D20111 | B58 (anti-BCMA antibody) | GT70 (trigger: glucuronide) |

### Example 4. Characterization of ADC

### 4-1. Detection of purity of prepared ADC

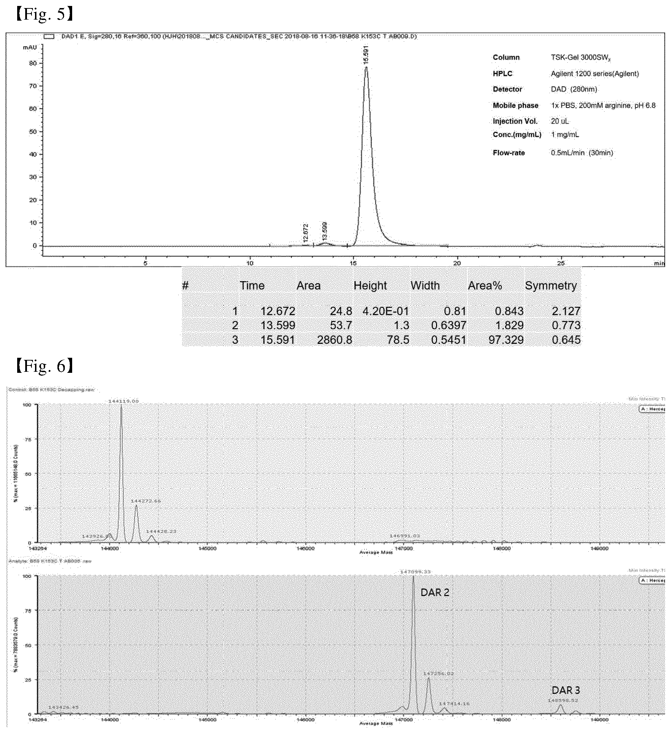

The purity of the prepared ADC was measured by size exclusion-high performance liquid chromatography (SE-HPLC). The position at which each sample is eluted and the area under the curve (AUC) were compared using a Tosoh TSKgel G3000SWxl column (Tosoh bioscience) on an Agilent 1200 series HPLC instrument, to determine the purity of D20106 and D20109. It was confirmed that the purity of the main peak of the ADC was 97% or more, and the overall process yield (based on antibody) was 20% or more. These results are shown in FIGS. 4 and 5, respectively.

### 4-2. Detection of drug-antibody ratio of prepared ADC

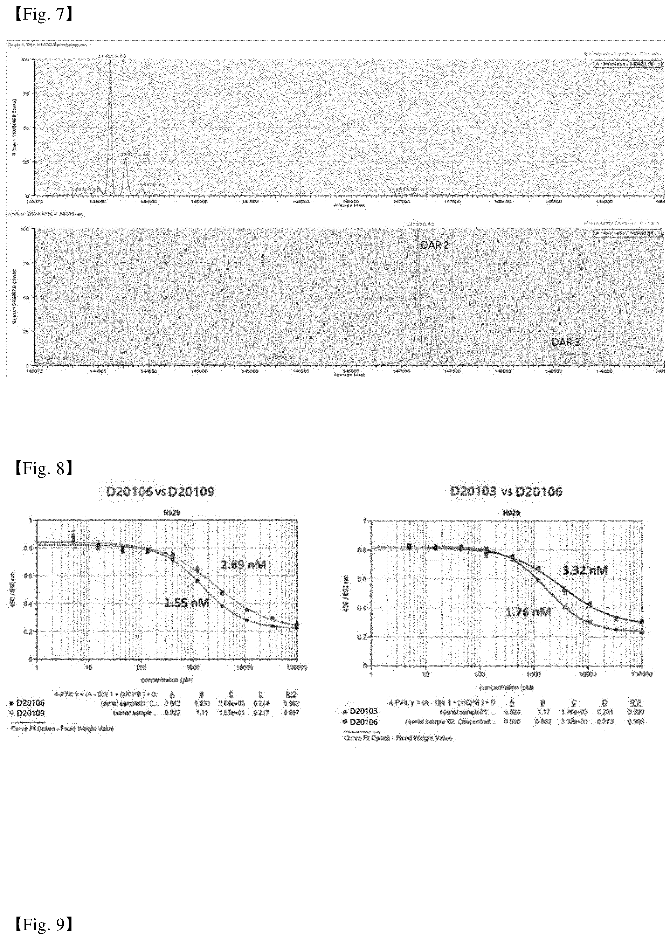

The drug-to-antibody ratio (DAR) of ADC was determined by a liquid chromatography-mass spectrometry (LC/MS) method. 1 unit of PNGaseF (NEB) per 100 µg of 1 mg/ml ADC (in PBS) antibody was added, followed by incubation at 37°C for 15 hours and removal of N-glycan. An Acquity UPLC BEH200 SEC 1.7 µm (4.6*150 mm) column was mounted on an LC/MS device including Waters UPLC I-class equipment and Waters Synapt G2-S, and equilibration was performed with a mobile phase of 30% (v/v) acetonitrile, 0.1% (v /v) formic acid, and 0.05% trifluoroacetic acid (TFA) . 5 µg of the sample from which N-glycan was removed was loaded thereon and LC/MS was performed. A weighted average of the relative content of each chemical species was calculated from the molecular weight distribution obtained as a result of LC/MS, to determine the DAR. These results are shown in FIGS. 6 and 7, respectively. As shown in FIGS. 6 and 7, the DAR of D20106 and D20109 was about 2. These results showed that PD006 and PD009 were linked to the antibody with the expected number of DARs.

### 4-3. Detection of in-vitro target-specific cytotoxicity of the prepared ADC

### (1) In-vitro cytotoxicity in multiple myeloma cell lines

The *in-vitro* cytotoxicity of anti-BCMA antibody-containing ADCs, namely, D20103 (B58 PD001), D20106 (B58 PD006) and D20109 (B58 PD009), was detected using the BCMA-expressing multiple myeloma cell line, MM NCI-H929. Specifically, 50 µl of the H929 cell line (ATCC, CRL-9068^{™}) cultured in a medium containing RPMI (ATCC), 10% FBS (Gibco), 0.05 mM beta-mercaptoethanol, and Antibiotic-Antimycotic (Gibco) was seeded at a density of 20,000 cells/well on a 96-well plate, and 50 µl of the ADC diluted in the same culture medium was seeded at each well. The concentration of ADC was changed from 200 nM to 5 pM through serial dilution. Then, the cells were incubated at 37°C and 5% CO₂ for about 6 days. After the incubation, 10 µl of WST-8 (Dojindo) was added to each well, followed by further incubation. Absorbance was measured at a wavelength of 450 nm using a SpectraMax plate reader. The response curve between the absorbance and the ADC concentration was subjected to 4PL curve fitting to calculate an IC₅₀ value (nM), which means a 50% apoptosis concentration. The results are shown in FIG. 8. All of D20103, D20106 and D20109 exhibited excellent potency, and in particular, D20109 including PD009 had the best efficacy. The amount of PD009 obtained after conjugation and then filtration was higher than in the case of PD006, and enables production of more ADCs with high DARs under the same conditions. In this regard, the following Table 4 shows the results of purification of the cation exchange resin after the reaction during the conjugation process. This process characteristic of PD009 is due to the relatively high solubility thereof, and PD009 is thus considered to be more process-friendly.

**[Table 4]**

| | **Fraction** | **Conc. (mg/mL)** | **vol. (mL)** | **Amount (m g)** | **DAR** | **Remarks** |
|---|---|---|---|---|---|---|
| D20106 | 1A2 | 0.23 | 32.90 | 7.57 | 2.90 | Total 263 mg |
| | 1A3 | 1.70 | 40.00 | 68.00 | 2.85 | |
| | 1B1 | 2.34 | 40.00 | 93.60 | 2.46 | |
| | 1B2 | 1.65 | 40.00 | 66.00 | 2.78 | |
| | 1B3 | 0.70 | 40.00 | 28.00 | 3.09 | |
| D20109 | 1A3∼1B1 | 0.29 | 27.40 | 7.95 | 3.05 | Total 304 mg |
| | 1B2 | 2.10 | 40.00 | 84.00 | 2.68 | |
| | 1B3 | 1.98 | 40.00 | 79.20 | 2.71 | |
| | 2A1 | 1.74 | 40.00 | 69.60 | 2.88 | |
| | 2A2 | 1.60 | 40.00 | 64.00 | 3.52 | |

### (2) In-vitro cytotoxicity in mantle cell lymphoma cell lines

The *in-vitro* cytotoxicity of D20204 (C2E3-PD001), an ADC containing an anti-ROR1 antibody, was detected using ROR1-expressing human mantle cell lymphoma cell lines, Mino and Jeko-1. Control groups used herein were (i) C2E3-PD003 that includes the same anti-ROR1 antibody, C2E3, but includes PD003, as a drug, containing not D-glucose β-pyranose but D-galactose β-pyranose as a trigger, and (ii) C2E3-CAAX-MMAE that includes C2E3, the same anti-ROR1 antibody, and includes MMAE a conventional drug. Specifically, 50 µl of each of the Jeko-1 cell line (ATCC, CRL-3006^{™}) and the Mino cell line (ATCC, CRL-3000^{™}) cultured in a medium containing RPMI (ATCC), 20% FBS (jeko-1) (Gibco) or 15% FBS (mino), and Antibiotic-Antimycotic (Gibco) was seeded at 20,000 cells/well on a 96-well plate, followed by performing a test for determining cytotoxicity. The subsequent experimental method was the same as that for the anti-BCMA ADC.

The results are shown in Table 5 and in FIG. 9. In the Mino cell line, D20204 was more cytotoxic than the control C2E3-CAAX-MMAE, and C2E3-PD003 containing glucose as a trigger was not cytotoxic. In Jeko-1, D20204 was slightly less cytotoxic than C2E3-CAAX-MMAE, and PD003 was not cytotoxic at all. This demonstrates that the ADC of the present invention containing galactose as a trigger exhibits significantly superior target-specific cytotoxicity compared to the ADC containing glucose as a trigger.

**[Table 5]**

| **IC50 (nM)** | **D20204** | **C2E3-PD003** | **C2E3-CAAX-MMAE** |
|---|---|---|---|
| Mino | 0.197 | No response | 0.635 |
| Jeko-1 | >10 | No response | 1.36 |

### (3) In-vitro cytotoxicity in gastric cancer cell line and breast cancer cell line

The *in-vitro* cytotoxicity of D20001 (T-PD001), an ADC containing an anti-Her2 antibody, was detected using a gastric cancer cell line (NCI-N87) and a breast cancer cell line (HCC1954) expressing Her2. The control group used herein was D20103 (B58-PD001) including an anti-BCMA antibody linked to the same drug, PD001. Specifically, 50 µl of each of the NCI-N87 cell line (ATCC, CRL-5822^{™}) and the HCC1954 cell line (ATCC, CRL-2338^{™}) cultured in a medium containing RPMI (ATCC), 10% FBS (Gibco), and Antibiotic-Antimycotic (Gibco) was seeded at 10,000 cells/well or 5,000 cells/well on a 96-well plate, followed by performing a test for determining target-specific cytotoxicity. The subsequent experimental method was the same as that for the anti-BCMA ADC.

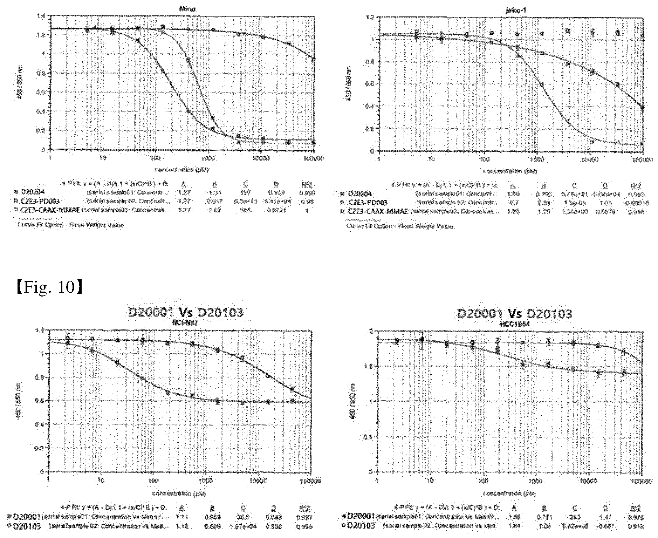

The results are shown in Table 6 and FIG. 10. The NCI-N87 and HCC1954 cell lines expressing Her2 exhibited cytotoxicity by D20001, but did not exhibit cytotoxicity by D20103, an anti-BCMA ADC. This demonstrates that the ADC according to the present invention has target-specific cytotoxicity depending on the type of antibody contained in the ADC.

**[Table 6]**

| **IC50(nM)** | **D20001** | **D20103(non-binder)** | **Non-binder/Binder ratio** |
|---|---|---|---|
| NCl-N87 | 0.0365 | 16.7 | 457 |
| HCC1954 | 0.263 | No response | n.d. |

### (4) In-vitro cytotoxicity in ovarian cancer cell line

The *in-vitro* cytotoxicity of D20002 (10H1-PD001), an ADC containing an anti-NaPi2b antibody, was detected using an ovarian cancer cell line (OVCAR-3) expressing NaPi2b. The control group used herein was D20103 (B58-PD001), in which an anti-BCMA antibody was linked to the same drug, PD001. Specifically, 50 µl of an OVCAR-3 cell line (ATCC, HTB-161^{™}) cultured in a medium containing RPMI (ATCC), 20% FBS (Gibco), and Antibiotic-Antimycotic (Gibco) were seeded at 5,000 cells/well to each well of a 96-well plate, followed by performing a test for determining *in vitro* cytotoxicity. The subsequent experimental method was the same as that of the anti-BCMA ADC.

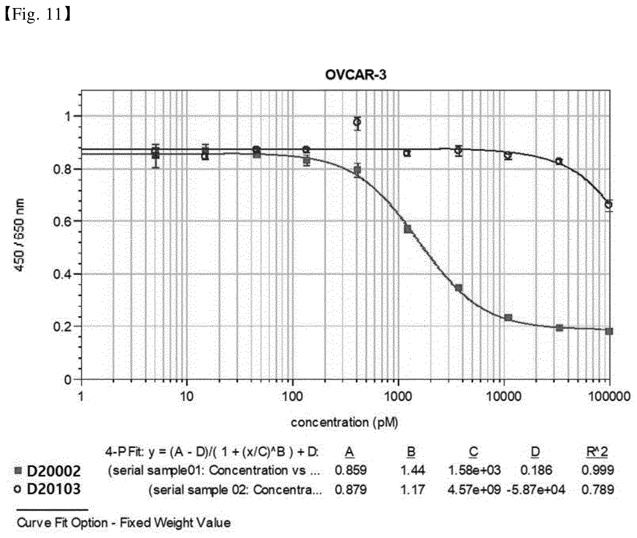

The results are shown in FIG. 11. As can be seen from FIG. 11, the ADC containing D20002 exhibited an IC₅₀ of 1.53 nM. The ADC containing D20002 had a non-binder/ binder ratio of 100 or more and thus exhibited the best reactivity among solid cancer cell lines. On the other hand, the ADC containing D20103 had no cytotoxicity by D20103, an anti-BCMA ADC. This demonstrates that the ADC according to the present invention has target-specific cytotoxicity depending on the type of antibody contained in the ADC.

### (5) Conclusion

The *in vitro* cytotoxicity of the ADC of the present invention prepared to include a drug containing D-galactose β-pyranose as a trigger was compared with those of various control ADCs. The result showed that most of the ADCs according to the present invention had an IC₅₀ of 1 nM or less in various cell lines, and the potential thereof as a potent drug was determined by an *in-vitro* experiment (Table 7) .

**[Table 7]**

| **Cell Lines** | **Cell line types** | **ADC targets** | **IC50 (nM)** | | |
|---|---|---|---|---|---|
| | | | **PD001** | **PD006** | **PD009** |
| **H929** | Multiple myeloma | BCMA | 0.2-1.5 | 1.4 | 1.5 |
| **Mino** | Mantle cell lymphoma | ROR1 | 0.197 | ND | ND |
| **Jako-1** | Mantle cell lymphoma | ROR1 | >10 | ND | ND |
| **NCl-N87** | Gastric cancer | Her2 | 0.05* | ND | ND |
| **HCC1954** | Breast cancer | Her2 | 0.2* | ND | ND |
| **OVCAR3** | Ovarian cancer | NaP128 | 1.5 | ND | NO |
| **EOL-1** | AML | CLL-1 | NA | NA | 0.3 |

| | | | | | |
|---|---|---|---|---|---|
| • **Sigmoidal response curve with incomplete response** • **ND: Tested, but not determined** • **NA:Not available** | | | | | |

### 4-4. Detection of in-vivo target-specific cytotoxicity

In order to determine the *in-vivo* target-specific cytotoxicity of ADCs, tumor suppression efficacy in xenograft mouse models of various cancer cell lines was tested. Specifically, 0.5 or 1x10⁷ cancer cells were mixed with Matrigel, and then subcutaneously injected into the flank of 6-8 week old female Fox chase SCID mice (CB17/Icr-*Prkdc*^{scid}/IcrIcoCrl) or nude mice. The mice were sequentially classified into respective experimental groups when the average tumor size reached 150 to 200 mm³. The drug was administered via the tail vein of each of the mice in the ADC and control groups and PBS (vehicle) was used as a negative control. The size of the tumor was determined by measuring the long axis and the short axis of the tumor using vernier calipers, and the tumor volume was calculated using the following equation:

Tumor volume (mm³) = (0.5) x (long axis) x (short axis)2

### (1) Cytotoxicity in human mantle cell lymphoma cell xenograft mouse model

The *in vivo* cytotoxicity of D20204 (C2E3-PD001) was detected in a xenograft mouse model using Jeko-1, a human mantle cell lymphoma cell line expressing ROR1. C2E3-mc-MMAF including C2E3 as an antibody and MMAF as a drug was used as a control group and the administration was performed at 4 mg/kg in a single ADC dose 3 times weekly.

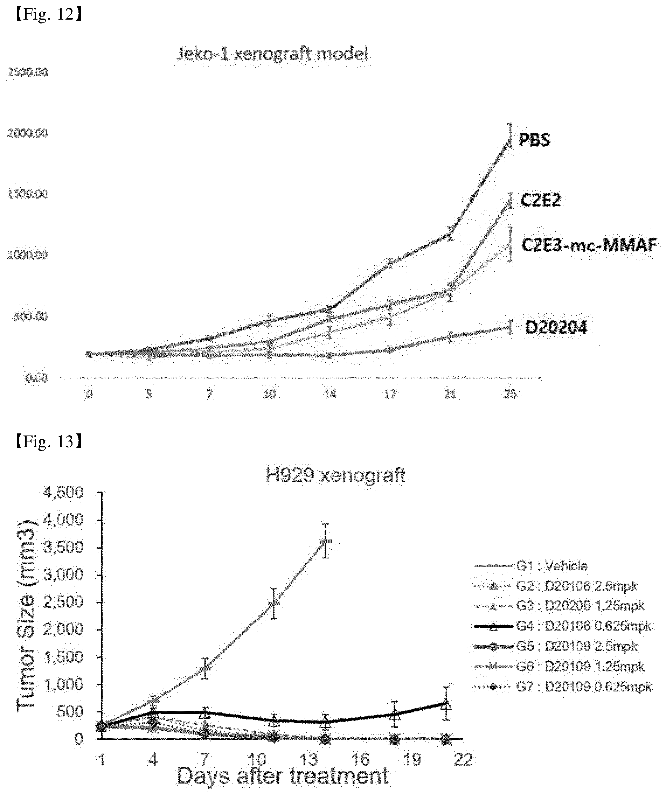

The results are shown in FIG. 12. D20204 exhibited an IC₅₀ of greater than 10 nM in an *in vitro* test (FIG. 9), but exhibited a remarkably superior effect than an ADC conjugated with MMAF in an *in vivo* experiment.

### (2) Cytotoxicity in human multiple myeloma cell xenograft mouse model

The *in-vivo* cytotoxicity of D20106 (B58-PD006) and D20109 (B58-PD009) was detected in a xenograft mouse model using NCI-H929, a multiple myeloma cell line expressing BCMA. Specifically, 6-7-week-old Fox chase SCID mice were used, and 1x10⁷ NCl-H929 cells were mixed with Matrigel and implanted subcutaneously in the flank of each of the mice. The drug was administered thereto when the average tumor size reached 180 to 200 mm³. The two substances, D20106 and D20109, were each administered in three doses (2.5 mpk, 1.25 mpk, 0.625 mpk), and the size of the tumor was measured twice a week and observed for 3 weeks.

As shown in FIG. 13, both D20106 and D20109 exhibited excellent cytotoxicity in the multiple myeloma cell xenograft model. Specifically, D20106 and D20109 effectively inhibited the growth of NCI-H929 tumors, and tumor disappeared in treatment groups other than the group administered with D20106 at a low dose (0.625 mpk).

### (3) Cytotoxicity in acute myeloid leukemia cell xenograft mouse model

The *in-vivo* cytotoxicity of D20502 (6E7-PD009) was detected in xenograft mouse models using CLL-1 expressing acute myeloid lymphoma (AML) cell lines, EOL-1 and HL-60. Specifically, 6-7-week-old Fox chase SCID mice were used, 5x10⁶ EOL-1 or HL-60 cells were mixed with Matrigel and implanted subcutaneously into the flank of each mouse, and the drug was administered thereto when the average tumor size was 180 to 200 mm³. D20502 was administered once in a single dose of 2 mpk, and the size of the tumor was measured twice a week and observed for 3 weeks.

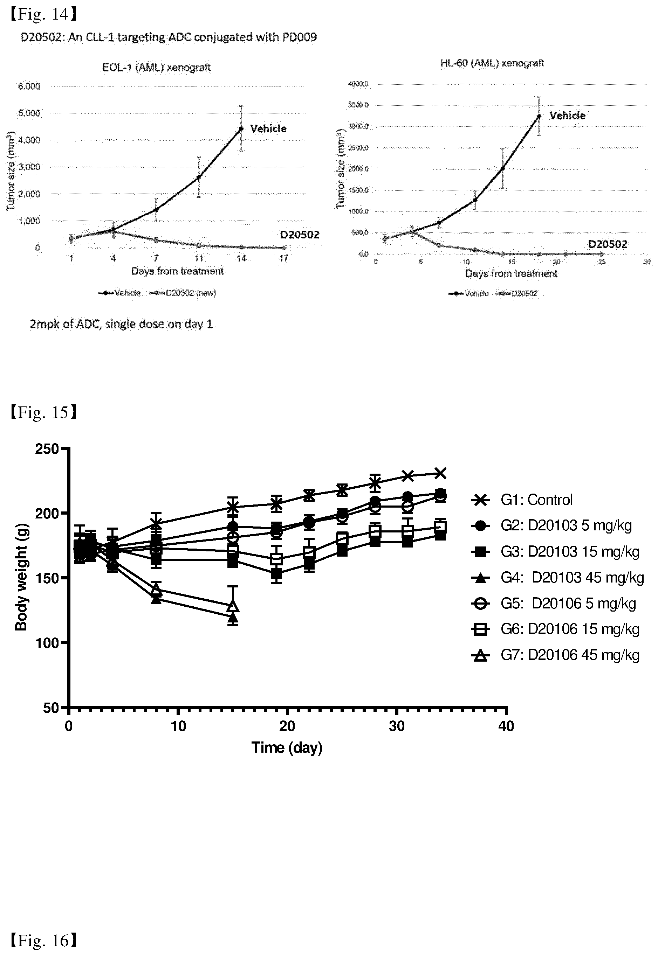

As can be seen from FIG. 14, at a dose of 2 mpk, D20502 effectively inhibited tumor growth in both AML xenograft models and all tumors disappeared at 2 weeks after administration.

### 4-5. Single-dose rodent toxicity test 1 to determine maximum tolerated dose

A single-dose rodent toxicity test was performed to evaluate the toxicity of ADC containing PD001 or PD006 and to determine the maximum tolerated dose. Specifically, 7-8-week-old female SD rats were grouped sequentially as shown in Table 8 below so that the average body weight was similar across the groups. The ADC was administered in a dose of 5 to 45 mpk to the tail vein thereof and blood samples were collected on days 3, 18 and 35 for hematological and serum biochemical analyses. All other animals were sacrificed on day 34, and histopathological analysis of major organs was performed. Group 1, a control group, was administered with 20 mM histidine and 7% trehalose at pH 6.0 as a vehicle.

**[Table 8]**

| | **Test substance** | **Dosage** | **Number of administrations** |
|---|---|---|---|
| **Group 1 (G1)** | Excipient | - | Once |
| **Group 2 (G2)** | D20103 | 5 mpk | Once |
| **Group 3 (G3)** | D20103 | 15 mpk | Once |
| **Group 4 (G4)** | D20103 | 45 mpk | Once |
| **Group 5 (G5)** | D20106 | 5 mpk | Once |
| **Group 6 (G6)** | D20106 | 15 mpk | Once |
| **Group 7 (G7)** | D20106 | 45 mpk | Once |

### (1) Weight change

All animals were weighed on day 1 (before administration), and on days 2, 4, 8, 15, 19, 22, 25, 28, 31, 34 and 35 using a small animal scale. The weight of dead and moribund animals was measured and an autopsy was subsequently performed thereon, immediately after such dead or moribund animals were found.

The results of measurement of weight change are shown in FIG. 15. As shown in FIG. 15, no weight loss was observed in groups other than Group 4 and Group 7 administered at a dose of 45 mpk. In Group 4 and Group 7, administered at the maximum dose, all subjects died before the end of the experiment.

### (2) Hematological analysis

The toxicity of ADCs was evaluated by hematological tests. After administration, intravenous blood collection was performed on Day 3 (first blood collection), Day 18 (second blood collection) and Day 35 (third blood collection), and animals were fasted (but provided with water) overnight before blood collection. After injecting about 0.5 mL of blood into a CBC bottle containing EDTA-2K, an anticoagulant, the change in the level of white blood cells was measured using an automated hematology analyzer and the results are shown in FIG. 16. The result of analysis showed that no notable toxicity was observed in groups other than Group 4 and Group 7 administered at high doses. The white blood cell count was temporarily decreased due to toxicity caused by the administered substance, but gradually recovered over time.

### (3) Serum biochemical analysis

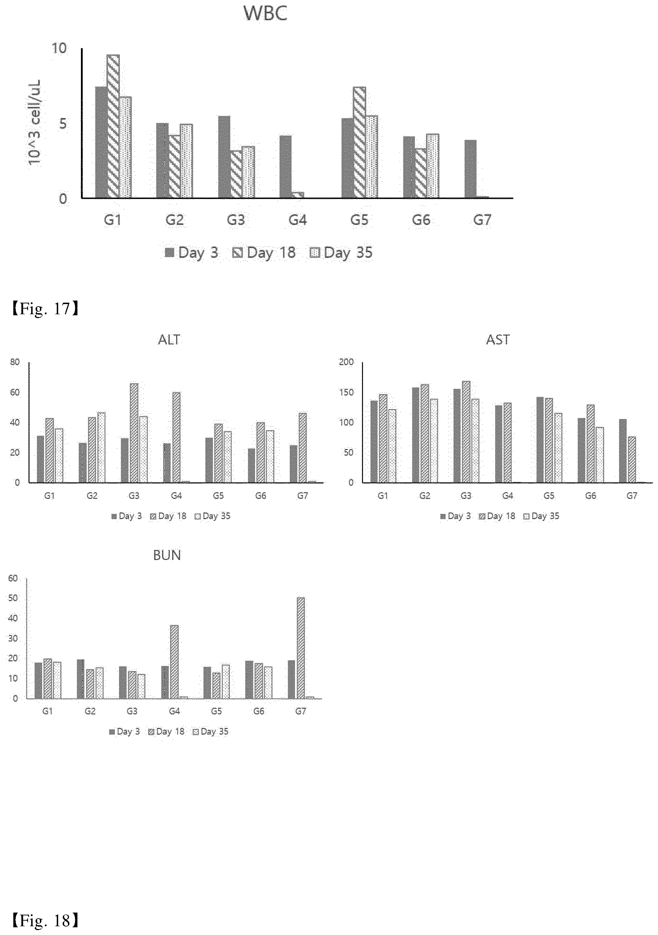

The toxicity of ADC was detected through analysis of blood biochemical parameters such as ALT (alanine aminotransferase), AST (aspartate aminotransferase), and blood urea nitrogen (BUN). After administration, intravenous blood collection was performed on Day 3 (primary blood collection), Day 18 (second blood collection), and Day 35 (third blood collection). About 1 mL of blood was injected into a 5 mL vacutainer containing a clot activator, was allowed to clot at room temperature for 15 to 20 minutes, and then centrifuged for 10 minutes. The obtained serum was tested using a blood biochemical analyzer. The result is shown in FIG. 17. As a result of the analysis, no notable toxicity was observed in groups other than Group 4 and Group 7, which were administered at a dose of 45 mpk.

### (4) Pathological analysis results

Table 9 shows the symptoms for each organ observed upon autopsy. Atrophy of the thymus was observed in the groups administered with the ADCs, namely, G2, G3, G4, and G7, but this was considered to be due to a decrease in leukocytes rather than the direct effect of the test substance. Other findings were found in the spleen, stomach, and lymph nodes of moribund animals, but were not accompanied by histopathological changes or were spontaneous changes, so they were considered not to be the symptoms caused by ADC toxicity.

Table 10 shows histopathological results. The formation of megakaryocytes was observed in the renal tubules and exhibited a dose-response correlation, which was considered to be due to the test substance. In particular, this phenomenon was observed only in D20103 and not in D20106, so it was assumed that the PEG group used for the linker was involved in reducing nephrotoxicity.

**[Table 9]**

| Location | Gross findings | Group(#animal ID) | Correlated microscopic fi ndings |
|---|---|---|---|
| Thymus | Small | G2(#5) | None |
| | | G3(#7) | |
| | | G3(#8, 9) | Atrophy |
| | | G4(#10, 11, 12) | |
| | | G7(#19, 20, 21) | |
| Spleen | Small | G4(#10, 11, 12) | Atrophy |
| | | G7(#20, 21) | |
| Glandular Stomach | Discoloration | G4(#10) | Erosion |
| | | G7(#20, 21) | Congestion |
| Nonglandular stomach | Discoloration | G7(#20) | None |
| Hind leg and Abdominal cavity | cannibalism | G7(#19) | No Tissue |
| Mesentric lymph node | Discoloration | G7(#20, 21) | Conjestion/hemorrha ge, Agonal |

**[Table 10]**

| **Organ** | **Microscopic findings** | | | **(D20103)** | | | **(D20106)** | | |
|---|---|---|---|---|---|---|---|---|---|
| | | | | | | | | | **G1: Vehicle** |
| | **Groups** | **1** | **2** | **3** | **4** | **5** | **6** | **7** | **G2: D20103 5 mpk** |
| | **Dosage of D20103 (mg/lcg/day)** | **0** | **5** | **15** | **45** | **0** | **0** | **0** | **G3: D20103 15 mpk** |
| | **Dosage of D20106 (mg/kg/day)** | **0** | **0** | **0** | **0** | **5** | **15** | **45** | **G4: D20103 45 mpk** |
| | **No. Animals examined** | **3** | **3** | **3** | **3¹⁾** | **3** | **3** | **3²⁾** | **G5: D20106 5 mpk** |
| **Kidney** | | | | | | | | | **G6: D20106 15 mpk** |
| **Karyomegaly** | | | | | | | | | **G7: D20106 45 mpk** |
| | **minimal** | **0** | **2** | **0** | **0** | **0** | **0** | **0** | |
| | **mild** | **0** | **0** | **3** | **0** | **0** | **0** | **0** | |
| | **severe** | **0** | **0** | **0** | **3** | **0** | **0** | **0** | |

### 4-6. Single-dose rodent toxicity test 2 to determine maximum tolerated dose

Experimental groups were set as shown in the following Table 11 and a single-dose rodent toxicity test was performed thereon to evaluate the toxicity of the ADC containing PD006 or PD009 and to determine the maximum tolerated dose.

**[Table 11]**

| Group | Test substance | Dose |
|---|---|---|
| G1 | Vehicle | |
| G2 | D20106 | 10 mpk, once |
| G3 | D20106 | 20 mpk, once |
| G4 | D20106 | 30 mpk, once |
| G5 | D20109 | 5 mpk, once |
| G6 | D20109 | 10 mpk, once |
| G7 | D20109 | 20 mpk, once |

The administration, breeding and weighing methods were the same as described in 4-5 above. Blood was collected on days 3, 14, and 35 after administration, and hematological examination was performed using an automated blood analyzer. After completion of the test, euthanasia, autopsy and pathological examination were performed. Group 1, a control group, was administered with 20 mM histidine with 7% trehalose at pH 6.0 as a vehicle.

Changes in the body weight of the test animals are shown in FIG. 18. An acute toxic reaction involving weight loss was observed for 2 weeks after administration to all experimental groups, but was recovered in groups other than G4 and G7, which were groups administered with the highest dose. G4 and G7 were slowly recovered within 3 weeks after administration. The weight loss of all groups on day 36 was the change due to fasting before sacrifice.

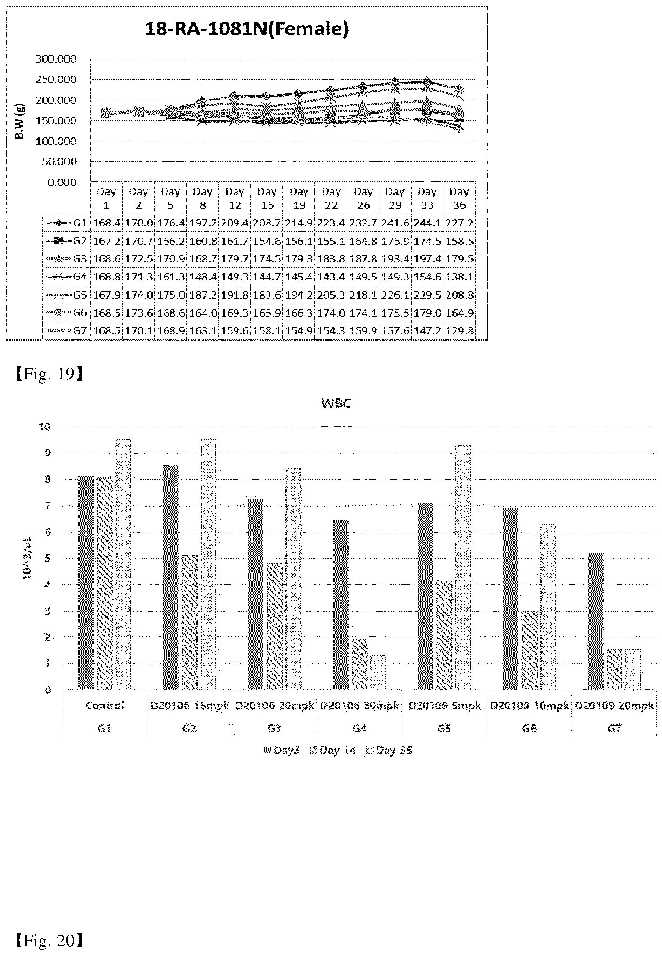

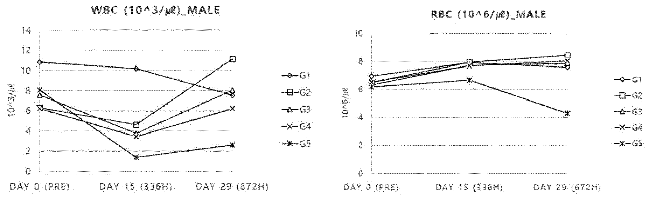

In addition, the result of measurement of the change in the leukocyte count upon hematological examination is shown in FIG. 19. A pattern similar to the change in body weight was observed, and in groups other than the groups administered at the highest dose, a decrease in leukocyte toxicity was observed on day 14 after administration and was recovered on day 35 after administration, which is the end date of the test. However, in the groups receiving the highest doses, namely, G4 and G7 groups, there was no pattern of recovery of white blood cell count.

Therefore, based on the two indicators of body weight and white blood cell count, the maximum tolerated dose (MTD) of D20106 containing PD006 was set to 20 mpk, and the maximum tolerated dose of D20109 containing PD009 was set to 10 mpk.

### 4-7. Single-dose rodent toxicity test for comparison with similar substances

In order to prove that a payload containing β-galactose as a trigger is superior in toxicity to a payload containing another trigger having a structure similar thereto, GT70 having the following structure and being based on CBI and β-glucuronide was synthesized, an ADC was prepared based thereon, and toxicity was compared. An ADC was prepared in the same manner as in Example 2, except that the GT70 as a linker-drug was conjugated to the B58 antibody, and was designated "D20111".

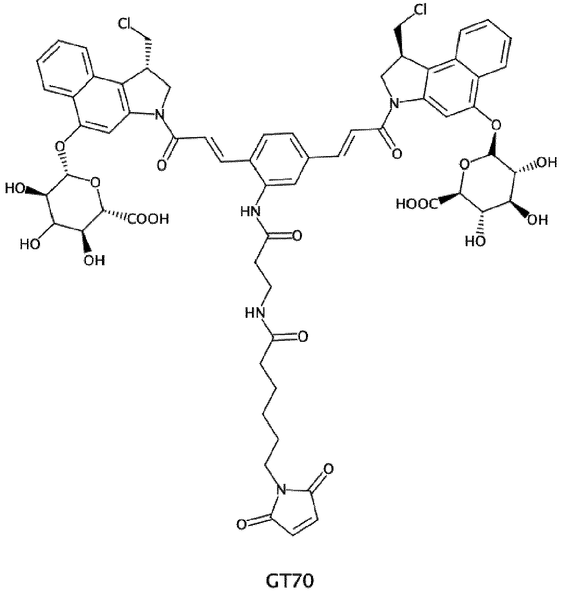

Each of D20109 and D20111 was administered once to SD rats, changes in body weight and hematologic and blood biochemical changes were observed for 28 days, and toxicity expression and recovery were determined based on these changes. The detailed method of the single-dose toxicity test is as described in Example 4-5 above, and the configuration of the experimental group is shown in Table 12 below.

**[Table 12]**

| | **Test substance** | **Dosage** | **Number of doses** |
|---|---|---|---|
| **Group 1** | Vehicle | | Single Dose |
| **Group 2** | D20109 | 6.16 mpk | Single Dose |
| **Group 3** | D20109 | 12.32 mpk | Single Dose |
| **Group 4** | D20111 | 6.30 mpk | Single Dose |
| **Group 5** | D20111 | 12.6 mpk | Single Dose |

The test results are shown in FIG. 20. All toxicological indicators showed a dose-dependent response, and a more toxic response was observed in the high-dose groups (Group 3 and Group 5) than in the low-dose groups (Group 2 and Group 4). When the change in body weight was observed, at the same dose level, the group administered with D20109 lost less weight than the group administered with D20111, and recovered more of the weight at the end of the test (FIG. 20). Even when the toxicity was compared based on the blood leukocyte count, which is a hematological indicator, the D20109-administered group exhibited a smaller reduction in leukocytes compared to the D20111-administered group and exhibited a recovery pattern (FIG. 20). In particular, the blood erythrocyte count for males did not recover in the high-dose group, D20111, until the end of the experiment (FIG. 20). This supports that β-galactose had remarkably superior stability compared to other materials having a similar structure when used as a trigger of ADC.

### [Industrial Applicability]

The ADC to which a payload containing a galactose trigger is linked according to the present invention has a target-specific property in that cytotoxicity is shown only when galactose is removed by beta-galactosidase, which is expressed highly in cancer or tumor cells, thereby remarkably reducing the risk of early release of the drug and subsequent systemic toxicity caused thereby. Moreover, it was found that the ADC containing the galactose trigger according to the present invention exhibits superior drug efficacy and lower *in-vivo* toxicity compared to ADCs having another trigger having a similar structure thereto, for example, ADCs having glucose or glucronide as a trigger. That is, an ADC that is less toxic, is highly stable *in vivo*, exhibits superior efficacy, and has a wider treatment window compared to conventional prodrugs can be prepared using the payload utilizing the galactose trigger according to the present invention.

Although specific configurations of the present invention have been described in detail, those skilled in the art will appreciate that this description is provided to set forth preferred embodiments for illustrative purposes, and should not be construed as limiting the scope of the present invention. Therefore, the substantial scope of the present invention is defined by the accompanying claims and equivalents thereto.

### [Sequence Listing Free Text]

An electronic file is attached.

## Claims

1. A compound represented by the following Formula (I) or a pharmaceutically acceptable salt or solvate thereof: 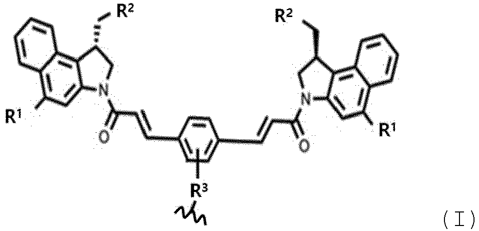
wherein R¹ is D-galactose β-pyranose or D-galactose α-pyranose;
R² is Cl or Br; and
R³ is a single bond, -O- or -NH-.

2. A compound represented by the following Formula (II) or a pharmaceutically acceptable salt or solvate thereof: 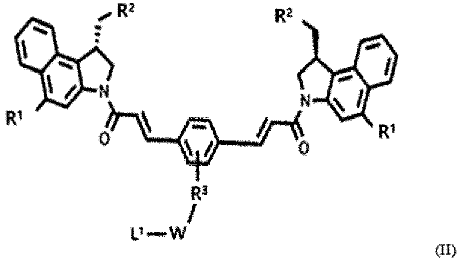 wherein R¹ is D-galactose β-pyranose or D-galactose α-pyranose;
R² is Cl or Br;
R³ is a single bond, -O- or -NH-;
W is a spacer; and
L¹ is a linker.

3. The compound according to claim 2, wherein W is -R⁴-A-R⁵-, -R⁴-A-, -(CH₂CH₂R⁶)ₓ-, -(CH₂)ᵣ(R⁷(CH₂)ₚ)_{q}-, - ((CH₂)ₚR⁷)_{q}-, -(CH₂)ᵣ(R⁷(CH₂)ₚ)_{q}R⁸-, - ((CH₂)ₚR⁷)_{q} (CH₂)ᵣ-, - R⁸ ((CH₂)ₚR⁷)_{q}- or -(CH₂)ᵣ(R⁷(CH₂)ₚ)_{q}R⁸CH₂-,
wherein R⁴ and R⁵ are each independently-(CH₂)ᵣ(V(CH₂)ₓ)ₚ(CH₂)_{q}, wherein A is a direct bond or a peptide bond; and V is a single bond, O or S; and
R⁶ is -O-, C₁-C₈ alkylene, -NR⁹- or -C(O)NR¹³-; and
R⁷ and R⁸ are each independently a single bond, -O-, - NR¹⁰-, -C (O) NR¹¹-, -NR¹²C(O) - or C₃-C₂₀ heteroaryl,
wherein R⁹ to R¹³ are each independently hydrogen, C₁-C₆ alkyl, (C₁-C₆ alkyl) C₆-C₂₀ aryl or (C₁-C₆ alkyl) C₃-C₂₀ heteroaryl;
X is an integer of 1 to 5;
r is an integer of 0 to 10;
p is an integer of 0 to 10; and
q is an integer of 0 to 20,
wherein 1 to 10 hydrogen atoms in W are optionally substituted with hydroxy, C₁-C₈ alkyl, C₁-C₈ alkoxy, amino, ONH₂ or oxo.

4. The compound according to claim 2, wherein L¹ is hydroxy, aldehyde, ONH₂, NH₂, or 4- to 7-membered heteroaryl containing 1 to 3 heteroatoms selected from N, O and S, wherein the heteroaryl can be substituted with 1 to 5 substituents independently selected from hydroxy, aldehyde, C₁-C₈ alkyl, C₁-C₈ alkoxy, amino, ONH₂ and oxo, or has a structure represented by the following Formula (I-a) or (I-b) : 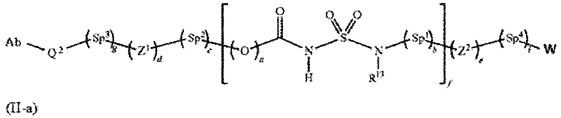 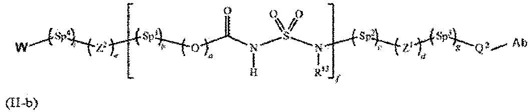
wherein Q¹ is cyclooctynyl or heterocyclooctynyl, wherein the cyclooctynyl or heterocyclooctynyl is optionally each independently fused with 1 or 2 rings selected from C₃-C₁₂ cycloalkyl, C₃-C₁₂ aryl and C₃-C₁₂ heteroaryl and is optionally substituted with hydroxy, C₁-C₈ alkyl, C₁-C₈ alkoxy, amino, ONH₂ or oxo;
R¹³ is selected from C₁-C₂₄ alkyl, C₃-C₂₄ cycloalkyl, C₃-C24 aryl, C3-C24 heteroaryl, C₃-C₂₄ alkylaryl, C₃-C₂₄ alkylheteroaryl, C₃-C₂₄ arylalkyl and C₃-C₂₄ heteroarylalkyl, wherein the heteroaryl contains a heteroatom selected from O, S and NR¹⁴, wherein R¹⁴ is hydrogen or a C₁-C₄ alkyl group;
Sp¹, Sp², Sp³ and Sp⁴ are spacer moieties and are each independently selected from a single bond, or straight or branched C₁-C₂₀₀ alkylene, C₂-C₂₀₀ alkenylene, C₂-C₂₀₀ alkynylene, C₃-C₂₀₀ cycloalkylene, C₅-C₂₀₀ cycloalkenylene, C₈-C₂₀₀ cycloalkynylene, C₇-C₂₀₀ alkylarylene, C₇-C₂₀₀ arylalkylene, C₈-C₂₀₀ arylalkenylene and C₉-C₂₀₀ arylalkynylene, wherein the alkylene, alkenylene, alkynylene, cycloalkylene, cycloalkenylene, cycloalkynylene, alkylarylene, arylalkylene, aryl alkenylene and arylalkynylene are optionally substituted with or contain a heteroatom selected from O, S and NR¹⁴;
Z¹ and Z² are each independently selected from O, C(O) and N (R¹³) ;
a is each independently 0 or 1;
b is each independently 0 or 1;
c is 0 or 1;
d is 0 or 1;
e is 0 or 1;
f is an integer from 0 to 150;
g is 0 or 1; and
i is 0 or 1.

5. The compound according to claim 2, comprising a compound represented by the following Formulae.

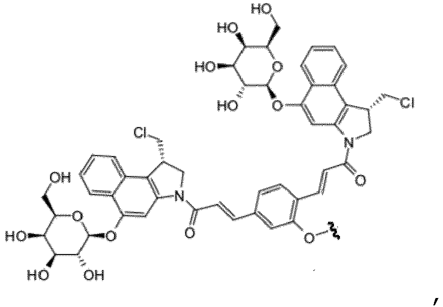

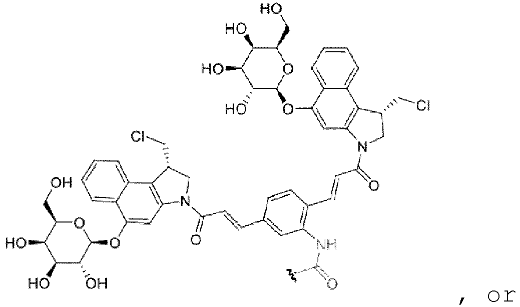

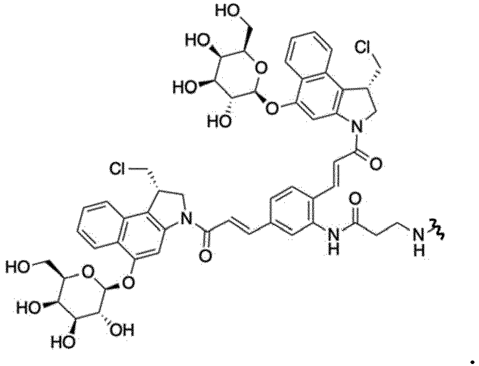

6. The compound according to claim 2, comprising a compound represented by the following Formulae: 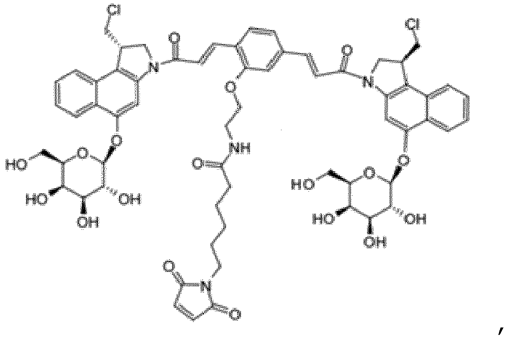 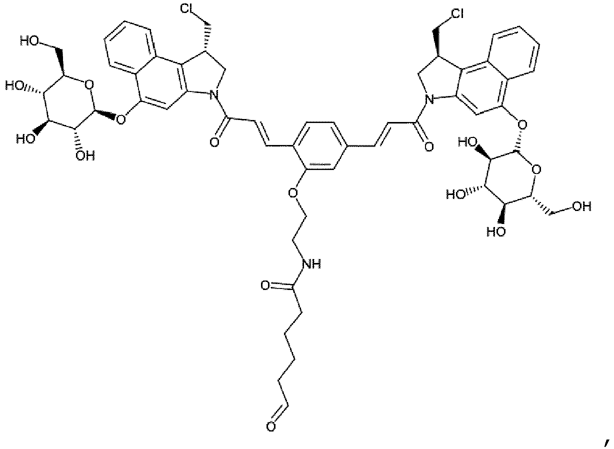 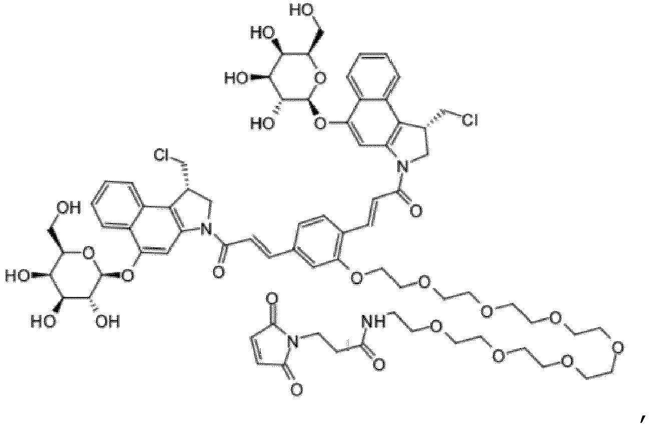 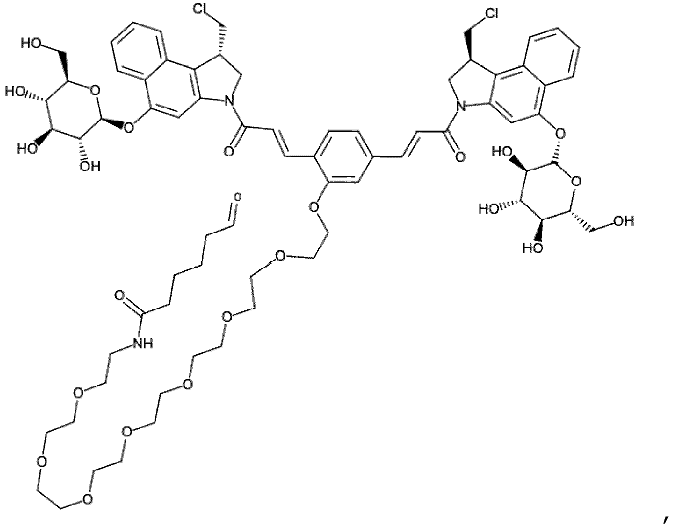 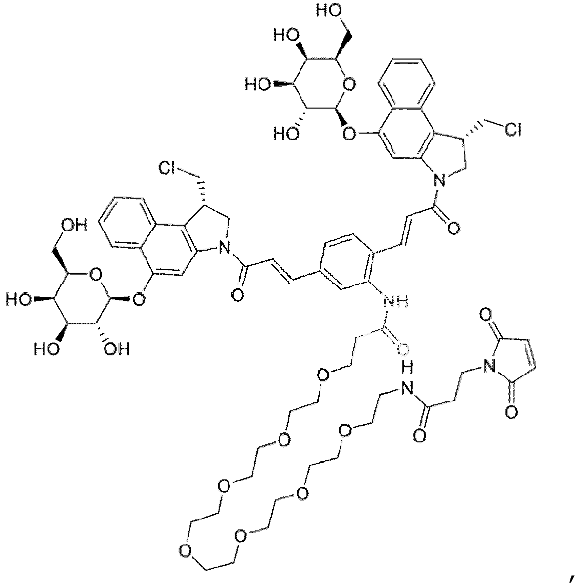 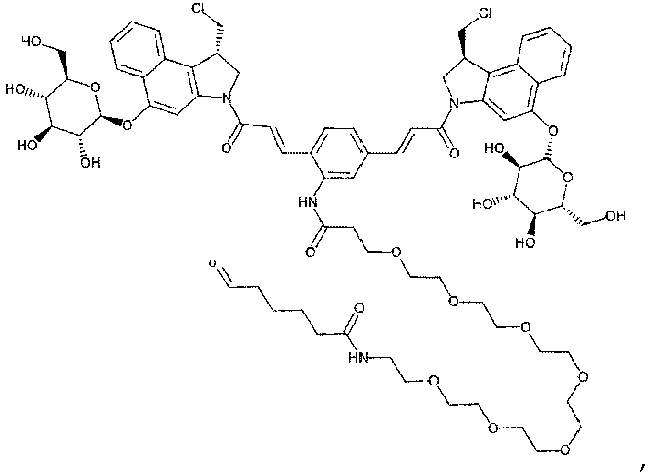 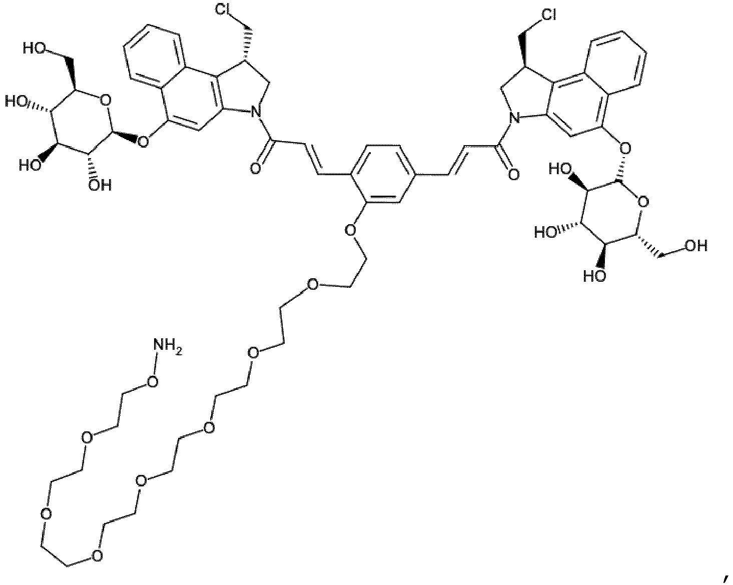 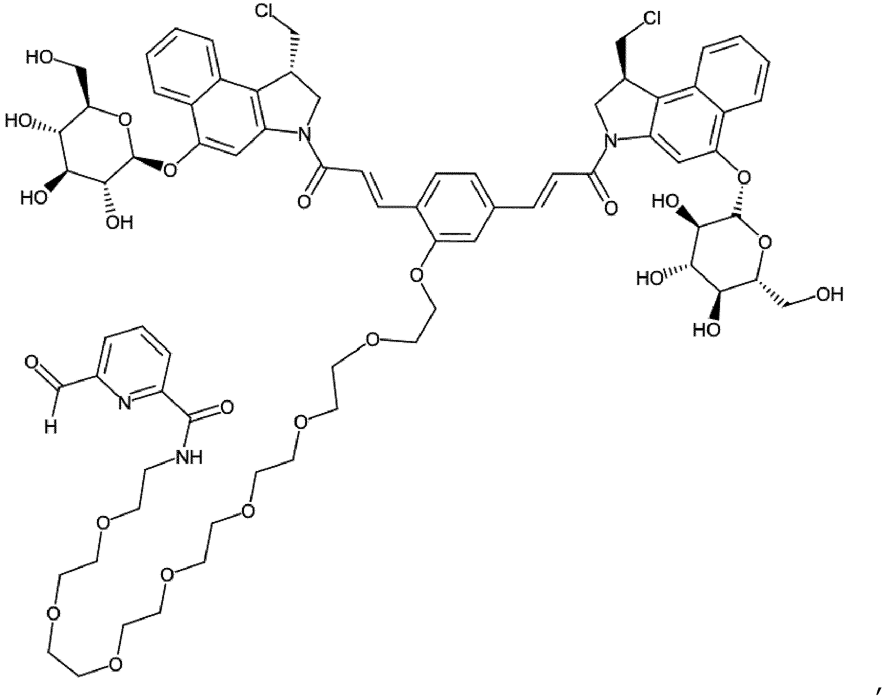 or

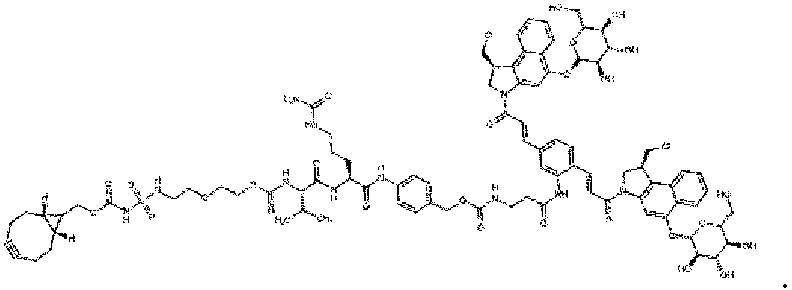

7. An antibody-drug conjugate comprising a compound represented by the following Formula (III), or a pharmaceutically acceptable salt or solvate thereof: 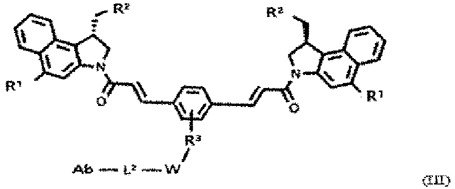 wherein
R¹ is D-galactose β-pyranose or D-galactose α-pyranose;
R² is Cl or Br;
R³ is a single bond, -O- or -NH-;
W is a spacer;
L² is a linker; and
Ab is an antibody or an antigen-binding fragment thereof.

8. The antibody-drug conjugate according to claim 7, wherein W is -R⁴-A-R⁵-, -R⁴-A-, - (CH₂CH₂R⁶)ₓ-, - (CH₂)ᵣ(R⁷(CH₂)ₚ)q⁻, -((CH₂)ₚR⁷)_{q}-, -(CH₂)ᵣ(R⁷(CH₂)ₚ)qR⁸- , - ((CH₂)ₚR⁷) _{q}(CH₂)ᵣ-, -R⁸((CH₂)ₚR⁷)_{q}- or - (CH₂)ᵣ(R⁷(CH₂)ₚ)_{q}R⁸CH₂-,
wherein R⁴ and R⁵ are each independently-(CH₂)ᵣ(V(CH₂)ₓ)ₚ(CH₂)_{q}, wherein A is a direct bond or a peptide bond; and V is a single bond, O or S; and
R⁶ is -O-, C₁-C₈ alkylene, -NR⁹⁻ or -C(O)NR₂-; and
R⁷ and R⁸ are each independently a single bond, -O-, - NR¹⁰-, -C (O) NR¹¹-, -NR¹²C(O) - or C₃-C₂₀ heteroaryl,
wherein R⁹ to R¹³ are each independently hydrogen, C₁-C₆ alkyl, (C₁-C₆ alkyl) C₆-C₂₀ aryl or (C₁-C₆ alkyl) C₃-C₂₀ heteroaryl;
X is an integer of 1 to 5;
r is an integer of 0 to 10;
p is an integer of 0 to 10; and
q is an integer of 0 to 20,
wherein 1 to 10 hydrogen atoms in W are optionally substituted with hydroxy, C₁-C₈ alkyl, C₁-C₈ alkoxy, amino, ONH₂ or oxo.

9. The antibody-drug conjugate according to claim 7, wherein L² is -CH₂NH-, -ON=C(CH₃)-, -ON=, -CH=, CH=N-, or 4-to 7-membered heterocycle containing 1 to 3 heteroatoms selected from N, O and S, wherein the heterocycle can be substituted with 1 to 5 substituents independently selected from hydroxy, aldehyde, C₁-C₈ alkyl, C₁-C₈ alkoxy, amino, ONH₂ and oxo, or has a structure represented by the following Formula (II-a) or (II-b): 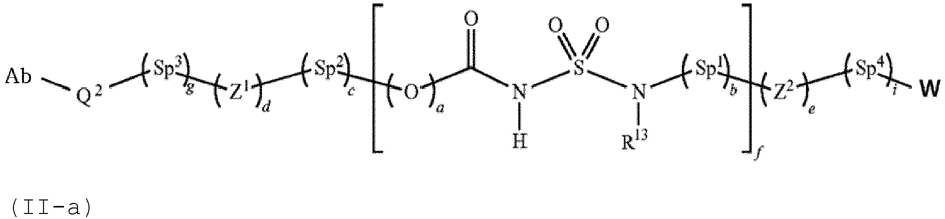 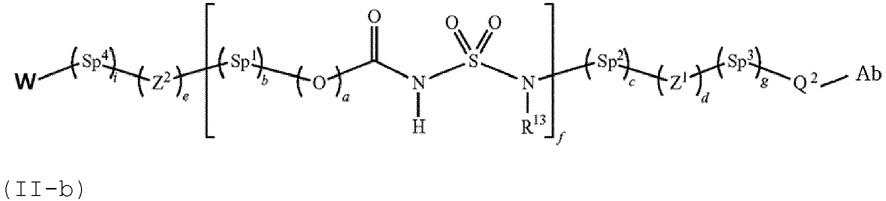
wherein Q² is cyclooctenyl fused with triazole or heterocyclooctenyl fused with triazole, wherein the cyclooctenyl or heterocyclooctenyl is optionally further fused with 1 or 2 rings each independently selected from C₃-C₁₂ cycloalkyl, C₃-C₁₂ aryl and C₃-C₁₂ heteroaryl, is optionally substituted with hydroxy, C₁-C₈ alkyl, C₁-C₈ alkoxy, amino, ONH₂ or oxo, wherein Q² is linked to Ab through a nitrogen atom contained in the triazole;
Sp¹, Sp², Sp³ and Sp⁴ are spacer moieties and are each independently selected from a single bond, or straight or branched C₁-C₂₀₀ alkylene, C₂-C₂₀₀ alkenylene, C₂-C₂₀₀ alkynylene, C₃-C₂₀₀ cycloalkylene, C₅-C₂₀₀ cycloalkenylene, C₈-C₂₀₀ cycloalkynylene, C₇-C₂₀₀ alkylarylene, C₇-C₂₀₀ arylalkylene, C₈-C₂₀₀ arylalkenylene and C₉-C₂₀₀ arylalkynylene, wherein the alkylene, alkenylene, alkynylene, cycloalkylene, cycloalkenylene, cycloalkynylene, alkylarylene, arylalkylene, aryl alkenylene and arylalkynylene are optionally substituted with or contain a heteroatom selected from O, S and NR¹⁴;
Z¹ and Z² are each independently selected from O, C(O) and N (R¹³) ;
a is each independently 0 or 1;
b is each independently 0 or 1;
c is 0 or 1;
d is 0 or 1;
e is 0 or 1;
f is an integer of 0 to 150;
g is 0 or 1; and
i is 0 or 1.

10. The antibody-drug conjugate according to claim 7, comprising a compound represented by the following Formulae: 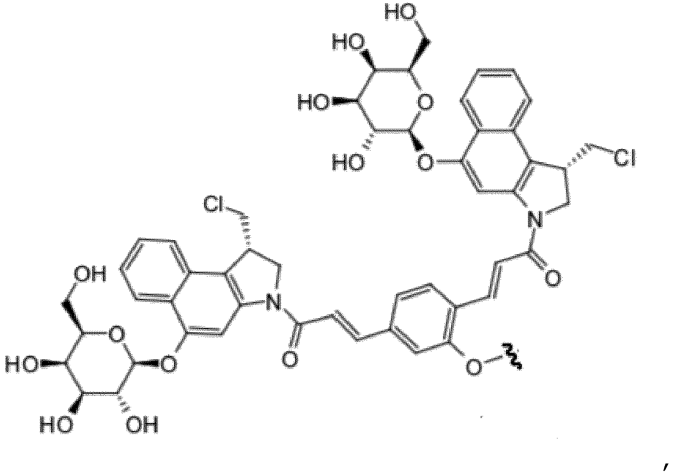 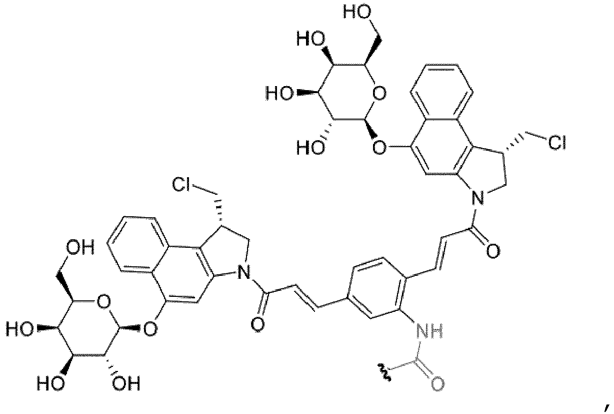 or

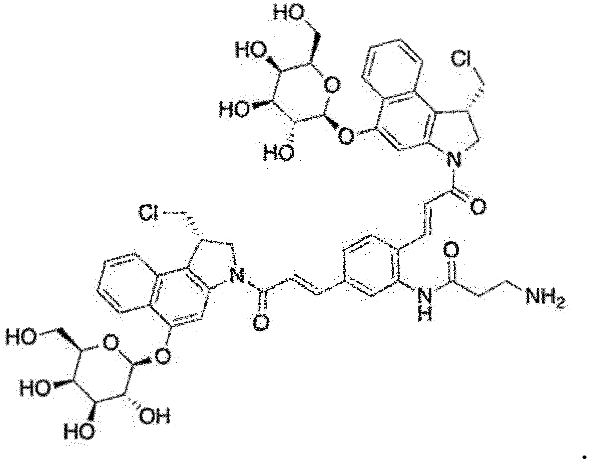

11. The antibody-drug conjugate according to claim 7, wherein the linker is linked to the antibody through cysteine, lysine, an azide group or a ketone group introduced into the antibody or through N-terminus of an antibody protein.

12. The antibody-drug conjugate according to claim 7, wherein the antibody is selected from the group consisting of an anti-BCMA antibody, an anti-ROR1 antibody, an anti-Her2 antibody, an anti-NaPi2b antibody and an anti-CLL1 antibody.

13. A pharmaceutical composition for preventing or treating a proliferative disease comprising the antibody-drug conjugate according to any one of claims 7 to 12.
